(19) **Europäisches Patentamt**

**European Patent Office**

**Office européen des brevets**

(11) **EP 1 120 464 B1**

(12) **EUROPEAN PATENT SPECIFICATION**

(45) Date of publication and mention
of the grant of the patent:
**06.10.2004 Bulletin 2004/41**

(51) Int Cl.[7]: **C12N 15/12**, C07K 16/00,
C12N 15/62, A61K 39/395,
A61K 31/7088, G01N 33/577,
C12Q 1/68

(21) Application number: **00204725.6**

(22) Date of filing: **21.12.2000**

(54) **Stabilizing peptides, polypeptides and antibodies which include them**

Stabilisierende Peptide, und Polypeptide und Antikörper die diese beinhalten

Peptides stabilisants, et polypeptides et anticorps les incluant

(84) Designated Contracting States:
**AT BE CH CY DE DK ES FI FR GB GR IE IT LI LU MC NL PT SE TR**

(30) Priority: **30.12.1999 IT RM990803**

(43) Date of publication of application:
**01.08.2001 Bulletin 2001/31**

(73) Proprietor: **Ente per le Nuove Tecnologie, l'Energia e l'Ambiente - ENEA**
**00196 Roma (IT)**

(72) Inventors:
• **Benvenuto, Eugenio**
**00144 Roma (IT)**
• **Franconi, Rosella**
**00061 Anguillara Sabazia RM (IT)**
• **Desiderio, Angiola**
**00061 Anguillara Sabazia RM (IT)**
• **Tavladoraki, Paraskevi**
**00197 Roma (IT)**

(74) Representative: **Germinario, Claudio**
**Società Italiana Brevetti S.p.A.**
**Piazza di Pietra 39**
**00186 Roma (IT)**

(56) References cited:
• **P. TAVLADORAKI ET AL.: "A single-chain antibody fragment is functionally expressed in the cytoplasm of both Escherichia coli and transgenic plants." EUROPEAN JOURNAL OF BIOCHEMISTRY, vol. 262, June 1999 (1999-06), pages 617-624, XP002167437 Berlin, Germany**

• **P. TAVLADORAKI ET AL.: "Transgenic plants expressing a functional single-chain Fv antibody are specifically protected from virus attack." NATURE, vol. 366, 2 December 1993 (1993-12-02), pages 469-472, XP000421325 London, GB**

• **R. FRANCONI ET AL.: "Functional expression in bacteria and plants of an scFv antibody fragment against tospoviruses." IMMUNOTECHNOLOGY, vol. 4, 1999, pages 189-201, XP004163547 Amsterdam, The Netherlands**

• **K. PROBA ET AL.: "Antibody scFv fragments without disulfide bonds made by molecular evolution." JOURNAL OF MOLECULAR BIOLOGY, vol. 275, no. 2, 16 January 1998 (1998-01-16), pages 245-253, XP000944603 London, GB**

• **P. MARTINEAU ET AL.: "In vitro folding and thermodynamic stability of an antibody fragment selected in vivo for high expression levels in Escherichia coli cytoplasm." JOURNAL OF MOLECULAR BIOLOGY, vol. 292, 1 October 1999 (1999-10-01), pages 921-929, XP000939362 London, GB**

• **E. OHAGE ET AL.: "Intrabody construction and expression. II. A synthetic catalytic Fv fragment." JOURNAL OF MOLECULAR BIOLOGY, vol. 291, no. 5, 3 September 1999 (1999-09-03), pages 1129-1134, XP002175836 London, GB**

• **Y. WU ET AL.: "Binding of intracellular anti-Rev single chain variable fragments to different epitopes of human immunodeficiency virus type 1 Rev: variations in viral inhibition." JOURNAL OF VIROLOGY, vol. 70, no. 5, May 1996 (1996-05), pages 3290-3297, XP002175837 Baltimore, MD, USA**

# EP 1 120 464 B1

**Description**

<u>Field of the invention</u>

The present invention relates to molecules capable of a specific interaction with target molecules.

<u>State of the art</u>

Molecules capable of a specific interaction with targets are known in the art.

In particular antibodies are soluble and stable molecules known for being capable of an interaction with targets which is at the same time specific and effective.

Such molecules are used accordingly for modulating the accumulation and/or expression of target molecules in numerous applications in biology, medicine and agriculture.

It is generally desirable in these applications to have antibody with a great stability.

In particular there are applications wherein is necessary to have an antibody particularly stable wherein the interaction shall, or can, be carried out in an extremely reducing environment, such as the cytoplasm, for example applications carried out in order to:

- block or stabilize interactions between macromolecules (for instance protein-protein or protein-DNA type);
- modulate enzymatic functions covering the active site, confining the substrate or blocking an enzyme in its active or inactive structure;
- remove proteins to their normal cellular compartment, for example confining transcription factors in the cytoplasm, or retaining membrane receptors in the endoplasmic reticulum;
- interact with pathogen-derived molecules in order to interfere with the relevant infective process;
- bind cytoplasmic molecules *in vivo* for diagnostic or therapeutic reasons (for instance oncogene products) inside an intracellular compartment.

Among the antibodies known in the art as showing these features, scFv(F8), an engineered antibody in the scFv ("single chain variable fragment")format, is one of the most representative (Tavladoraki *et al* 1993).

scFv(F8) in fact shows a significant molecular stability, as evidenced by measuring variations of free energy (ΔG) in denaturation and renaturation experiments *in vitro* (Tavladoraki *et al*. 1999).

This molecule is also characterized as having:

- high efficiency in folding (Tavladoraki *et al*. 1999);
- long half-life inside the cytoplasm (Tavladoraki *et al*. 1999);
- functionality in the cytoplasm, in terms of ability to recognize antigens and interfere with the replication of the AMCV virus (Tavladoraki *et al*. 1993);
- high levels of expression as soluble protein in bacterium and plant cytoplasm (Tavladoraki *et al*. 1999);
- ability to interact *in vivo* with its antigen in the cytoplasm of eukaryotic cells, as shown by experiments carried out in the yeast "two-hybrid" system (Visintin *et al*. 1999).

Studies on the redox state of the protein, have further shown that no disulphide bond is formed in the protein inside the cytoplasm (Tavladoraki *et al*. 1999).

ScFv(F8) is a very interesting molecule for biotechnological applications, especially for those pointed out above.

scFv(F8) as such however, being capable of interacting with AMCV virus only, has a relatively limited field of application.

On the contrary, antibodies "scFv(F8)-like" (from the functional point of view) capable of interacting with targets other than AMCV, would be instead of great interest. They would enable a number of applications on specific targets in the cytoplasm or, given the stability, in other different environments.

<u>Summary of the invention</u>

Object of the present invention are peptides able to confer stability and solubility to an antibody including them.

According to the present invention such an object is achieved by a peptide characterized in having a sequence selected from the group consisting of the sequences reported in the annexed sequence listing from SEQ ID NO: 1 to SEQ ID N: 8, and in that included in a variable region of a scFv antibody make said antibody soluble and stable in cytoplasm medium.

In order to make said antibody stable and soluble the peptides having the sequences SEQ ID NO: 1 (H-FR1),

2

SEQ ID NO: 2, (H-FR2), SEQ ID NO: 3, (H-FR3), and SEQ ID NO: 4 (H-FR4), herein also denominated H-FR peptides, shall be included in the variable region of the heavy chain of said antibody (VH region), covalently linked to peptides having the sequences reported in the annexed sequence listing from SEQ ID NO:88 (H-CDR1), SEQ ID NO:89 (H-CDR2) and SEQ ID NO:90 (H-CDR3), herein also denominated H-CDR peptides, in the order

SEQ ID NO: 1 (H-FR1)-SEQ ID NO: 88-(H-CDR1)-SEQ ID NO: 2 (H-FR2)-SEQ ID NO: 89 (H-CDR2)-SEQ ID NO: 3(H-FR3)-SEQ ID NO: 90 (H-CDR3)-SEQ ID NO: 4(H-FR4)

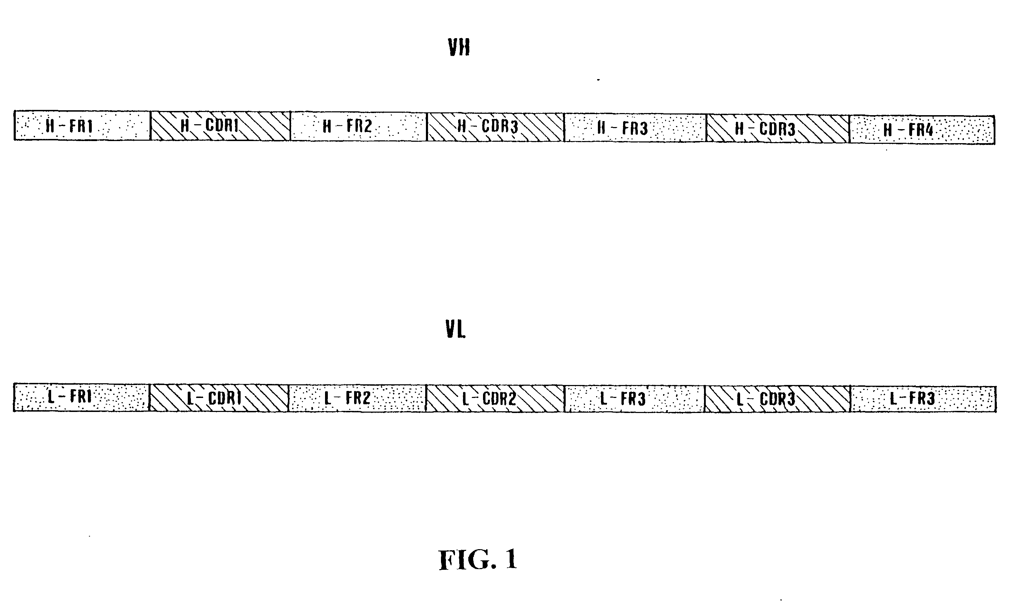

According to the arrangement shown in figure 1.

The peptides having the sequences SEQ ID NO: 5 (L-FR1), SEQ ID NO: 6, (L-FR2), SEQ ID NO: 7, (L-FR3), and SEQ ID NO: 8 (L-FR4), generally denominated L-FR peptides, shall instead be included in the variable region of the light chain of said antibody (VL region), covalently linked to peptides having the sequences reported in the annexed sequence listing from SEQ ID NO:91 (L-CDR1), SEQ ID NO:92 (L-CDR2) and SEQ ID NO:93 (L-CDR3), herein also denominated L-CDR peptides, in the order

SEQ ID NO: 5(L-FR1)-SEQ ID NO: 91-(L-CDR1)-SEQ ID NO: 6 (L-FR2)-SEQ ID NO: 92(L-CDR2)-SEQ ID NO: 7 (L-FR3)-SEQ ID NO: 93 (L-CDR3)-SEQ ID NO: 8 (L-FR4),

The H-FR and L-FR peptides are herein also generally denominated FRs (Framework Regions) peptides.

An advantage of the FRs peptides of the invention is the fact that they have surprisingly shown the ability of confer solubility and stability to any antibody including them in the above orders. Said stabilizing capability at least in the antibody of the scFv type, can render said antibody stable and soluble even in a reducing medium such as the cytoplasm.

The thermodynamic stability acquired by an scFv antibody with this arrangement according to the process described in Tavladoraki *et al*. 1999, is in fact of the same order of magnitude as that of the scFv(F8), with values of free energy ($\Delta$G) for denaturation and renaturation reaction *in vitro* of the same order of amplitude as those registered for scFv(F8) (Tavladoraki *et al*. 1999).

In particular at pH 7.2 the $\Delta$G values are greater than or equal to 7 Kcal/mole and in particular values encompassing the range between 7.8 and 10.5 Kcal/mole.

Analogous stability can be reached for Fv and Dab antibody including the FRs peptides of the invention.

In any case all the antibodies including the above peptides, are stabilized as an effect of the relevant inclusion in the variable region of the heavy chain or light chain. In particular this is shown on constructs utilizing as a backbone the constant regions of immunoglobulin like IgG or IgA, or also of antibodies like Fab, stability of said construct is in any case improved.

In a preferred embodiment of the H-Fr peptides of the invention, Xaa in position 24 of H-FR1 (SEQ ID NO: 1) is Ala; Xaa in position 12 of H-FR2 (SEQ ID NO: 2) is Leu; Xaa in position 2 of H-FR3 (SEQ ID NO: 3) is Pro, Xaa in position 3 of H-FR3 (SEQ ID NO: 3) is Asp; Xaa in position 13 of H-FR3 (SEQ ID NO: 3) is Arg, Xaa in position 18 of H-FR3 (SEQ ID NO: 3) is Asn; Xaa in position 20 of H-FR3 (SEQ ID NO: 3) is Leu; Xaa in position 12 of H-FR7 (SEQ ID NO: 7) is Arg, Xaa in position 15 of H-FR7 (SEQ ID NO: 7) is Phe.

According to the invention all these peptides can be produced by means of a series of conventional techniques known in the art such as for example recombinant DNA but also'constructions of synthetic polypeptides.

The relevant process for obtaining peptides stabilizing an antibody including them in a variable region comprising the steps of:

- deriving a polypeptides from a monoclonal antibody,
- mutagenizing said polypeptide by techniques, as hierarchical mutagenesis, error-prone PCR, DNA shuffling, or ribosome display,

is included in the object of the invention.

Alternatively said peptides can be derived by chemical synthesis, as for instance by translating a polynucleotide coding for them.

The H-CDR and L-CDR peptides of the invention are herein also denominated CDRs (Complementarity Determining Regions) peptides. CDRs peptides according to the present invention correspond to the parts of the regions VH and VL of the antibody which determine the binding specificity of the antibody. In particular H-CDRs peptides correspond to the parts of VH and L-CDRs peptides correspond to the parts of VL, which determine the binding specificity of the antibody.

The structural characteristics of the CDRs peptide are such that, when included in the polypeptides VH and VL covalently bound to the peptides FRs according to the above arrangement, they can give specificity to an antibody including them without modifying its stability and solubility.

Accordingly their sequences depend on the antigen that the antibody in which they are included specifically bind.

In order to derive polypeptides suitable as VH or VL region of an antibody can be used a process for deriving a polypeptide suitable as a variable region of an antibody and specific for a predetermined antigen comprising the step of:

- producing an antibody having as a variable region of the heavy chain the polypeptide having the sequence reported in the sequence listing as SEQ ID NO: 101, and as a variable region of the light chain the polypeptide having the sequence reported in the annexed sequence listing as SEQ ID NO: 102;
- putting in contact said antibody with said antigen and
- selecting the antibody binding said antigen,
- isolating the polypeptide of the variable region of the heavy chain and/or the polypeptide of the variable region of the light chain of the antibody binding said antigen.

This process, which is an object of the invention, in a preferred embodiment further comprises the step of

- sequencing the variable regions of said antibody binding said antigen.

The predetermined antigen of the process of the invention can be any antigen of interest in particular the viral ones, and specifically the antigens associated to HIV, HCV and HPV, as Tat, Rev, E7 or NS3 protein, which shall be considered preferred. Other antigens like, bovine seroalbumin, lysozime, AMCV virus, "tomato spotted wilt virus" or "cucumber mosaic virus", are used in a preferred embodiment of the invention.

Object of the invention are further the polypeptides obtainable by the above process, which are suitable for constituting the variable region of the heavy chain (VH) or the light chain (VL) of a given.antibody. Regarding the polypeptides of the invention, polypeptides suitable for constituting the VH region of an antibody, are also herein denominated VH polypeptides, region of an antibody are also herein denominated VL polypeptides.

In particular in this regard the following polypeptides VH and VL polypeptides are an object of the present invention:

- VH-F8 (SEQ ID NO: 31) and VL-F8 (SEQ ID NO: 32) which respectively constitute the VH and VL regions, of specific AMCV antibodies and in particular of scFv(F8);
- VH-LYS/P5 (SEQ ID NO: 33) and VL-LYS/P5 (SEQ ID NO: 34) that respectively constitute the VH and VL regions, of antibodies specific for the lysozyme;
- VH-LYS/11E (SEQ ID NO: 35) and VL-LYS/11E (SEQ ID NO: 36), which constitute the VH and VL regions respectively of specific antibodies for the lysozyme;
- VH-BSA/9F (SEQ ID NO: 37)and VL-HSA/9F (SEQ ID NO:38) which constitute the VH and VL regions respectively of antibodies specific for bovine seroalbumin;
- VH-TSWV(BR01)/6H (SEQ ID NO: 39) and VL-TSWV(BR01)/6H(SEQ ID NO: 40), which constitute the VH and VL regions respectively of antibodies specific for the "tomato spotted wilt virus";
- VH-TSWV(P105)/1C (SEQ ID NO: 41) and VL-TSWV(P105)/1C (SEQ ID NO: 42) which constitute the VH and VL regions respectively of antibodies specific for the "tomato spotted wilt virus";
- VH-CMV/4G (SEQ ID NO: 43) and VL-CMV/4G(SEQ ID NO: 44), which constitute the VH and VL regions respectively of antibodies specific for the "cucumber mosaic virus";
- VH-CMV/4B (SEQ ID NO: 45) and VL-CMV/4B(SEQ ID NO: 46), which constitute the VH and VL regions respectively of antibodies specific for the "cucumber mosaic virus"; and
- VH-CMV/2G (SEQ ID NO: 47) and VL-CMV/2G(SEQ ID NO: 48), which constitute the VH and VL regions respectively of antibodies specific for the "cucumber mosaic virus".

On the basis of the polypeptides of the invention specific CDRs peptides can be derived giving binding specificity to the antibodies including them.

Said CDRs peptides can be derived by a process for deriving a peptide conferring to an antibody binding specificity to a predetermined antigen comprising the step of:

- producing an antibody having as a variable region of the heavy chain the polypeptide having the sequence reported in the sequence listing as SEQ ID NO: 101, and as a variable region of the light chain the polypeptide having the sequence reported in the annexed sequence listing as SEQ ID NO: 102;
- putting in contact said antibody with said antigen and
- selecting the antibody binding said antigen,
- isolating the polypeptide of the variable region of the heavy chain and/or the polypeptide of the variable region of

EP 1 120 464 B1

the light chain of the antibody binding said antigen;
- isolating from said polypeptide the part conferring binding specificity to said antibody. In a preferred embodiment the above process further comprises the step of
- sequencing the variable regions of said antibody binding said antigen.

The antigen can be any antigen and preferably the ones described above.

Object of the present invention are also all the peptides obtainable by the above described process.

In particular the peptides CDRs which give the binding specificity for

- ACMV (artichoke mottle crinkle virus) in the antibody scFv(F8), having respectively the sequences SEQ ID NO: 9 (H-CDR1-F8), SEQ ID NO: 10 (H-CDR2-F8), SEQ ID NO: 11 (H-CDR3-F8), SEQ ID NO: 12 (L-CDR1-F8), SEQ ID NO: 13 (L-CDR2-F8), and SEQ ID NO: 14 (L-CDR3-F8);
- lysozyme (in particular lysozyme of chicken egg albumen), with the sequences SEQ ID NO: 97 (H-CDR1-LYS/P5), SEQ ID NO: 98. (H-CDR2-LYS/P5), SEQ ID NO: 15 (H-CDR3-LYS/P5), SEQ ID NO: 99 (L-CDR1-LYS/P5), SEQ ID NO: 100 (L-CDR2-LYS/P5), and SEQ ID NO: 16 (L-CDR3-LYS/P5), are a'further object of the present invention.

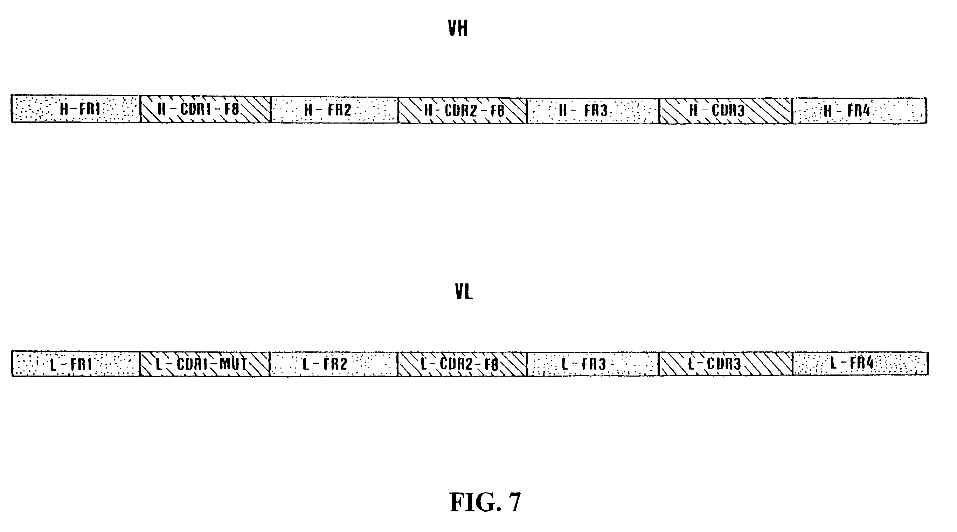

Object of the present invention are also all the peptides H-CDR3 and L-CDR3 as above defined which give binding specificity for antigens different from ACMV to the antibodies which include them in the VH and VL polypeptides substituting the original H-CDR3-F8 and L-CDR3-F8, together with other peptides H-CDR1, H-CDR2 and L-CDR2 of F8 and with the peptides L-CDR1-MUT (SEQ ID NO:76) included instead of the peptide L-CDR1-F8, according to the arrangement shown in figure 7.

Among these in particular object of the invention are the peptides H-CDR3 and L-CDR3 which give specificity for

- lysozyme (H-CDR3-LYS/11E, SEQ ID NO: 17 and L-CDR3-LYS/11E, SEQ ID NO: 18),
- bovine seroalbumin (H-CDR3-BSA/9F, SEQ ID NO: 19 and L-CDR3-BSA/9F, SEQ ID NO: 20),
- "tomato spotted wilt virus" nucleoprotein (H-CDR3-TSWV(BR01)/6H, SEQ ID NO: 21 and L-CDR3-TSWV(BR01)/6H, SEQ ID NO: 22; H-CDR3-TSWV(P105)/1C, SEQ ID NO: 23 and L-CDR3-TSWV(P1053/1C, SEQ ID NO: 24), and
- "cucumber mosaic virus" (H-CDR3-CMV/4G, SEQ ID NO: 25 and L-CDR3-CMV/4G, SEQ ID NO: 26; but also H-CDR3-CMV/4B, SEQ ID NO: 27 and L-CDR3-CMV/4B, SEQ ID NO: 28; and finally H-CDR3-CMV/2G, SEQ ID NO: 29 and L-CDR3-CMV/2G, SEQ ID NO: 30).

The term H-CDR3 in figure 7 indicates position of the peptides H-CDR3-LYS/11E, H-CDR3-BSA/9F, H-CDR3-TSWV(BR01)/6H, H-CDR3-TSWV(P105)/1C, H-CDR3-CMV/4G, H-CDR3-CMV/4B in the VH region. The term L-CDR3 indicates position of peptides L-CDR3-LYS/11E, L-CDR3-BSA/9F, L-CDR3-TSWV(BR01)/6H, L-CDR3-TSWV(P105)/1C, L-CDR3-CMV/4G and L-CDR3-CMV/4B in the VH region.

The L-CDR1-MUT peptide(SEQ ID NO:94) constitutes also an object of the present invention.

All the above CDRs peptides can be used for the detection of molecule of interest in a sample according to the techniques known in the art; said use shall be considered included in the object of the invention.

A further object of the present invention is given also by all the antibodies which include at least one of the above mentioned VH and VL polypeptides of the present invention.

In this regard, not only engineered antibodies of the scFv type, but also engineered antibodies of FAb, Fv, dAb type as well as all the immunoglobins, in particular those of the type IgG and IgA are object of the present invention.

In particular, object of the present invention are the engineered antibody scFv(F8) (SEQ ID NO: 49) and the scFv antibodies: scFv(P5) (SEQ ID NO: 50), scFv(LYS11E), scFv(BSA9F), scFv(BR01-6H), scFv(P105-1C), scFv(CMV-4G), scFv(CMV4B), and scFv(CMV-2G).

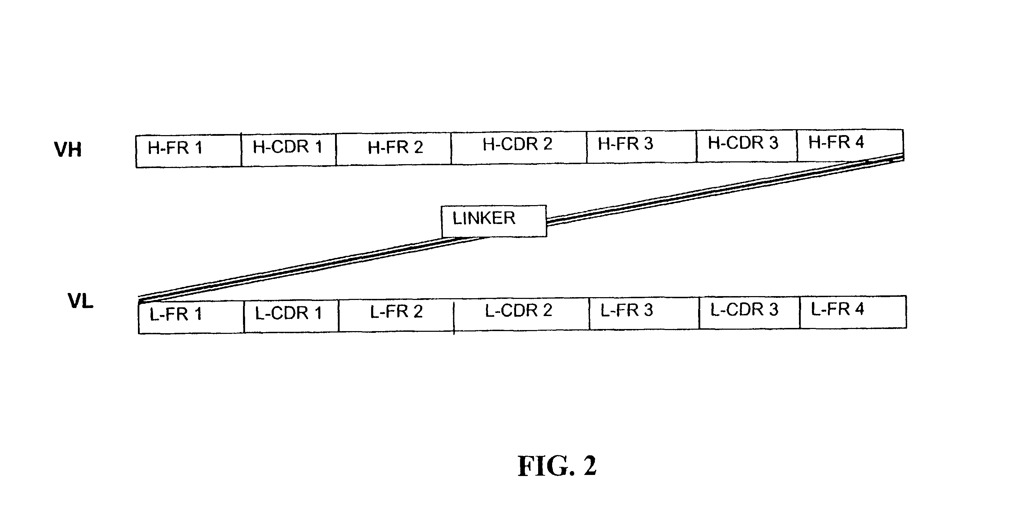

Such scFv antibodies are obtainable by connecting the respective VH and VL polypeptides with a linker covalently bound to the carboxy-terminal end of the VH polypeptide through its amino-terminal end, and to the amino-terminal end of the VL polypeptide through its carboxy-terminal extremity (see the diagram of in Figure 2).

This linker can be one of any of the linkers known in the art to be suitable for connecting VH and VL polypeptides. In particular can be the linker with the sequence given as SEQ ID NO: 51 in the list of sequences in the annex. All variants of the above mentioned peptides and polypeptides or the antibodies containing them must also

- a muteine of the above mentioned FRs and CDRs peptides, VH and VL polypeptides and antibodies including them,
- a molecule including at least one of the above mentioned FRs and CDRs peptides, VH and VL polypeptides and antibodies including them, or at least one of the relevant muteins,
- a fragment or a part of the above mentioned FRs and CDRs peptides, VH and VL polypeptides and antibodies including them, or at least one of the relevant muteines,

- a fragments or a part of the molecules which include at least one of the above mentioned FRs and CDRs peptides, VH and VL polypeptides and antibodies including them, or at least one of the relevant muteines,

all as essentially consisting of at least one of the above mentioned FRs and CDRs peptides, VH and VL polypeptides and antibodies including them.

Each molecule derived directly or indirectly from one of the peptides, polypeptides and antibodies of the present invention and in particular the ones having the sequences reported in the annexed sequences listing must be considered as essentially consisting of one of said peptides, polypeptides and antibodies, when:

- in the case of the variants of the above mentioned peptides/polypeptides, is contained in antibodies which are functional and stable at least in the cytoplasm of any cell;
- in the case of variants of the above mentioned antibodies is itself an antibody which is functional and stable at least in a cytoplasmic compartment.

For the purposes of the present application, a muteine of the above mentioned peptides/polypeptides/antibodies similar to the original peptide/polypeptide/antibody so that it can suggest a derivation of the second from the first.

A fragment of at least one of the above mentioned peptide/polypeptide/antibody or of one of its muteines or of a larger molecule larger includes it, is given for the purposes of the present invention by a molecule in which one or more amino acids of the original peptide or muteine have been truncated.

In regard to the larger molecule, which contains at least one of these peptides/polypeptides/antibodies and/or muteines, the portion of the molecule different from these peptides and/or muteines can be partly or totally coincident with the sequence of scFv(F8).

All these molecules can be produced by means of a series of conventional techniques known in the art such as for example recombinant DNA but also constructions of synthetic polypeptides.

A further object of the present invention is given by polynucleotides (both polydeoxyribonucleotides and polyribonucleotides) coding for the peptides/polypeptides of the present invention or for their variants. In particular polynucleotides having the sequences reported as SEQ ID NO: 52 (H-FR1-F8), SEQ ID NO: 54 (H-FR2-F8), SEQ ID NO: 56 (H-FR3-F8), SEQ ID NO: 58 (H-FR4-F8), SEQ ID NO: 60 (L-FR1-F8), SEQ ID NO: 62 (L-FR2-F8), SEQ ID NO: 64 (L-FR3-F8), SEQ ID NO: 66 (L-FR4-F8), SEQ ID NO: 68 (H-CDR1-F8), SEQ ID NO: 70 (H-CDR2-F8), SEQ ID NO: 72 (H-CDR3-F8), SEQ ID NO: 74 (L-CDR1-F8), SEQ ID NO: 76 (L-CDR2-8), and SEQ ID NO: 78 (L-CDR3-F8), SEQ ID NO: 80 (VH-F8), and SEQ ID NO: 82 (VL-F8), SEQ ID NO: 95 (L-CDR1-MUT), are included in the object of the present invention.

Included in the present invention are also polynucleotides coding for the engineered antibodies obtained using the peptides/polypeptides of the present invention and in particular those with sequences given as SEQ ID NO: 84 (scFv(F8) and SEQ ID NO: 86 (scFv(P5)).

A further object of the present invention is given by the peptides/polypeptides, engineered antibodies particular for the therapy of all the pathological states associated with an accumulation of at least one molecule in an intra or extra cellular environment. Here in particular, reference is made to infectious, tumor, metabolic and immunitary (especially auto-immunitary) pathologies. In regard to the infectious pathologies, reference is made in particular to those associated with viruses, either in animals (see for example pathologies associated with the HIV, in particular HIV-1, HPV, Herpes Virus or HCV virus in humans), or in plants (see for example the pathologies associated with the CMV or TSWV viruses in tomatoes).

Further also the pharmaceutical compositions which include a therapeutically effective amount of at least one of the above mentioned peptides, polypeptide, antibodies and/or variant thereof, and/or a therapeutically effective amount of at least one of the above mentioned polynucleotides and a pharmaceutically suitable vehicle.

This pharmaceutically acceptable vehicle carrier or auxiliary agent can be any vehicle carrier or auxiliary agent known in the art as being suitable for preparing pharmaceutical compositions or material containing the above mentioned molecules. In particular according to the invention such a carrier can be the liquid or solid carrier which are used or in any case known by a person skilled in the art to be suitable with protein and antibody.

Object of the present invention is also a method for the treatment of pathologies associated to the accumulation of a molecule inside or outside human, animal cell, comprising the step of

- administering a therapeutically effective amount of above mentioned antibody or variant thereof to a subject in need thereof.

In particular said administration can be carried out parenterally for treating cancer and pathologies associated thereof, or by oral route as passive immunotherapy, according administration techniques known to a person skilled in the art.

Object of the present invention is also a method for the treatment of pathologies associated to the accumulation of a molecule inside or outside human, animal or plant cell, comprising the step of

- administering a therapeutically effective amount of above mentioned polynucleotides or variant thereof coding for the antibodies of the present invention to a subject in need thereof.

Said administration shall be carried out by inserting said polynucleotides in vectors suitable for trasfection. According to the present invention any of the vectors, in particular the viral ones, currently adopted by a person skilled in the art for treating subject in need thereof, can be used to this purpose.

A further object of the invention is the use of the above mentioned antibodies and/or polynucleotides or variants thereof for the treatment of pathologies associated to accumulation of a molecule inside or outside the human, animal or plant cell.

The use of these above mentioned peptide/polypeptide/antibody or a variant thereof for diagnosis of these pathologies is also included in the object of the present invention.

In particular such molecules can be used in diagnostic applications fused to genes encoding for protein having an enzymatic activity (i.e., alkaline phosphatase) or reporter proteins such green fluorescent protein. Other reporter genes or proteins known in the art as suitable in diagnostic application can be used as well.

Among the diagnostic application wherein the antibodies of the present invention can be used imaging is particularly preferred.

Object of the present invention is a diagnostic kit including the above molecules as reagents, and in particular a diagnostic kit for infectious pathologies characterized in that it includes at least one composition including at least one of the above mentioned polynucleotide, peptide, polypeptide, antibody and/or a variant thereof.

Included in the object of present invention is also the use of the above mentioned antibodies for identifying new molecules and/or the relevant function.

Such identification can be carried out for instance according to techniques known in the art referred to genomics and proteomics.

In particular object of the present invention is the use of the above mentioned peptide/polypeptide/antibody or a variant thereof, for deriving molecules to be used in therapy of infectious pathology, and the relevant kit including at least one of such peptide/polypeptide/antibody or a variant thereof as a reagent.

Further object of the present invention is also a composition suitable in experimental applications including at least one of the above mentioned molecules and a vehicle chemically compatible with them.

This chemically compatible vehicle can be any vehicle known in the art as being suitable for pharmaceutical compositions or material containing the above mentioned molecules.

In particular according to the invention such a carrier can be the liquid or solid carrier which are used or however known by a person skilled in the art to be suitable with protein and antibody.

The invention will be better described with the help of the figures in the annex.

Description of the figures

Figure 1 shows the arrangement of the peptides of the present invention in the VH and VL polypeptides of an antibody according to the present invention.

Figure 2 shows the connection of the VH and VL polypeptides of the present invention in antibodies of the scFv type.

Figure 3 shows the summary diagram of the modifications of the sequence of scFv(F8) which do not alter the actual characteristics of stability of the antibody. The regions (FRs and CDRs) are individualized according to AbM (Oxford Molecular Ltd) and the numbering is according to Kabat (Kabat *et al* 1991).

The residues shown in gray colored squares are the residues which must remain unchanged, the residues shown in black colored squares are the residues which can be substituted with any amino acid, the residues shown in white squares are modifiable as follows:

- the residue VH 24 is substitutable with residues of equivalent chemical nature;
- the residue VH 47 is substitutable with residues of equivalent chemical nature;
- the residue VH 52 is substitutable with residues of equivalent chemical nature or different chemical nature, or can be eliminated by deletion;
- the residue VH 60 is substitutable with residues of equivalent chemical nature or different chemical nature;

- the residue VH 61 is substitutable with residues of equivalent chemical nature or different chemical nature;
- the residue VH 71 is substitutable with residues of equivalent chemical nature;
- the residue VH 76 is substitutable with residues of equivalent chemical nature;
- the residue VH 78 is substitutable with residues of equivalent chemical nature;
- the residues VH 100-100G are substitutable with residues of equivalent chemical nature or different chemical nature, or can be eliminated by deletion;
- the residues VL 27C-29 are substitutable with residues of equivalent chemical nature or different chemical nature, or can be eliminated by deletion;
- the residue VL 68 is substitutable with residues of equivalent chemical nature or different chemical nature;
- the residue VL 71 is substitutable with residues of equivalent chemical nature or different chemical nature.

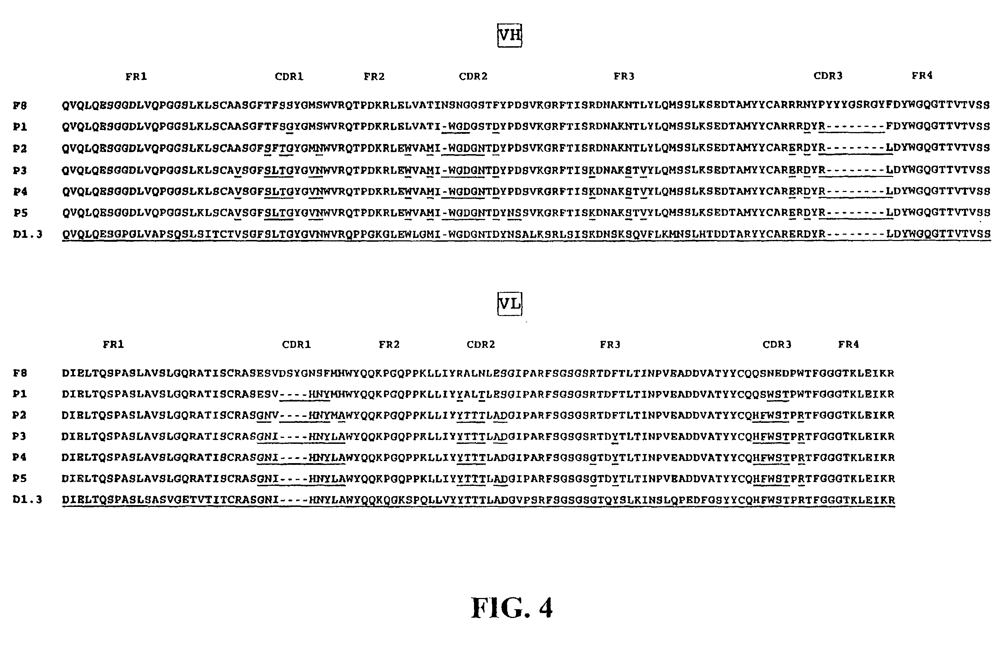

Figure 4 shows a summary diagram of the strategy of mutagenesis adopted by means of rational substitutions of the VH and VL polypeptides of the antibody scFv(F8). The actual amino acidic residues of the sequence of scFv (F8) are shown in normal characters, the amino acids of the sequence of scFv(D1.3) (Bhat *et al*. 1990) are shown in underlined characters. The sequences are shown for all the products of intermediate mutagenesis (P1, P2, P3 and P4) and for the last product of mutagenesis (P5), so that the modifications (substitutions and deletions) effected on the original sequence of scFv(F8) are marked by underlining.

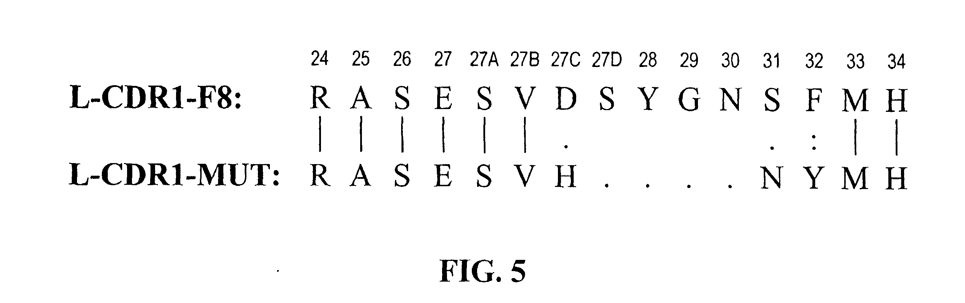

Figure 5 shows the modification effected on the CDR1 of the VL according to the approach of casual mutations for the derivation of mutants of scFv(F8).

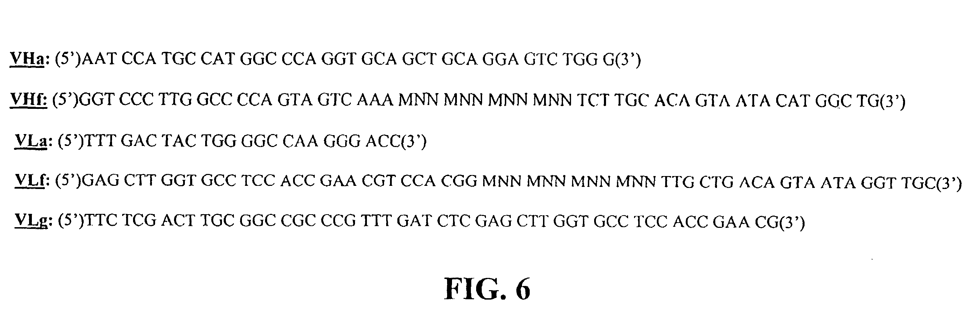

Figure 6 shows the oligonucleotides used as primers for the construction of the "monoscaffold" library as described in the examples. The term N indicates any nucleotide, the term M indicates the dATP or dCTP nucleotide.

Figure 7 shows the layout of the peptides of the present invention in the VH and VL polypeptides of antibodies in which the binding specificity is in particular given by the H-CDR3 and L-CDR3 peptides.

Detailed description of the invention

As mentioned above the peptides of the present invention are capable of rendering the antibodies including them stable even in a reducing environment.

Regions determining the complementarity (CDRs) with the antigen and framework regions (FRs) of scFv(F8) antibody have been identified by sequencing according to criteria known in the state of art.

ScFv(F8) has been used as a scaffold onto which new specificities have been engineered either by loop grafting ("rational approach") or by mutation and selection through repertoire generation ("molecular evolution") Antibodies deriving from site-directed mutagenesis have been analyzed to verify maintenance of stability and solubility in cytoplasmic environment and the acquirement of binding specificity different from AMCV.

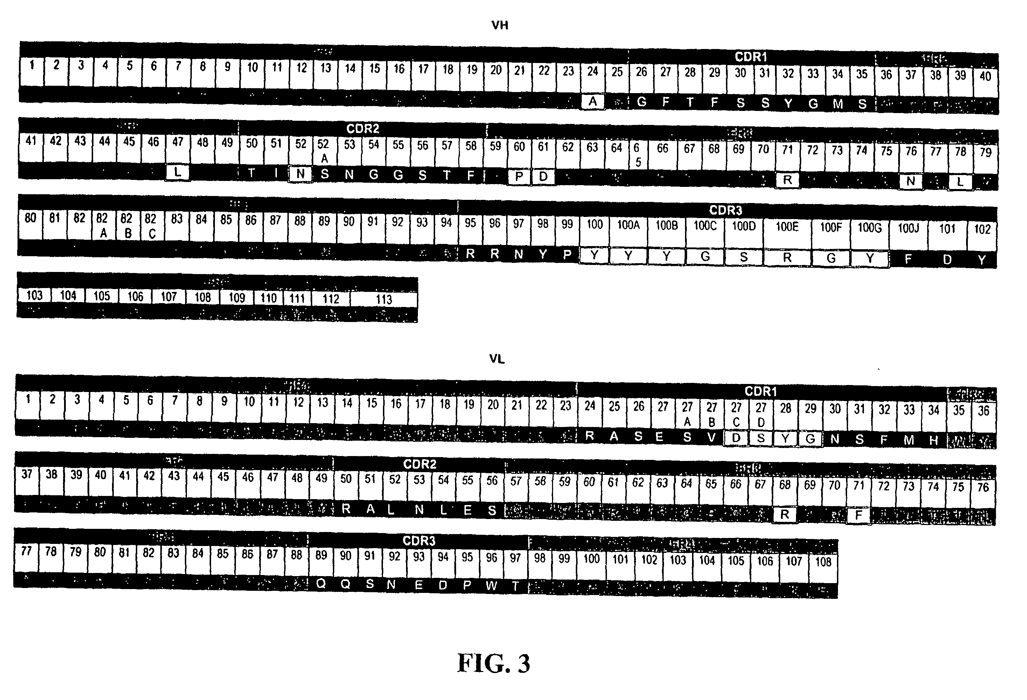

In the "rational approach" residues of both CDRs and FRs regions were substituted with residues of scFv (D1.3) that recognizes lysozyme, whereas in "molecular evolution" residues of the CDR3 regions were casually substituted and the binding properties of the resulting scFv were checked. These experiments demonstrated that scFv (F8) scaffold tolerates substitution of defined residues in both CDRs and FRs regions, which can be redefined in terms of stability preservation of the antibody scFv(F8) (see in particular figure 3).

*"Rational substitution" approach*

The inventors substituted scFv(F8) aminoacids with aminoacids of the CDRs and FRs of scFv(D1.3) which recognizes lysozyme. These substitutions were performed in a rational way, modifying each residue on the basis of a theoretical design formulated by means of molecular "modelling".

According to this approach extensive aminoacid modifications have been made, including all CDRs of VH and VL. These regions were substituted (by means the technique known in art as "grafting") with the corresponding regions of another antibody of "normal type" (which does not show particular stability or cytoplasmic functionality), which binds another target molecule which can be lysozime, bovine seroalbumin, nucleoprotein of tomato spotted wilt virus cucumber mosaic virus, or any other target molecule of interest.

The diagram shown in figure 4 summaries the strategy of mutagenesis adopted for scFv(F8) antibody grafting.

The analysis of mutagenised products, in the specific case shown in the figure referred to mutagenesis direct to have an antibody binding lysozime, demonstrated the success of grafting strategy. In fact, the resulting chimerical antibodies (named P2, P3, P4 and P5) were able to specifically recognise lysozime, as predicted by the molecular design. At the same time the capacity of scFv(F8) scaffold to support extensive mutations was demonstrated, particularly at the level of the CDRs, without affecting molecular stability. In fact, the new binding molecules obtained with this approach showed to be soluble and functional in the reducing environment of cell cytoplasm as well as the cognate scFv(F8).

As a result, except for few FRs residues modified to allow the correct folding of the regions involved in the new antigen recognition, the support structure of scFv(F8) was not substantially altered. The structure capable of functioning as "central nucleus" was identified, on which it is possible to graft, by means of protein engineering, sequences capable to confer binding ability for new antigens. This central nucleus is composed of the FRs peptides described in the summary of the invention.

The properties of these mutant molecules were verified by the inventors by means of techniques of analysis of the expression in both periplasm and cytoplasm of *Escherichia coli*, better explained and exemplified in the examples. In particular, the analysis of the expression in the bacterial periplasm has enabled to evaluate the ability of the grafted antibodies to recognise the new antigen. The expression in the bacterial cytoplasm has instead permitted to analyze protein solubility and functionality in reducing environment.

In the case of antibodies mutagenized for lysozime specificity the ELISA (Enzyme Linked Immunosorbent Assay) test carried out on periplasmic extracts normalized for total protein content has shown that all the products of mutagenesis, with the exception of P1 (the mutant with the least number of substitutions), were able to recognise lysozime. The P3 mutant, which presents the CDRs of scFv(D1.3) and some substitutions in the frameworks, showed the highest binding activity. Lysozime recognition observed for P3 was slightly lower than those showed by scFv(D1.3) (about 15 % less). These data were combined with the Western blot analysis which showed comparable expression levels of the various products of mutagenesis and between mutagenesis products and the original scFv(F8) and scFv (D1.3). This indicate that the differences of signal observed in ELISA are exclusively due to different specificity for the antigen, rather than to different expression levels.

In regard to the expression in the bacterial cytoplasm, ELISA carried out on extracts normalized for total proteins gave a higher activity for P5 mutant, characterized by the highest number of aminoacidic substitutions. A poor binding activity, in each case higher than that measured for scFv(D1.3), was also registered for P3 and P4.

Even in this case Western blot analyses showed an equivalent expression of the scFv mutants expressed in the cytoplasm, confirming that, once again, the differences of ELISA signal are attributable to the different ability of recognition of the lysozyme.

"*Molecular evolution*" *approach*

On the basis of the indications obtained from the rational substitution approach, a library of new molecules, derived from scFv(F8) has been construed.

The starting point for library construction was scFv(MUT-VL1), a derivative of scFv(F8) antibody, which was modified in the CDRs. The CDR1 of the VL domain was shortened and partially modified according to a modeling-assisted design. Moreover, 9 aminoacids were removed from the unusually long CDR3 of the VH domain of the original scFv (see figure 5) .

Structural variability was introduced by targeted random PCR-mutagenesis in four aminoacid positions in the CDR3 of both VH and VH domains. Degenerated oligonucleotides, randomizing residues between and including 95 and 98 of VH and 91 and 94 of VL , were used. After mutagenesis, a repertoire with an estimated diversity of $5 \times 10^7$ different phage clones was obtained.

The pool of molecules thus obtained was mounted on phage in a library for the selection on antigens different from AMCV, originally recognized by scFv(F8). This "library", constructed using as a basic structure a single starting antibody ("monoscaffold library"), provided antibodies with different specificity, which at the same time retain the peculiar characteristics of stability of scFv(F8) molecule (cytoplasmic solubility, denaturation and renaturation *in vitro*). These conclusions were arrived at by guanidinium chloride denaturation/renaturation studies of isolated scFv molecules. The expression of the same ScFv fragments in the *Escherichia coli* cytoplasm as soluble and functional molecules confirmed the stability of these proteins also in *in vivo* system.

Further, for some of them (scFv(CMV)4B and scFv(CMV) 4G), it has been shown that they are expressed as soluble and functional molecules also in the plant cytosol. The presence of scFv(CMV)4B or scFv(CMV)4G antibodies in the soluble fraction of potato virus X (PVX)-infected tobacco plants shows that these molecules, differently from most antibodies, are folded and stable in the plant cytoplasm. Identical results were achieved in transgenic tomato plants, confirming the capability of a correct folding in a reducing environment. These antibodies interfere with CMV assembly and/or replication, providing an engineered resistance to the virus ('plantibody-mediated resistance').

CDR3 sequences of VH and VL domains of the antibodies isolated from the library and characterized (see table I infra) showed that residues substitution in the above mentioned positions is totally casual. Moreover, aminoacid composition and distribution in mutated CDR3 indicated that charge and hindrance of aminoacids do not substantially affect the folding and solubility of these scFv fragments.

The data obtained from the construction and from the analysis of the "monoscaffold library" has shown the possibility of using the scaffold of scFv(F8) to construct a pool of polyvalent antibodies with the same intrinsic stability as the original antibody scFv(F8). This library is destined to all applications in which the expression of antibodies in the cytoplasm is required or

where particular characteristics of molecular stability are essential.

As a result of these experiments, the stable antibody molecules with a new binding specificity and in particular the ones described in the summary of the invention have been obtained.

*Conclusions*

The results of all these experimental approaches are summarized in the diagram shown in the following table I.

In this table the results and the information obtained as a result of the derivation of mutants through the approach of casual substitutions are reported. The non polar residues are shown with double underlined characters, the residues with polar R group with no charge are shown in normal characters; the acid residues (negative charge) are shown with dashed underlining and the basic residues (positive charge) are shown with single underlining.

Table I

| Clone | Specificity | VH CDR3[a] | VL CDR3[b] |
|---|---|---|---|
| **scFv(F8)** | AMCV | RRNYPYYYGSRGY | SN ED |
| scFv(LYS-11E) | Lisozyme | TRPY | VTYK |
| scFv(BSA-9F) | BSA | ERWD | ALS P |
| scFv(BR01-6H) | Nucl. ofTSWV from BR01 | NWRR | GASI |
| scFv(P105-1C) | Nucl. of TSWV fromP105 | GRHK | YGRR |
| scFv(CMV-4G) | CMV | NNWS | GQRK |
| scFv(CMV-4B) | CMV | NNYS | GRR A |
| scFv(CMV-2G) | CMV | VTYN | SRRP |

*a* indicates: when referred to scFv(F8), the positions from residue 95 to residue 100J; when referred to clones isolated from "monoscaffold library" the positions from residue 95 to residue 98 (numeration according to Kabat *et al*., 1991).

*b* indicates positions from residue 91 to residue 94 (numeration according to Kabat *et al*., 1991).

On the basis of the above results the peptides, polypeptides, antibodies reported in the summary of the invention have been obtained following the process reported also therein.

The antibodies obtained thereby in particular have been proved for the improved stability, and in the antibodies of the type FAB, Fv, dAb, IgG or IgA have in particular shown an improved stability due to the presence of the peptides of the invention in the VH and VL regions.

Up until now a general description of the present invention has been given. With the help of the following examples, a more detailed description will now be provided, in order to clarify scope, characteristics, advantages and operating method.

Examples

Example 1: Determination of the sequence of scFv(F8)

The single chain antibody scFv(F8) derives from a MAb (secretion from a hybridoma) of the class IgG2b directed against the coat protein of the plant virus AMCV (artichoke mottle crinkle virus). To obtain this antibody, Balb/c mice were immunized with the purified virus. The techniques for isolating the lymphocytes and obtaining the hybridoma were the standard ones reported in literature (Harlow and Lane 1988). The hybridoma line expressing this antibody was selected by ELISA, on the basis of its high affinity for the antigen. To isolate the genes of the heavy (H) and light (L) chain of this antibody, the complete cDNA were selected according to published protocol (Tavladoraki *et al*. 1993). The variable regions (VH and VL) were amplified by means of PCR (polymerase chain reaction) using universal primers for the variable regions and successively cloned in different types of vectors (Tavladoraki *et al*. 1993). The sequence was determined according to the Sanger method (Sambrook *et al* 1989) following the protocol of the Sequenase kit (USB).

Example 2: Determination of the site specific mutagenesis in the rational approach

The mutagenesis was carried out on the plasmid pMUT-VL1, containing the gene that codes for an antibody derived from the scFv(F8), from which only the CDR1 of the VL is differentiated for, partially modified in the aminoacidic composition and reduced by four amino acids.

The Stratagene ("Quick Change Site-directed Mutagenesis Kit") system of mutagenesis was used following the indications provided by the manufacturer. This system is based on the enzymatic extension of two complementary primers containing mutated sequences, using pMUT-VL1 as a template. The plasmid was denatured allowing the annealing of the two oligonucleotides to the opposite DNA strands, so as to prime the extension reaction of the Pfu DNA polymerase. The result is a plasmid with a double helix that has incorporated the oligonucleotides causing the desired mutation.

The bacterial stock of *E. Coli* XL1-Blue used for the purification of the plasmidic vector has methyltransferase activity, therefore the DNA plasmid extracted from bacteria results methylated. On the contrary the mutant plasmids obtained by extension of the Pfu DNA polymerase, do not contain methylations. This has enabled the selection of mutated plasmids by digestion of the products of reaction with the endonucleasic enzyme DpnI that recognizes frequent specific sequences of methylated DNA in the parent plasmid. The products of the reaction were transfected in *E. Coli* XL1-Blue where the "nick" sites, produced during the synthesis *in vitro* of the mutant plasmid are phosphorylated by the cellular repair systems.

The mutagenesis reaction was carried out in 25 $\mu$l containing: 2.5 $\mu$l reaction buffer 10x, 2.5 mM dNTP, 10 ng DNA template, 62,5 ng each primer, 1.25 U Pfu DNA polymerase (Stratagene).

The conditions adopted were the following: denaturation at 95 °C of 30"; 18 cycles: denaturation at 95 °C for 30", annealing at 55 °C for 1' and enzymatic extension at 68 °C for 7'; 5' at 68 °C to complete the extension. The reaction was performed using a Perkin Elmer/Cetus apparatus.

The products of the reaction were incubated for one hour at 37 °C with 5 U of the restriction enzyme DpnI (Stratagene) and analysed for electrophoresis on agarose gel.

The products of mutagenesis were cloned in pGEM-7Zf(+) vector (Promega) and transfected in *E. Coli* XL1-Blue, according to the conventional methods of molecular biology. Mutagenesis was verified by sequencing.

Proteins expressed by mutated sequences were analysed after cloning in the expression vector pDN332 (Neri *et al*. 1996) and induction of expression in bacteria, according to the technique described in the following examples.

The functionality of the antibodies and the solubility in cytoplasmic reducing environment were analysed by means of Western blotting and ELISA, as described below.

Example 3: Extraction of the protein expressed in *E. Coli* periplasm

Single colonies of *E. Coli* HB2151 expressing scFv antibodies were inoculated in 50 ml SB culture medium containing 100 $\mu$g/ml ampicillin and 2% glucose and grown for 16 hours at 37 °C. This preculture was diluted up to 1 litre with fresh SB medium and underwent shaking at 37°C for two additional hours (until $A_{600\,nm}$=0.7-0.8). Then cells were centrifuged at room temperature at 3000 g for 15'.

The induction of the lacZ promoter was obtained by resuspending the bacterial precipitate in 1 litre SB medium containing 100 $\mu$g/ml ampicillin and 1 mM IPTG and shaking at 30 °C for 3 hours. The culture was then centrifuged at 3000 g for 15' at 4 °C to precipitate the cells.

Soluble proteins were extracted from bacterial periplasm by osmotic "shock". The pelleted cells were resuspended in 15 ml of TES buffer solution (0.5 M saccarose, 0.2 M Tris-HCl, 0.5 mM EDTA, pH 8.0) containing protease inhibitors ("Complete Mini", Boehringer).

Then 22.5 ml of 1:5 diluted TES and protease inhibitors were added. The bacterial suspension was subjected to a slow rotation for 15' at room temperature. The extract was then centrifuged at 15000 g, at 4 °C for 20' to recover periplasmic proteins present in the supernatant.

Example 4: Expression of proteins in *E. Coli* cytoplasm

*a. Preparation of intracellular constructs*

To obtain the expression of scFv fragments in the bacterial cytoplasm signal sequenceless constructs were prepared. The sequence, encoding the scFv(F8) devoid of the PelB secretion signal (Tavladoraki *et al*., 1993), was cloned in HindIII-NotI sites of pDN332 phagemid, yielding the phagemid named pDN-F8intra. Constructs of *scFv* genes destined to intracellular expression were obtained by substituting a *Pst*I-*Not*I 744 bp fragment of pDN-F8intra, corresponding to the scFv(F8) gene, with the analogue *Pst*I-*Not*I restriction fragments obtained by digestion of mutated scFv genes. Plasmids containing the signal sequenceless version of the scFv genes were used to transform *E. coli* HB2151.

*b. Expression of recombinant protein in E. Coli*

Fifty ml of 2×TY-AG were inoculated with an overnight culture at $A_{600nm}$ =0.05. Cells were grown at 37°C until $A_{600nm}$=0.6, then they were pelleted and resuspended in 50 ml SB medium containing 100 $\mu$g/ml ampicillin and

0.4 mM IPTG. After 3 h induction at 30°C, bacteria were collected in 2.5 ml extraction buffer (20 mM Tris-HCl, 2 mM EDTA, 10 mM iodacetamide, pH 8), frozen/thawed at -20°C, and then sonicated for 3 min at 150 Watt power (Soniprep 150, Sonyo) on ice. The soluble fraction was collected by centrifugation for 30 min at 18000 g.

The pellet, containing inclusion bodies, was resuspended in electrophoresis buffer (20 % glycerol, 2% SDS, 0.06 M Tris-HCl pH 6.8, 0.02 % bromophenol blue, 5% β-mercaptoethanol), boiled for 5' and the supernatant obtained after a brief centrifugation was loaded on polyacrylamide gel.

Example 5: Analysis of recombinant antibodies

*a. Determination of the protein concentration*

The concentration of the total protein present in the extracts was determined using the BioRad reagent and bovine seroalbumin as a standard.

The concentration of the scFv after purification was calculated on the basis of absorbance at 280 nm. After electrophoretic separation protein bands were visualised with silver nitrate or Coomassie blue staining methods.

*b. Electrophoresis on polyacrylamide denaturing gel (SDS-PAGE)*

The analysis of the antibody fragments obtained after purification was carried out by means of SDS-PAGE according to the protocol known in art. The separation gel was prepared using polyacrylamide (acryilamide/biacrylamide 29:1) at 12% final concentration, in the presence of 2% SDS. Loading buffer (final concentrations: 10% glycerol, 0.06 M Tris-HCl pH6.8, 0.025% bromophenol blue, 2% SDS and 5% β-mercaptoethanol) was added to the samples before boiling for 5'. Electrophoresis was carried out using MiniProtean (BioRad) apparatus at 100 Volt.

*c. Western blot analysis*

After separation on SDS-PAGE, the proteins were then electro-transferred from the gel onto a nitrocellulose membrane (Hybond-C Super, Amersham) at 100 Volt for one hour at 4 °C.

The nitrocellulose membrane was then blocked in PBS buffer (0.2 M $NaH_2PO_4$, 0.2 M $Na_2HPO_4$, 0.15 M NaCl) containing 0.1% Tween-20 (PBST) and 4 % skimmed milk, at 4 °C for 16 hours.

After three washes with PBST, respectively of 30", 5', 5', and a wash of 5' in PBS, the membrane was incubated in PBS, 2% skimmed milk (PBSM) containing 2.5 μg/ml monoclonal antibody anti-Flag M2 (Sigma), for 2 hours at room temperature. The membrane was washed and immunodetection was realised by incubation for 1 hour at room temperature in PBSM containing biotinylated anti-mouse IgG antibody (Amersham), followed by incubation in PBSM with streptavidin-horseradish peroxidase conjugate (Amersham). Signal development was obtained by enhanced chemioluminescence (ECL Plus, Amersham).

*d. ELISA test*

Immunoplates (Maxisorp, Nunc) were coated with antigens (100 μg/ml lysozyme (Sigma)and 3 μg/ml AMCV) in 100 μl carbonate buffer (50 mM $NaHCO_3$ pH9.6), at 4 °C for 16 hours.

After 3 washes with PBST and 1 wash with PBS, the plates were blocked with PBSM for 2 hours at 37 °C. Each well was then washed and filled with 80 μl extract from induced bacterial cultures and 20 μl PBSM containing 2.5 μg/ml anti-FlagM2 (Sigma). The plates were incubated for 16 hours at 4 °C and washed. 100 μl PBSM were then added to each well containing a goat anti-mouse antibody conjugated to the peroxydase (KPL). The plates were incubated at 37 °C for one hour and washed. For signal development 100 μl of 1:1 solution of peroxydase substrate, ABTS [acid 2'2'-azinebi(3-ethylbenzthiazolin) sulphonic]: $H_2O_2$ (KPL) were used. Signals were measured at 405 nm absorbance by means of ELISA reader (Labsystem Multiscan Plus).

Example 6: Construction and cloning of the "monoscaffold" library

As a template for the construction of the phage library the plasmid pMUT-VL1 described in example 2 was used. The library was obtained by introducing variability in the CDR3 of the VH and VL, through the random modification of the sequence encoding four aminoacidic residues in the positions indicated in table I. For this purpose, partially degenerated oligonucleotides were synthesized, designed to avoid the introduction of transcription stop codons and to reduce the length of the CDR3 of the VH from 13 to 4 aminoacids. The mutagenesis was carried out by means of PCR, independently amplifying the coding sequences for the VH and VL domains, using respectively, the primers VHa and VHf and VLa and VLf (Fig. 6). The reaction of PCR was prepared as follows: 300 ng pMUT-VL1, 0.4 mM sense primer (VHa or VLa), 0.8 µM degenerated antisense primer (VHf or VLf), 250 µM of each dNTP, in 50 µl of incubation buffer 1x Appligene, (Oncor); 2.5 U of Taq DNA Appligene polymerase, (Oncor) were added at 94 °C (hot start). The amplification reaction was performed according to the following program: 94 °C for 3 minutes; 25 cycles: 94 °C for 1 minute, 60 °C for 1 minute, 72 °C for 1 minute; 72 °C for 2 minutes. Amplification products were purified from agarose gel, using the QIAquick Gel Extraction Kit (QIAgen), eluting in 30 ml of 3 mM Tris/HCl pH 8.0. The assembly of the scFv antibodies was carried out by means of PCR under the same conditions as described above, using 10 µg of the purified VH and VL fragments, 0.8 µM sense primer (VHa) and 0.8 µM antisense primer (VLg), in a final volume of 500 µl subdivided into 10 reactions of 50 µl. The assembled scFvs were purified using the QIAquick PCR Purification Kit (QIAgen). After *Nco*I-*Not*I digestion, the entire product of assembly was cloned in the vector pDN332.

Ligation products were transfected by electroporation in the *E. Coli* TG1 strain, adopting the following conditions: 200 ohm, 25 mF, 2.5 kVolt. The transformants were selected on 2xYT + 2 % glucose + ampicillin 100 g/ml. The phages were prepared according to the protocol described by Nissim *et al.* (1994).

Example 7: selection of the mutants from the "monoscaffold" library and analysis of their properties

*a.Selection of the library*

Antigens immobilization on immunotubes (Maxisorp, NUNC) was carried out in PBS (8mM $Na_2HPO_4 \cdot 12H_2O$ + 1mM $NaH_2PO_4 \cdot H_2O$ + 0.15M NaCl) or in carbonate buffer 50mM (CB), at concentration varying between 10 and 100 µg/ml depending on the antigen, incubating for 16 hours at room temperature or at 4 °C.

$10^{12}$-$10^{13}$ phages in 4 ml of PBS + 2% milk (PBS-M) were used for each selection cycle, incubating for 2 hours, with the first 30 minutes of slight agitation. After 10-15 washes with PBS + 0.1% Tween20 and 10-15 washes with PBS, the elution was carried out with 1 ml of 100 mM triethylamine, immediately neutralized with 0.5 ml of 1 M Tris/HCl pH 8.0. The phage suspension was then used to infect 10 ml of *E. Coli* TG1 strain (37 °C for 30 minutes) exponentially growing. After centrifugation, the bacteria were resuspended and plated on plates containing agar medium: 2xYT + 100 µg/ml ampicillin + 1% glucose (2xYT-AG).

The characterization for binding ability of single clones deriving from the last panning cycles was carried out in some cases on clones expressed in the form of phage and in others expressed as soluble scFv.

In the case of phage clones, 96 single colonies obtained from the last selection cycle were inoculated in 150 ml of 2xYT-AG and grown at 30 °C in agitation for 16 hours. Then an aliquot of 10 ml of each pre-culture was used to inoculate 150 ml of 2xYT-AG until reaching exponential growth. The production of phages was obtained by infecting with about $10^{11}$ t.u. (transforming units) of 'helper' phage VCSM13 (Stratagene) and incubating at 37°C for 30 minutes. The infected bacteria were centrifuged, resuspended in 150 ml of 2xYT + 100 µg/ml ampicillin + 25 µg/ml kanamicine and incubated for 16 hours at 30°C. The culture supernatants were analyzed by ELISA, with antigens immobilized under the same conditions used for the immunotubes. After incubating 2 hr at 37°C, a peroxidase-conjugated monoclonal antibody anti-M13 (Pharmacia) was used 1:5000 in PBS-M, 1 hour at 37°C. The colorimetric reaction was developed using the "ABTS peroxidase substrate system" (KPL). Positive clones obtained from this preliminary selection were further analyzed in order to verify the functionality as soluble scFvs. For this purpose, plasmids from positive clones were extracted by means of the QIAprep Spin Miniprep (QIAGEN), sequenced and transfected in the *E. Coli* HB2151 strain. Competent bacteria were made by resuspension at 0°C in TSS (for 100 ml: 1 gr bacto-triptone, 0.5 gr yeast extract, 0.5 gr NaCl, 10 gr PEG 3350, 5 ml DMSO, 50 mM $MgCl_2$, pH6.5). DNA plasmid (1-5 ng) was added to 1 ml of competent cells and incubated on ice for 45 minutes. After a brief shock at 42 °C for 2 minutes, 1 ml of LB was added, then the cells were placed in agitation at 37°C for 1 hour and plated on LB + 100 µg/l ampicillin. The analysis of expression of single colonies from transformation was carried out by ELISA, according to the method described below.

For selection as soluble scFvs, 10-100 µl of phages, eluted from the last panning on antigens, were used to infect cells of the *E. Coli* HB2151 strain in exponential growth. 96 single colonies, were inoculated 150 µl of 2xYT-AG for 16 hours in agitation. 10 µl of each pre-culture were diluted in 150 µl of 2xYT + 100 µg/ml ampicillin + 0.1% glucose and grown at 37°C for 1 hour. Protein expression was induced by adding 1 mM IPTG, 16 hours incubation. Single culture supernatants (containing 2.5 µg/ml of an anti-FLAG M2 monoclonal antibody, Sigma, in PBS-M) were incubated 2 hr at 37°C and analyzed by ELISA. A rabbit anti-mouse antibody (KPL), conjugated to the peroxydase, diluted 1: 10000 in PBS-M wa then added 1 hour at 37°C.

*b. Analysis of cross-reactivity*

Positive clones derived from ELISA screening were further analysed for their binding specificity on antigens other than those selected on. Fifty ml of E. Coli HB2151 strain in exponential growth in SB-A (35 g/l tripton + 20g/l yeast extract + 5 g/l sodium chloride + 100 µg/ml ampicillin, pH7.5) were induced at 30 °C for 3 hours by adding 1

mM IPTG. After centrifugation, the cells were resuspended in 500 µl of TES (0.2 M Tris-HCl pH8 + 0.5 mM EDTA + 0.5 M saccarose) containing protease inhibitors (Complete™, EDTA-free, Boehringer), then 750 µl of TES diluted 1:5 were added and the sample was incubated in orbital agitation for 10 minutes at room temperature. Protein extracts, obtained as supernatant after centrifugation for 20 minutes at 18000 g at 4 °C, were used in an ELISA test with different antigens. In each ELISA well 80 µl of periplasmic extract were loaded then 20 1 PBS + 10% milk. Detection was carried out by using the antibody anti-FLAG M2, as described.

*c. Sequencing*

Plasmids from positive selected clones were sequenced on both DNA strands by using a 373 DNA Sequencer (Applied Biosystems).

*d.Purification of the scFv*

In order to produce high amounts of scFv, a single colony of *E. Coli* HB2151 was inoculated in 100 ml of SB containing 100 µg/ml ampicillin and 2% glucose (SB-AG). After 16 hours incubation at 30 °C, the culture was diluted with 900 ml of SB-AG and left in agitation for a further hour (up to O.D.$_{600}$= 0.9). After centrifugation, pellets were resuspended in SB-A + 1 mM IPTG and induced for 3 hours at 30°C. After centrifugation, the bacteria were resuspended in 10 ml of TES + protease inhibitors, then 15 ml of TES diluted 1:5 + protease inhibitors were added and the sample was incubated for 10 minutes at room temperature. After centrifugation (20 minutes at 18000 g at 4 °C), the supernatant was recovered (fraction 1A) and the pellet was resuspended in 15 ml of 5 mM MgSO$_4$ + protease inhibitors for a further protein extraction. After 10 minutes incubation at room temperature, a second centrifugation step was performed and the supernatant was kept separately as fraction 2A. The fractions 1A and 2A were concentrated independently by ultrafiltration on Diaflo YM10 membrane (Amicon) and purified by chromatography of affinity on protein-L Sepharose (Actigene) or on Ni-NTA (QIAgen), according to the protocols suggested by the manufacturers. Quantification was carried out by reading the absorbance at 280 nm and protein purity was verified on SDS-PAGE followed by AgNO$_3$ staining.

*e.Analysis of thermodynamic stability*

*i) Unfolding and refolding studies of scFv mutants.*

Equilibrium unfolding experiments were performed at 20°C by incubating each scFv mutant (35 µg/ml) at increasing guanidinium chloride (GdmCl) concentrations (0-4 M) in PBS. After 3 h, intrinsic fluorescence emission spectra were recorded at 20°C. To test the reversibility of the unfolding, the mutants (0.70 mg/ml) were unfolded at 20°C in 4 M GdmCl in PBS for 3 h. Refolding was started by 20-fold dilution at 20°C into solutions of the same buffer used for unfolding containing decreasing GdmCl concentrations. After 3 h, intrinsic fluorescence emission spectra were recorded at 20°C. Equilibrium unfolding experiments under reducing conditions were performed by incubating for 3 h at 20°C 35 µg/ml of scFv(HEL-11E) in 20 mM Tris-HCl pH 9.0, 0.15 M NaCl, 2 mM DTT and 0.1 mM EDTA at increasing GdmCl concentrations (0-4 M) before recording fluorescence emission spectra. For refolding experiments, the mutant (0.70 mg/ml) was 3 h unfolded in 4 M GdmCl, 18 mM DTT and 1.0 mM EDTA at pH 9.0, the solution was then 25-fold diluted into decreasing GdmCl concentrations and, after 3h, fluorescence emission spectra were recorded. The functionality of all the mutants refolded under reducing conditions was tested by ELISA (see above).

*ii) Data analysis.*

The changes in intrinsic fluorescence emission spectra at increasing GdmCl concentrations were quantified as the intensity-averaged emission wavelengh, $\bar{\lambda}$(Roger *et al*., 1993) calculated according to the following equation:

$$\bar{\lambda} = \Sigma \ (Ii \ \lambda i) \ / \ \Sigma \ (Ii) \qquad Eq. \ 1$$

where $\lambda i$ and $Ii$ are the emission wavelength and its corresponding fluorescence intensity at that wavelength. Baseline and transition region data for scFv(F8) GdmCl denaturation were fit to a two-state (Santoro *et al*., 1988) linear extrapolation model (LEM) according to the following equation:

$$\Delta G_{unfolding} = \Delta G^{H_2O} + m_g \ [GdmCl] = - \ RT \ lnK_{unfold}. \qquad Eq.2$$

where $\Delta G_{unfold.}$ is the free energy change for unfolding for a given denaturant concentration, $\Delta G^{H_2O}$ is the free energy change for unfolding in the absence of denaturant and $m$ is a slope term which quantitates the change in $\Delta G_{unfolding}$ per unit concentration of denaturant, $R$ is the gas constant, $T$ is the temperature and $K_{unfolding}$ is the equilibrium constant for unfolding. The model expresses the signal as a function of denaturant concentration:

$$y_i = \frac{y_N + m_N \, [X]i + (y_D + m_D \, [X]_i)^* \exp[(-\Delta G^{H_2O} - m_g[X]_i)/RT]}{1 + \exp[(-\Delta G^{H_2O} - m_g[X]_i)/RT]} \qquad Eq.\ 3$$

where $y_i$ is the observed signal, $y_N$ and $y_D$ are the baseline intercepts corresponding to native and denatured proteins, respectively, $m_N$ and $m_D$ are the corresponding baseline slopes, $[X]_i$ is the denaturant concentration after the $i$th addition, $\Delta G^{H_2O}$ is the extrapolated free energy of unfolding in the absence of denaturant, $m_g$ is the slope of a $\Delta G$ unfolding versus $[X]$ plot, $R$ is the gas constant and $T$ is the temperature. The $[GdmCl]_{0.5}$ is the denaturant concentration at the midpoint of the transition and, according to Eq. 2, is calculated as:

$$[GdmCl]_{0.5} = \Delta G^{H_2O} / m_g \qquad Eq.\ 4$$

*f. Affinity measurements*

Affinity-purified scFvG4 and scFvB4 antibodies against the cucumber mosaic virus (CMV) were concentrated to 100 g/ml, assuming that an $OD_{280\ nm}$ reading of 1.0 corresponds to an scFv concentration of 0.7 mg/ml. Binding properties were evaluated using surface plasmon resonance (SPR). Real time interaction analysis were performed in BIAcoreX biosensor system (Pharmacia Biosensor AB).

Approximately 5400 resonance units (RU) of purified CMV (200 ng/µl in 7 mM acetate buffer pH 4.0) were coupled to a CM5 sensor chip by using the Amine Coupling kit (Biacore AB) in order to immobilise the antigen. Kinetic analysis wwere performed under continuous flow of 20 µl/min. After each binding measurement, the surface was regenerated with 10 mM HCl. Rate constants were measured on the basis of a single site model using the BIAevaluation 2.1 software (Biacore AB). The association rate constants ($k_{on}$) were determined from a plot of $\ln(dR/dt)/t$ *versus* concentration over a range of six concentrations (120, 150, 250, 300, 400 and 450 nM) for both scFvs. The dissociation rate constant ($k_{off}$) was determined from the dissociation phase in the sensorgram utilizing the following equation:

$$\ln Rt_0/Rt = k_{off}\ (t\text{-}t_0)$$

where $Rt_0$ is the response 30 s after completion of antibody injection. A linear plot of $\ln Rt/Rt_0$ *vs* $(t\text{-}t_0)$ yields $k_{off}$ directly as a measurement of the slope. The whole affinity constants ($k_D$) were calculated from the association and dissociation rate constants as $k_D = k_{off} / k_{on}$. Affinity values were 60 nM for scFvG4 and 10 nM for scFvB4.

Example 8: Expression of proteins in the plant cytoplasm.

*a. Cloning in PVX-derived vector and expression in N. benthamiana plants*

We delivered the scFvs constructs to plants through a potato virus X (PVX)-derived vector (Chapman *et al.*, 1992) in order to obtain high expression levels. Sequences encoding the scFvs were amplified utilizing oligonucleotides designed to insert the recognition sites *ClaI*, *SphI and XbaI* upstream (<u>*PVX CSX back*</u> = TTC ATC GAT TTG CAT GCT CTA GAC ATG CAG GTG CAG CTG CAG) and the recognition site *SalI* downstream (<u>*PVX flag*</u> = TCC GTC GAC CTA CTT GTC GTC GTC GTC TCC GTA GTC) the scFv genes. The scFvs genes were then inserted as *ClaI- SalI* fragments into the polylinker of the vector pPVX201 (Baulcombe *et al.*, 1995), a derivative of pGC3 vector (Chapman *et al.*, 1992), containing unique cloning sites engineered downstream of the PVX coat protein duplicated subgenomic promoter and a CaMV (cauliflower mosaic virus) 35S promoter. Thus, the PVXscFv(G4) and PVXscFv(B4) constructs were obtained. These plasmids could be used directly as inoculum. DNA of each construct (40 µg) was used to mechanically inoculate *N. benthamiana* plants at the three-four leaf stage, two leaves per plant. Four separate experiments were performed, infecting at least 10 plants with each construct.

Symptoms appeared on the upper leaves usually 7-9 days after inoculation. Symptomatic leaves were frozen in liquid nitrogen and subsequently homogenised in PBS buffer containing a cocktail of protease inhibitor (Complete™, EDTA-free, Boehringer). After centrifugation at 20,000 x g for 30 min at 4°C, the supernatants were used to determine total soluble protein (TSP) concentration by using the Bio-Rad Protein assay and BSA as a standard. Western blots were performed using the monoclonal antibody anti-FLAG M2 (Sigma). Immunoblots confirmed the presence of a 30 K molecule, corresponding to scFvB4 or scFvG4, in the soluble fraction.

b. *Tomato transformation*

The scFvB4 and scFvG4 genes were subsequently subcloned from the PVXscFv(G4) and PVXscFv(B4) constructs as *Xba I-Sal I* fragments into a pBI-derived vector under the control of the CaMV 35S promoter. The plasmids were then transferred into the *Agrobacterium tumefaciens* strain EHA105 by electroporation and used for leaf disc transformation of the miniatur *Lycopersicon esculentum* cultivar, Micro-Tom (microtomato) (Meissner *et al*., 1997). Transgenic microtomato plants were regenerated essentially as described (van Roekel *et al*., 1993).

Trasformation of plants included three independent experiments for both constructs and primary transformants were selected for functional expression. Signals corresponding to the scFvB4 and scFvG4 antibodies were detected both by ELISA and immunoblotting. In particular, the ELISA test was performed according to the following procedure: 5 µg/ml of purified CMV antigens in PBS were coated O/N at 4°C on a microtitre plate. After blocking with 5% milk in PBS, soluble plant extracts (obtained as described above) were added O/N at 4°C. Functional binding was assessed by using the monoclonal antibody anti-FLAG M2 (2.5 µg/ml) and a rabbit anti-mouse peroxidase-conjugated antibody (KPL). For signal development, ABTS Peroxidase Substrate (KPL) was used. Several transgenic plants expressing the recombinant antibody have been obtained and challanged with the virus.

BIBLIOGRAPHY

- Harlow E., & Lane D. (1988) in *Antibodies: a Laboratory Manual* (Cold Spring Harbor Laboratory Press, New York.
- Kabat E. A., Wu T. T., Perry H. M., Gottesmann K. S., & Foeller C. (1991) 'Sequences of Proteins of Immunological Interest'. 5th ed. US Department of Health and Human Services, US Government Printing Office.
- Neri D., Petrul H., Winter G., Light Y., Marais R., Britton K. E., & Creighton A. M. (1996) in *Nature Biotechnology* **14:** 485-490
- Nissim A., Hoogenboom H. R., Tomlison I. M., Flynn G., Midgley C., Lane D. & Winter G. (1994) in *The Embo Journal*, **13:** 692-698.
- Sambrook J., Fritsch E.F., & Maniatis T. (1989) in Molecular Cloning (Cold Spring Harbor Laboratory Press, New York).
- Tavladoraki P., Benvenuto E., Trinca S., De Martinis D., Cattaneo A., & Galeffi, P. (1993) in *Nature* **366:** 469-472.
- Tavladoraki P., Girotti A., Donini M., Arias F. J., Mancini C., Morea V., Chiaraluce R., Consalvi V., & Benvenuto E. (1999) in *European Journal of Biochemistry* **262:** 617-624.
- Visintin M., Tse E., Axelson H., Rabbitts T.H., & Cattaneo A. (1999) *Proc.Natl.Acad. Sci.* **96:** 11723-11728.
- Bhat T.N.Bentley G.A., Fischman T.O., Boulot G., and Poljak R.G. (1990) in Nature **347**: 483-485.
- Jung, S. Honegger A. & Plücthun A. (1997). improving in vivo folding and stability of a single chain Fv antibody fragment by loop grafting Protein Eng. 10, 959-966
- Baulcombe D. C., Chapman S. & Santa Cruz S. S. (1995). in *Plant Journal* **7**, 1045-1053.
  Chapman S., Kavanagh T. & Baulcombe D. C. (1992). in *Plant Journal* **2**, 549-557.
  Meissner R., Jacobson Y., Melamed S., Levyatuv S., Shalev G., Ashri A., Elkind Y & Levy A. (1997). in *Plant Journal* **12,** 1465-1472.
- Santoro, M. M. & Bolen, D.W. (1988). Unfolding free energy changes determined by the linear extrapolation method. 1. Unfolding of phenylmethanesulfonyl alpha-chymotrypsin using different denaturants. *Biochemistry,* **27,** 8063-8068.
- Van Roekel J. S. C., Damm B., Melchers L. S. & Hoekema A. (1993). in *Plant Cell Reports* **12,** 644-647.

**Claims**

1.  A polypeptide pair, consisting of:

- a polypeptide comprising heavy chain framework peptides (H-FR) and heavy chain complementary determining regions peptides (H-CDR) H-FR1, H-CDR1, H-FR2, H-CDR2, H-FR3, H-CDR3, H-FR 4 covalently linked together in the said order, and
- a polypeptide comprising light chain framework peptides (L-FR) and light chain complementary determining regions peptides (L-CDR) L-FR1, L-CDR1, L-FR2, L-CDR2, L-FR3, L-CDR3, L-FR 4 covalently linked together in the said order,

wherein H-FR-1 is SEQ ID N.1, H-FR-2 is SEQ ID N.2, H-FR-3 is SEQ ID N.3, H-FR-4 is SEQ ID N.4, L-FR-1 is SEQ ID N.5, L-FR-2 is SEQ ID N.6, L-FR-3 is SEQ ID N.7 and L-FR-4 is SEQ ID N.8, said polypeptides being capable of making an antibody soluble and stable in the cytoplasm when included, the former, in the heavy chain variable region and, the latter, in the light chain variable region of said antibody.

2. The polypeptide pair according to claim 1, wherein
   the Xaa in position 24 of SEQ ID NO. 1 is Ala;
   the Xaa in position 12 of SEQ ID NO. 2 is Leu;
   the Xaa in position 2 of SEQ ID NO. 3 is Pro;
   the Xaa in position 3 of SEQ ID NO. 3 is Asp;
   the Xaa in position 13 of SEQ ID NO. 3 is Arg;
   the Xaa in position 18 of SEQ ID NO. 3 is Asn;
   the Xaa in position 20 of SEQ ID NO. 3 is Leu;
   the Xaa in position 12 of SEQ ID NO. 7 is Arg; and
   the Xaa in position 15 of SEQ ID NO. 7 is Phe.

3. The polypeptide pair of claims 1 or 2, wherein the polypeptide comprising H-FR1, H-CDR1, H-FR2, H-CDR2, H-FR3, H-CDR3, H-FR 4 covalently linked together in the said order corresponds to SEQ ID NO. 101 and the polypeptide comprising L-FR1, L-CDR1, L-FR2, L-CDR2, L-FR3, L-CDR3, L-FR 4 covalently linked together in the said order corresponds to SEQ ID NO. 102.

4. The polypeptide according to claim 3, wherein said polypeptide comprisies a sequence selected from the group consisting of the sequences reported in the sequence listing from SEQ ID NO:31 to SEQ ID NO: 48.

5. A process for preparing a polypeptide suitable as a variable region of an antibody stable and soluble in cytoplasm and specific for a predetermined antigen comprising the step of:

   - producing an antibody having as a variable region of the heavy chain the polypeptide comprising heavy chain framework peptides (H-FR) and heavy chain complementary determining regions (H-CDR) peptides according to claims 1 to 3, and as a variable region of the light chain the polypeptide comprising light chain framework peptides (L-FR) and light chain complementary determining regions peptides (L-CDR) according to claims 1 to 3;
   - putting in contact said antibody with said antigen and
   - selecting the antibody binding said antigen,
   - isolating the polypeptide of the variable region of the heavy chain and/or the polypeptide of the variable region of the light chain of the antibody binding said antigen.

6. The process according to claim 5, futher comprising the step of

   - sequencing the variable regions of said antibody binding said antigen.

7. The process according to claim 5 or 6, wherein said antigen is Tat, Rev, E7 or NS3 protein.

8. A process for producing peptides conferring to an antibody either binding specificity to a predetermined antigen or solubility and stability in cytoplasm comprising the step of:

   - producing an antibody having as a variable region of the heavy chain the polypeptide comprising heavy chain framework peptides (H-FR) and heavy chain complementary determining regions (H-CDR) peptides according to claims 1 to 3, and as a variable region of the light chain the polypeptide comprising light chain framework peptides (L-FR) and light chain complementary determining regions peptides (L-CDR) according to claims 1 to 3;

- putting in contact said antibody with said antigen and
- selecting the antibody binding said antigen,
- isolating the polypeptide of the variable region of the heavy chain and/or the polypeptide of the variable region of the light chain of the antibody binding said antigen;
- isolating from said polypeptide either the part conferring binding specificity to said antibody or the part conferring stability and solubility in cytoplasm to said antibody.

9. A peptide conferring binding specificity to a predetermined antigen obtainable by the process according to claim 8.

10. The peptide according to claim 9 **characterized by** the fact of comprising a sequence selected from the group consisting of the sequences reported in the annexed sequence listing from SEQ ID NO:9 to SEQ ID NO: 30 and as SEQ ID NO: 94.

11. An antibody comprising a heavy chain, the variable region of said heavy chain comprising heavy chain framework peptides (H-FR) and heavy chain complementary determining regions peptides (H-CDR) H-FR1, H-CDR1, H-FR2, H-CDR2, H-FR3, H-CDR3, H-FR 4 covalently linked together in the said order and a light chain, the variable region of said light chain comprising light chain framework peptides (L-FR) and light chain complementary determining regions peptides (L-CDR) L-FR1, L-CDR1, L-FR2, L-CDR2, L-FR3, L-CDR3, L-FR 4 covalently linked together in the said order, wherein H-FR-1 is SEQ ID N.1, H-FR-2 is SEQ ID N.2, H-FR-3 is SEQ ID N.3, H-FR-4 is SEQ ID N.4, L-FR-1 is SEQ ID N.5, L-FR-2 is SEQ ID N.6, L-FR-3 is SEQ ID N.7 and L-FR-4 is SEQ ID N.8.

12. An antibody according to claim 11 **characterized by** the fact of including as variable region of the heavy chain a polypeptide comprising the sequence reported in the annexed sequence listing as SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, or SEQ ID NO: 47, and respectively as variable region of the light chain a polypeptide comprising the sequences reported in the annexed sequence listing as SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, or SEQ ID NO:48.

13. An antibody according to claim 11, wherein said antibody is a scFv, FAB, Fv, dAb, IgG or IgA.

14. An antibody according to claim 12, wherein said antibody is a scFv, FAB, IgG or IgA.

15. An antibody according to claim 13 or 14, wherein said antibody is a scFv and the polypeptides included as variable regions of the heavy chain and of the light chain are connected by a linker.

16. An antibody according to claim 15, wherein said linker comprises the sequence reported in the annexed sequence listing in as SEQ ID NO: 51.

17. A process for obtaining an antibody according to any of claim 11 to 16 having a binding specificity to a predetermined antigen comprising the steps of:

- producing an antibody having as a variable region of the heavy chain the polypeptide comprising heavy chain framework peptides (H-FR) and heavy chain complementary determining regions peptides (H-CDR) according to claim 1 to 3, and as a variable region of the light chain the polypeptide comprising comprising light chain framework peptides (L-FR) and light chain complementary determining regions peptides (L-CDR) according to claims 1 to 3;
- putting in contact said antibody with said antigen and
- selecting the antibody binding said antigen.

18. A polynucleotide **characterized by** the fact of coding for a polypeptide according to any one of claims 1 to 4.

19. A polynucleotide **characterized by** the fact of coding for a peptide according to any of claims 9 and 10.

20. A polynucleotide according to claim 18, comprising a sequence selected from the group consisting of sequences reported in the annexed sequence listing as SEQ ID NO. 52, SEQ ID NO. 54, SEQ ID NO. 56, SEQ ID NO. 58, SEQ ID NO. 60, SEQ ID NO. 62, SEQ ID NO. 64, SEQ ID NO. 66, SEQ ID NO. 70, SEQ ID NO. 72, SEQ ID NO. 74, SEQ ID NO. 76, SEQ ID NO. 78, SEQ ID NO. 80, SEQ ID NO. 82, SEQ ID NO. 84, SEQ ID NO. 86, SEQ ID NO. 88, SEQ ID NO. 89, SEQ ID NO. 90, SEQ ID NO. 91, SEQ ID NO. 92, SEQ ID NO. 93 and SEQ ID NO. 95.

**21.** A polynucleotide **characterized by** the fact of coding for an antibody according to any one of claims 11 to 16.

**22.** A polynucleotide according to claim 21, comprising a sequence selected from the group consisting of sequences reported in the annexed sequence listing as SEQ ID NO: 84 to SEQ ID NO: 86.

**23.** A pharmaceutical composition **characterized by** the fact of including as an active agent a therapeutically effective amount of an antibody according to any one of claims 11 to 16 together with a pharmaceutically acceptable carrier vehicle or auxiliary agent.

**24.** A pharmaceutical composition **characterized by** the fact of including as an active agent a therapeutically effective amount of the polynucleotides according to any one of claims 18 to 22 together with a pharmaceutically acceptable carrier vehicle or auxiliary agent.

**25.** The antibody according to any one of claims 11 to 16, for use as a medicament.

**26.** Use of the antibody according to any one of claims 11 to 16, for the manufacture of a medicament for the treatment of pathologies associated with accumulation of a molecule inside or outside a human, or animal cell.

**27.** A polynucleotide according to any one of the claims 18 to 22, for use as a medicament.

**28.** Use of the polynucleotide according to any one of the claims 18 to 22, for the manufacture of a medicament for the gene therapy of pathologies associated with the accumulation of a molecule inside or outside a human or animal cell.

**29.** Use of the antibodies according to any one of claims 11 to 16, and/or of the polynucleotides according to any one of claims 18 to 22, for the in vitro diagnosis of pathologies associated with the accumulation of a molecule inside or outside a human, animal or plant cell.

**30.** A diagnostic kit comprising as a reagent an antibody according to any of claims 11 to 16, and/or the polynucleotides according to any one of claims 18 to 22.

**31.** A diagnostic kit comprising as a reagent a peptide according to claim 9 or 10.

**32.** Use of an antibody according to any of claims 11 to 16 and/or of a polynucleotide according to claim 18 to 22 for the treatment of pathologies associated with the accumulation of a molecule inside or outside a plant cell.

**Patentansprüche**

**1.** Ein Polypeptidpaar, bestehend aus:

- einem Polypeptid, das Gerüstpeptide der schweren Kette (H-FR) und Peptide der komplementaritätsbestimmenden Regionen der schweren Kette (H-CDR) umfasst, wobei H-FR1, H-CDR1, H-FR2, H-CDR2, H-FR3, H-CDR3, H-FR4 kovalent miteinander in der genannten Reihenfolge verbunden sind, und

- einem Polypeptid, das Gerüstpeptide der leichten Kette (L-FR) und Peptide der komplementaritätsbestimmenden Regionen der leichten Kette (L-CDR) umfasst, wobei L-FR1, L-CDR1, L-FR2, L-CDR2, L-FR3, L-CDR3, L-FR4 kovalent miteinander in der genannnten Reihenfolge verbunden sind,

worin H-FR-1 SEQ ID NO: 1 ist, H-FR-2 ist SEQ ID NO: 2, H-FR3 ist SEQ ID NO: 3, H-FR 4 ist SEQ ID NO: 4, L-FR-1 ist SEQ ID NO: 5, L-FR-2 ist SEQ ID NO: 6, L-FR-3 ist SEQ ID NO: 7 und L-FR-4 ist SEQ ID NO: 8, wobei die genannten Polypeptide in der Lage sind, einen Antikörper im Zytoplasma löslich und stabil zu machen, wenn das erste in der variablen Region der schweren Kette und das letztere in der variablen Region der leichten Kette von besagtem Antikörper mitumfasst sind.

**2.** Das Polypeptidpaar gemäß Anspruch 1, worin
das Xaa in Position 24 von SEQ ID NO: 1 Ala ist;
das Xaa in Position 12 von SEQ ID NO: 2 Leu ist;

das Xaa in Position 2 von SEQ ID NO. 3 Pro ist;
das Xaa in Position 3 von SEQ ID NO: 3 Asp ist;
das Xaa in Position 13 von SEQ ID NO: 3 Arg ist;
das Xaa in Position 18 von SEQ ID NO: 3 Asn ist;
das Xaa in Position 20 von SEQ ID NO: 3 Leu ist;
das Xaa in Position 12 von SEQ ID NO: 7 Arg ist; und
das Xaa in Position 15 von SEQ ID NO: 7 Phe ist.

3. Das Polypeptidpaar der Ansprüche 1 oder 2, worin das Polypeptid, das H-FR1, H-CDR1, H-FR2, H-CDR2, H-FR3, H-CDR3, H-FR4 umfasst, die kovalent miteinander in der genannten Reihenfolge verbunden sind, mit SEQ ID NO: 101 korrespondiert und das Polypeptid, das L-FR1, L-CDR1, L-FR2, L-CDR2, L-FR3, L-CDR3, L-FR4 umfasst, die kovalent miteinander in der genannten Reihenfolge verbunden sind, mit SEQ ID NO: 102 korrespondiert.

4. Das Polypeptid gemäß Anspruch 3, worin besagtes Polypeptid eine Sequenz umfasst, die aus der Gruppe ausgewählt ist, die aus den Sequenzen besteht, die in der Sequenzauflistung von SEQ ID NO: 31 bis SEQ ID NO: 48 gezeigt werden.

5. Ein Verfahren zur Herstellung eines Polypeptids, das als eine variable Region eines Antikörpers geeignet ist, der stabil und löslich im Zytoplasma ist und spezifisch für ein vorbestimmtes Antigen ist, das den folgenden Schritt umfasst:

- die Herstellung eines Antikörpers, der als eine variable Region der schweren Kette das Polypeptid aufweist, das Gerüstpeptide der schweren Kette (H-FR) und Peptide der komplementaritätsbestimmenden Regionen der schweren Kette (H-CDR) gemäß den Ansprüchen 1 - 3 umfasst, und als eine variable Region der leichten Kette das Polypeptid, das die Gerüstpeptide der leichten Kette (L-FR) und die Peptide der komplementaritätsbestimmenden Regionen der leichten Kette (L-CDR) gemäß den Ansprüchen 1 - 3 umfasst;

- das in Kontakt bringen von besagtem Antikörper mit besagtem Antigen und,

- das Auswählen des Antikörpers, der besagtes Antigen bindet,

- das Isolieren des Polypeptids der variablen Region der schweren Kette und/oder des Polypeptids der variablen Region der leichten Kette des Antikörpers, der besagtes Antigen bindet.

6. Das Verfahren gemäß Anspruch 5, das zusätzlich den folgenden Schritt umfasst:

- das Sequenzieren der variablen Regionen von besagtem Antikörper, der besagtes Antigen bindet.

7. Das Verfahren gemäß Anspruch 5 oder 6, worin besagtes Antigen das Tat-, Rev-, E7- oder NS3-Protein ist.

8. Ein Verfahren zur Herstellung von Peptiden, die einem Antikörper entweder Bindungsspezifität gegenüber einem vorbestimmten Antigen oder Löslichkeit und Stabilität im Zytoplasma vermitteln, das den folgenden Schritt umfasst:

- die Herstellung eines Antikörpers, der als eine variable Region der schweren Kette das Polypeptid aufweist, das Gerüstpeptide der schweren Kette (H-FR) und Peptide der komplementaritätsbestimmenden Regionen der schweren Kette (H-CDR) gemäß den Ansprüchen 1 - 3 umfasst, und als eine variable Region der leichten Kette das Polypeptid, das die Gerüstpeptide der leichten Kette (L-FR) und die Peptide der komplementaritätsbestimmenden Regionen der leichten Kette (L-CDR) gemäß den Ansprüchen 1 - 3 umfasst;

- das in Kontakt bringen von besagtem Antikörper mit besagtem Antigen und

- das Auswählen des Antikörpers, der besagtes Antigen bindet,

- das Isolieren des Polypeptids der variablen Region der schweren Kette und/oder des Polypeptids der variablen Region der leichten Kette des Antikörpers, der besagtes Antigen bindet,

- das Isolieren entweder des Teils von besagtem Antikörpers, der die Bindungsspezifität des besagtm Antikörper vermittelt oder des Teils, der die Stabilität und Löslichkeit im Zytoplasma von besagtem Antikörper vermittelt.

9. Ein Peptid, das Bindungsspezifität für ein vorbestimmten Antigen vermittelt, das durch das Verfahren gemäß Anspruch 8 herstellbar ist.

10. Das Peptid gemäß Anspruch 9, das durch die Tatsache gekennzeichnet ist, dass es eine Sequenz umfasst, die aus der Gruppe ausgewählt ist, die aus den Sequenzen besteht, die in der beigefügten Sequenzauflistung von SEQ ID NO: 9 - SEQ ID NO: 30 und als SEQ ID NO: 94 gezeigt werden.

11. Ein Antikörper, der eine schwere Kette umfasst, wobei die variable Region von besagter schweren Kette Gerüstpeptide der schweren Kette (H-FR) und Peptide der komplementaritätsbestimmenden Regionen der schweren Kette (H-CDR) umfasst, wobei H-FR1, H-CD1, H-FR2, H-CDR2, H-FR3, H-CDR3, H-FR4 kovalent miteinander in der genannten Reihenfolge verbunden sind, und eine leichte Kette umfasst, wobei die variable Region von besagter leichten Kette Gerüstpeptide der leichten Kette (L-FR) und Peptide der komplementaritätsbestimmenden Regionen der leichten Kette (L-CDR) umfasst, wobei L-FR1, L-CDR1, L-FR2, L-CDR2, L-FR3, L-CDR3, L-FR4 kovalent miteinander in der genannten Reihenfolge verbunden sind, worin H-FR-1 SEQ ID NO: 1 ist, H-FR-2 ist SEQ ID NO: 2, H-FR-3 ist SEQ ID NO: 3, H-FR-4 ist SEQ ID NO: 4, L-FR-1 ist SEQ ID NO: 5, L-FR-2 ist SEQ ID NO: 6, L-FR-3 ist SEQ ID NO: 7 und L-FR-4 ist SEQ ID NO: 8.

12. Ein Antikörper gemäß Anspruch 11, der durch die Tatsache gekennzeichnet ist, dass er als variable Region der schweren Kette ein Polypeptid umfasst, das die Sequenz umfasst, die in der beigefügten Sequenzauflistung als SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45 oder SEQ ID NO: 47 aufgelistet ist, und respektive als variable Region der leichten Kette ein Polypeptid, das die Sequenzen umfasst, die in der beigefügten Sequenzauflistung als SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46 oder SEQ ID NO: 48 aufgelistet sind.

13. Ein Antikörper gemäß Anspruch 11, worin besagter Antikörper ein scFv, FAB, Fv, dAb, IgG oder IgA ist.

14. Ein Antikörper gemäß Anspruch 12, worin besagter Antikörper ein scFv, FAB, IgG oder IgA ist.

15. Ein Antikörper gemäß Anspruch 13 oder 14, worin besagter Antikörper ein scFV ist und die Polypeptide, die als variable Region der schweren Kette und der leichten Kette mit umfasst sind, durch einen Linker verbunden sind.

16. Ein Antikörper gemäß Anspruch 15, worin besagter Linker die Sequenz umfasst, die in der beigefügten Sequenzauflistung in SEQ ID NO: 51 aufgezeigt wird.

17. Ein Verfahren zur Gewinnung eines Antikörpers gemäß einem der Ansprüche 11 - 16 mit einer Bindungsspezifität für ein vorbestimmtes Antigen, das die folgenden Schritte umfasst:

   - die Herstellung eines Antikörpers, der als eine variable Region der schweren Kette das Polypeptid aufweist, das Gerüstpeptide der schweren Kette (H-FR) und Peptide der komplementaritätsbestimmenden Regionen der schweren Kette (H-CDR) gemäß den Ansprüchen 1 - 3 umfasst, und als eine variable Region der leichten Kette das Polypeptid, das die Gerüstpeptide der leichten Kette (L-FR) und die Peptide der komplementaritätsbestimmenden Regionen der leichten Kette (L-CDR) gemäß den Ansprüchen 1 - 3 umfasst;

   - das in Kontakt bringen von besagtem Antikörper mit besagtem Antigen und

   - das Auswählen des Antikörpers, der besagtes Antigen bindet.

18. Ein Polypeptid, das durch die Tatsache gekennzeichnet ist, dass es für ein Polypeptid gemäß einem der Ansprüche 1 - 4 kodiert.

19. Ein Polypeptid, das durch die Tatsache gekennzeichnet ist, dass es für ein Polypeptid gemäß einem der Ansprüche 9 und 10 kodiert.

20. Ein Polynukleotid gemäß Anspruch 18, das eine Sequenz umfasst, die aus der Gruppe ausgewählt ist, die aus den Sequenzen besteht, die in der beigefügten Sequenzauflistung als SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, SEQ ID NO: 89, SEQ ID NO: 90, SEQ ID NO: 91, SEQ ID NO: 92, SEQ ID NO: 93 und SEQ ID NO: 95 gezeigt werden.

21. Ein Polypeptid, das durch die Tatsache gekennzeichnet ist, dass es für einen Antikörper gemäß einem der Ansprüche 11 - 16 kodiert.

22. Ein Polynukleotid gemäß Anspruch 21, das eine Sequenz umfasst, die aus der Gruppe ausgewählt ist, die aus den Sequenzen besteht, die in der beigefügten Sequenzauflistung als SEQ ID NO: 84 bis SEQ ID NO: 86 gezeigt werden.

23. Eine pharmazeutische Zusammensetzung, die durch die Tatsache gekennzeichnet ist, dass sie als ein aktives Mittel eine therapeutisch wirksame Menge eines Antikörpers gemäß einem der Ansprüche 11 - 16 zusammen mit einem pharmazeutisch verträglichen Trägermittel oder Hilfsstoff umfasst.

24. Eine pharmazeutische Zusammensetzung, die durch die Tatsache gekennzeichnet ist, dass sie als ein aktives Mittel eine therapeutisch wirksame Menge der Polynukleotide gemäß einem der Ansprüche 18 - 22 zusammen mit einem pharmazeutisch verträglichen Trägermittel oder Hilfsstoff umfasst.

25. Der Antikörper gemäß einem der Ansprüche 11 - 16 zur Verwendung als ein Medikament.

26. Verwendung des Antikörpers gemäß einem der Ansprüche 11 - 16 zur Herstellung eines Medikaments zur Behandlung von Pathologien, die mit der Anhäufung eines Moleküls innerhalb oder außerhalb einer menschlichen oder tierischen Zelle assoziiert sind.

27. Ein Polynukleotid gemäß einem der Ansprüche 18 - 22 zur Verwendung als ein Medikament.

28. Verwendung des Polynukleotids gemäß einem der Ansprüche 18 - 22 zur Herstellung eines Medikaments zur Gentherapie von Pathologien, die mit der Anhäufung eines Moleküls innerhalb oder außerhalb einer menschlichen oder tierischen Zelle assoziiert sind.

29. Verwendung der Antikörper gemäß einem der Ansprüche 11 - 16 und/oder der Polynukleotide gemäß einem der Ansprüche 18 - 22 zur *in-vitro*-Diagnose von Pathologien, die mit der Anhäufung eines Moleküls innerhalb oder außerhalb einer menschlichen, tierischen oder pflanzlichen Zelle assoziiert sind.

30. Ein diagnostisches Kit, das als ein Reagenz einen Antikörper gemäß einem der Ansprüche 11 - 16 und/oder die Polynukleotide gemäß einem der Ansprüche 18 - 22 umfasst.

31. Ein diagnostisches Kit, das als ein Reagenz ein Peptid gemäß Anspruch 9 oder 10 umfasst.

32. Verwendung eines Antikörpers gemäß einem der Ansprüche 11 - 16 und/oder eines Polynukleotid gemäß Anspruch 18 - 22 zur Behandlung von Pathologien, die mit der Anhäufung eines Moleküls innerhalb oder außerhalb einer pflanzlichen Zelle assoziiert sind.

**Revendications**

1. Paire de polypeptides, constituée de :

- un polypeptide comprenant des peptides de structure de la chaîne lourde (H-FR) et des peptides des régions déterminant la complémentarité de la chaîne lourde (H-CDR) H-FR1, H-CDR1, H-FR2, H-CDR2, H-FR3, H-CDR3, H-FR4 liés ensembles de façon covalente dans ce dit ordre et

- un polypeptide comprenant des peptides de structure de la chaîne légère (L-FR) et des peptides des régions déterminant la complémentarité de la chaîne légère (L-CDR) L-FR1, L-CDR1, L-FR2, L-CDR2, L-FR3, L-CDR3, L-FR4 liés ensembles de façon covalente dans ce dit ordre

- dans lequel H-FR-1 est la SEQ ID n° : 1, H-FR-2 est la SEQ ID n° : 2, H-FR-3 est la SEQ ID n° : 3, H-FR-4 est la SEQ ID n° : 4, L-FR-1 est la SEQ ID n° : 5, L-FR-2 est la SEQ ID n° : 6, L-FR-3 est la SEQ ID n° : 7 et L-FR-4 est la SEQ ID n° : 8, lesdits polypeptides étant capables de rendre un anticorps soluble et stable dans le cytoplasme quand il est inclus, le premier, dans la région variable de la chaîne lourde et le dernier dans la région variable de la chaîne légère dudit anticorps.

2. Paire de polypeptides selon la revendication 1, dans lesquels
le Xaa à la position 24 de la SEQ ID n° : 1 est une Ala ;
le Xaa à la position 12 de la SEQ ID n° : 2 est une Leu ;
le Xaa à la position 2 de la SEQ ID n° : 3 est une Pro ;
le Xaa à la position 3 de la SEQ ID n° : 3 est une Asp ;
le Xaa à la position 13 de la SEQ ID n° : 3 est une Arg ;
le Xaa à la position 18 de la SEQ ID n° : 3 est une Asn ;
le Xaa à la position 20 de la SEQ ID n° : 3 est une Leu ;
le Xaa à la position 12 de la SEQ ID n° : 7 est une Arg ; et
le Xaa à la position 15 de la SEQ ID n° : 7 est une Phe.

3. Paire de polypeptides selon les revendications 1 ou 2, dans lesquels le polypeptide comprenant H-FR1, H-CDR1, H-FR2, H-CDR2, H-FR3, H-CDR3, H-FR4 liés ensembles de façon covalente dans ce dit ordre, correspond à la SEQ ID n° : 101, et le polypeptide comprenant L-FR1, L-CDR1, L-FR2, L-CDR2, L-FR3, L-CDR3, L-FR4 liés ensembles de façon covalente dans ce dit ordre, correspond à la SEQ ID n° : 102.

4. Polypeptide selon la revendication 3, dans lequel ledit polypeptide comprend une séquence choisie dans le groupe constitué par les séquences rapportées dans le listing de séquences de la SEQ ID n° : 31 à la SEQ ID n° : 48.

5. Procédé de préparation d'un polypeptide convenable en tant que région variable d'un anticorps stable et soluble dans le cytoplasme et spécifique pour un antigène prédéterminé, comprenant les étapes de :

- production d'un anticorps ayant en tant que région variable de la chaîne lourde, le polypeptide comprenant les peptides de structure de la chaîne lourde (H-FR) et les peptides des régions déterminantes complémentaires de la chaîne lourde (H-CDR) selon les revendications 1 à 3, et en tant que région variable de la chaîne légère, le polypeptide comprenant les peptides de structure de la chaîne légère (L-FR) et les peptides des régions déterminantes complémentaires de la chaîne légère (L-CDR) selon les revendications 1 3 ;

- mise en contact dudit anticorps avec ledit antigène et

- sélection de l'anticorps se fixant audit antigène,

- isolement du polypeptide de la région variable de la chaîne lourde et/ou du polypeptide de la région variable de la chaîne légère de l'anticorps se fixant audit antigène.

6. Procédé selon la revendication 5, comprenant en outre, l'étape de

- séquençage des régions variables dudit anticorps se fixant audit antigène.

7. Procédé selon les revendications 5 ou 6, dans lequel ledit antigène est la protéine Tat, Rev, E7 ou NS3.

8. Procédé pour la production de peptides conférant à un anticorps, soit une spécificité de fixation à un antigène prédéterminé, soit la solubilité et la stabilité dans le cytoplasme, comprenant les étapes de :

- production d'un anticorps ayant en tant que région variable de la chaîne lourde, le polypeptide comprenant les peptides de structure de la chaîne lourde (H-FR) et les peptides des régions déterminantes complémentaires de la chaîne lourde (H-CDR) selon les revendications 1 à 3, et en tant que région variable de la chaîne légère, le polypeptide comprenant les peptides de structure de la chaîne légère (L-FR) et les peptides des régions déterminant la complémentarité de la chaîne légère (L-CDR) selon les revendications 1 à 3 ;

- mise en contact dudit anticorps avec ledit antigène et

- sélection de l'anticorps se fixant audit antigène,

- isolement du polypeptide de la région variable de la chaîne lourde et/ou du polypeptide de la région variable de la chaîne légère de l'anticorps se fixant audit antigène.

- isolement à partir dadit polypeptide, soit de la partie conférant la spécificité de fixation audit anticorps, soit de la partie conférant la stabilité et la solubilité dans le cytoplasme audit anticorps.

9. Peptide conférant une spécificité de fixation à un antigène prédéterminé que l'on peut obtenir par le processus selon la revendication 8.

10. Peptide selon la revendication 9, **caractérisé par** le fait de comprendre une séquence choisie dans le groupe constitué des séquences rapportée dans le listing de séquences en annexe de la SEQ ID n° : 9 à la SEQ ID n° : 30 et en tant que SEQ ID n° : 94.

11. Anticorps comprenant une chaîne lourde, la région variable de ladite chaîne lourde comprenant des peptides de structure de la chaîne lourde (H-FR) et des peptides des régions déterminant la complémentarité de la chaîne lourde (H-CDR) H-FR1, H-CDR1, H-FR2, H-CDR2, H-FR3, H-CDR3, H-FR4 liés ensembles de façon covalente dans ce dit ordre, et une chaîne légère, la région variable de ladite chaîne légère comprenant les peptides de structure de la chaîne légère (L-FR) et les peptides des régions déterminantes complémentaires de la chaîne légère (L-CDR) L-FR1, L-CDR1, L-FR2, L-CDR2, L-FR3, L-CDR3, L-FR4 liés ensembles de façon ccvalente dans ce dit ordre, dans lequel H-FR-1 est la SEQ ID n° : 1, H-FR-2 est la SEQ ID n° : 2, H-FR-3 est la SEQ ID n° : 3, H-FR-4 est la SEQ ID n° : 4, L-FR-1 est la SEQ ID n° : 5, L-FR-2 est la SEQ ID n° : 6, L-FR-3 est la SEQ ID n° : 7 et L-FR-4 est la SEQ ID n° : 8.

12. Anticorps selon la revendication 11, **caractérisé par** le fait d'inclure en tant que région variable de la chaîne lourde, un polypeptide comprenant la séquence rapportée dans le listing de séquences en annexe en tant que SEQ ID n° : 31, SEQ ID n° : 33, SEQ ID n° : 35, SEQ ID n° : 37, SEQ ID n° : 39, SEQ ID n° : 41, SEQ ID n° : 43, SEQ ID n° : 45 ou SEQ ID n° : 47, et respectivement en tant que région variable de la chaîne légère, un polypeptide comprenant les séquences rapportées dans le listing de séquences en annexe en tant que SEQ ID n° : 32, SEQ ID n° : 34, SEQ ID n° : 36, SEQ ID n° : 38, SEQ ID n° : 40, SEQ ID n° : 42, SEQ ID n° : 44, SEQ ID n° : 46 ou SEQ ID n° : 48.

13. Anticorps selon la revendication 11, dans lequel ledit anticorps est un scFv, un FAB, un Fv, un dAb, une IgG ou une IgA.

14. Anticorps selon la revendication 12, dans lequel ledit anticorps est un scFv, un FAB, une IgG ou une IgA.

15. Anticorps selon la revendication 13 ou 14, dans lequel ledit anticorps est un scFv, et les polypeptides inclus en tant que région variable de la chaîne lourde et de la chaîne légère sont connectés par un lieur.

16. Anticorps selon la revendication 15, dans lequel ledit lieur comprend la séquence rapportée dans le listing de séquences en annexe en tant que SEQ ID n° : 51.

17. Processus pour l'obtention d'un anticorps selon l'une quelconque des revendications 11 à 16 ayant une spécificité de fixation à un antigène prédéterminé comprenant les étapes de :

- production d'un anticorps ayant en tant que région variable de la chaîne lourde, le polypeptide comprenant les peptides de structure de la chaîne lourde (H-FR) et les peptides des régions déterminantes complémentaires de la chaîne lourde (H-CDR) selon les revendications 1 à 3, et en tant que région variable de la chaîne légère, le polypeptide comprenant les peptides de structure de la chaîne légère (L-FR) et les peptides des régions déterminantes complémentaires de la chaîne légère (L-CDR) selon les revendications 1 à 3 ;

- mise en contact dudit anticorps avec ledit antigène et

- sélection de l'anticorps se fixant audit antigène.

18. Polynucléotide **caractérisé par** le fait de coder pour un polypeptide selon l'une quelconque des revendications 1 à 4.

**19.** Polynucléotide **caractérisé par** le fait de coder pour un polypeptide selon l'une quelconque des revendications 9 et 10.

**20.** Polynucléotide selon la revendication 18, comprenant une séquence choisie dans le groupe constitué par les séquences rapportées dans le listing de séquences en annexe, en tant que SEQ ID n° : 52, SEQ ID n° : 54, SEQ ID n° : 56, SEQ ID n° : 58, SEQ ID n° : 60, SEQ ID n° : 62, SEQ ID n° : 64, SEQ ID n° : 66, SEQ ID n° : 70, SEQ ID n° : 72, SEQ ID n° : 74, SEQ ID n° : 76, SEQ ID n° : 78, SEQ ID n° : 80, SEQ ID n° : 82, SEQ ID n° : 84, SEQ ID n° : 86, SEQ ID n° : 88, SEQ ID n° : 89, SEQ ID n° : 90, SEQ ID n° : 91, SEQ ID n° : 92, SEQ ID n° : 93 et SEQ ID n° : 95.

**21.** Polynucléotide **caractérisé par** le fait de coder pour un anticorps selon l'une quelconque des revendications 11 à 16.

**22.** Polynucléotide selon la revendication 21, comprenant une séquence choisie dans le groupe constitué par les séquences rapportées dans le listing de séquences en annexe, en tant que SEQ ID n° : 84 à SEQ ID n° : 86.

**23.** Composition pharmaceutique **caractérisée par** le fait d'inclure en tant qu'ingrédient actif, une quantité thérapeutiquement efficace d'un anticorps selon l'une quelconque des revendications 11 à 16, ensemble avec un vecteur, un véhicule ou un agent auxiliaire pharmaceutiquement acceptable.

**24.** Composition pharmaceutique **caractérisée par** le fait d'inclure en tant qu'ingrédient actif une quantité thérapeutiquement efficace des polynucléotides selon l'une quelconque des revendications 18 à 22, ensemble avec un vecteur, un véhicule ou un agent auxiliaire pharmaceutiquement acceptable.

**25.** Anticorps selon l'une quelconque des revendications 11 à 16, à utiliser en tant que médicament.

**26.** Utilisation de l'anticorps selon l'une quelconque des revendications 11 à 16, pour la fabrication d'un médicament pour le traitement de pathologies associées à l'accumulation d'une molécule à l'intérieur ou à l'extérieur d'une cellule humaine ou animale.

**27.** Polynucléotide selon l'une quelconque des revendications 18 à 22, à utiliser en tant que médicament.

**28.** Utilisation du polynucléotide selon l'une quelconque des revendications 18 à 22, pour la fabrication d'un médicament pour la thérapie génique de pathologies associées à l'accumulation d'une molécule à l'intérieur ou à l'extérieur d'une cellule humaine ou animale.

**29.** Utilisation des anticorps selon l'une quelconque des revendications 11 à 16 et/ou des polynucléotides selon l'une quelconque des revendications 18 à 22, pour le diagnostic *in vitro* de pathologies associées à l'accumulation d'une molécule à l'intérieur ou à l'extérieur d'une cellule humaine, animale ou de plante.

**30.** Kit de diagnostic comprenant en tant que réactif un anticorps selon l'une quelconque des revendications 11 à 16 et/ou les polynucléotides selon l'une quelconque des revendications 18 à 22.

**31.** Kit de diagnostic comprenant en tant que réactif un peptide selon la revendication 9 ou 10.

**32.** Utilisation d'un anticorps selon l'une quelconque des revendications 11 à 16 et/ou d'un polynucléotide selon les revendications 18 à 22, pour le traitement de pathologies associées à l'accumulation d'une molécule à l'intérieur ou à l'extérieur d'une cellule de plante.

FIG. 1

EP 1 120 464 B1

| VH | H-FR 1 | H-CDR 1 | H-FR 2 | H-CDR 2 | H-FR 3 | H-CDR 3 | H-FR 4 |
|---|---|---|---|---|---|---|---|

LINKER

| VL | L-FR 1 | L-CDR 1 | L-FR 2 | L-CDR 2 | L-FR 3 | L-CDR 3 | L-FR 4 |
|---|---|---|---|---|---|---|---|

**FIG. 2**

**VH**

| | | | | | | | | | | | | | | | | | | | | | | | 24 | 25 | 26 | 27 | 28 | 29 | 30 | 31 | 32 | 33 | 34 | 35 | | | | | |
|1|2|3|4|5|6|7|8|9|10|11|12|13|14|15|16|17|18|19|20|21|22|23|24|25|26|27|28|29|30|31|32|33|34|35|36|37|38|39|40|

CDR1

| | | | | | | | | | | | | | | | | | | | | | | | A | | G | F | T | F | S | S | Y | G | M | S | | | | | |

| 41 | 42 | 43 | 44 | 45 | 46 | 47 | 48 | 49 | 50 | 51 | 52 | 52A | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 |

CDR2

| | | | | | L | | | T | I | N | S | N | G | G | S | T | F | | P | D | | | | | | | | | | R | | | | | N | | L | | |

| 80 | 81 | 82 | 82A | 82B | 82C | 83 | 84 | 85 | 86 | 87 | 88 | 89 | 90 | 91 | 92 | 93 | 94 | 95 | 96 | 97 | 98 | 99 | 100 | 100A | 100B | 100C | 100D | 100E | 100F | 100G | 100J | 101 | 102 |

CDR3

| | | | | | | | | | | | | | | | | | R | R | N | Y | P | Y | Y | Y | G | S | R | G | Y | F | D | Y |

| 103 | 104 | 105 | 106 | 107 | 108 | 109 | 110 | 111 | 112 | 113 |

**VL**

| 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 27A | 27B | 27C | 27D | 28 | 29 | 30 | 31 | 32 | 33 | 34 | 35 | 36 |

CDR1

| | | | | | | | | | | | | | | | | | | | | | | | R | A | S | E | S | V | D | S | Y | G | N | S | F | M | H |

| 37 | 38 | 39 | 40 | 41 | 42 | 43 | 44 | 45 | 46 | 47 | 48 | 49 | 50 | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 |

CDR2

| | | | | | | | | | | | | R | A | L | N | L | E | S | | | | | | | | | | | | | R | | F | | | | | |

| 77 | 78 | 79 | 80 | 81 | 82 | 83 | 84 | 85 | 86 | 87 | 88 | 89 | 90 | 91 | 92 | 93 | 94 | 95 | 96 | 97 | 98 | 99 | 100 | 101 | 102 | 103 | 104 | 105 | 106 | 107 | 108 |

CDR3

| | | | | | | | | | | | | Q | Q | S | N | E | D | P | W | T |

## FIG. 3

EP 1 120 464 B1

VH

|  | FR1 | CDR1 | FR2 | CDR2 | FR3 | CDR3 | FR4 |
|---|---|---|---|---|---|---|---|

F8   QVQLQESGGDLVQPGGSLKLSCAASGFTFSSYGMSWVRQTPDKRLELVATINSNGGSTFYPDSVKGRFTISRDNAKNTLYLQMSSLKSEDTAMYYCARRRNYPYYYGSRGYFDYWGQGTTVTVSS

P1   QVQLQESGGDLVQPGGSLKLSCAASGFTFSGYGMSWVRQTPDKRLELVATI-WGDGSTDYPDSVKGRFTISRDNAKNTLYLQMSSLKSEDTAMYYCARRRDYR-------FDYWGQGTTVTVSS

P2   QVQLQESGGDLVQPGGSLKLSCAASGFSFTGYGMNWVRQTPDKRLEWVAMI-WGDGNTDYPDSVKGRFTISRDNAKNTLYLQMSSLKSEDTAMYYCARERDYR-------LDYWGQGTTVTVSS

P3   QVQLQESGGDLVQPGGSLKLSCAVSGFSLTGYGVNWVRQTPDKRLEWVAMI-WGDGNTDYPDSVKGRFTISKDNAKSTVYLQMSSLKSEDTAMYYCARERDYR-------LDYWGQGTTVTVSS

P4   QVQLQESGGDLVQPGGSLKLSCAVSGFSLTGYGVNWVRQTPDKRLEWVAMI-WGDGNTDYPDSVKGRFTISKDNAKSTVYLQMSSLKSEDTAMYYCARERDYR-------LDYWGQGTTVTVSS

P5   QVQLQESGGDLVQPGGSLKLSCAVSGFSLTGYGVNWVRQTPDKRLEWVAMI-WGDGNTDYNSSVKGRFTISKDNAKSTVYLQMSSLKSEDTAMYYCARERDYR-------LDYWGQGTTVTVSS

D1.3 QVQLQESGPGLVAPSQSLSITCTVSGFSLTGYGVNWVRQPPGKGLEWLGMI-WGDGNTDYNSALKSRLSISKDNSKSQVFLKMNSLHTDDTARYYCARERDYR-------LDYWGQGTTVTVSS

VL

|  | FR1 | CDR1 | FR2 | CDR2 | FR3 | CDR3 | FR4 |
|---|---|---|---|---|---|---|---|

F8   DIELTQSPASLAVSLGQRATISCRASESVDSYGNSFMHWYQQKPGQPPKLLIYRALNLESGIPARFSGSGSRTDFTLTINPVEADDVATYYCQQSNEDPWTFGGGTKLEIKR

P1   DIELTQSPASLAVSLGQRATISCRASESV----HNYMHWYQQKPGQPPKLLIYYALTLESGIPARFSGSGSRTDFTLTINPVEADDVATYYCQQSWSTPWTFGGGTKLEIKR

P2   DIELTQSPASLAVSLGQRATISCRASGNV----HNYMAWYQQKPGQPPKLLIYYTTTLADGIPARFSGSGSRTDFTLTINPVEADDVATYYCQHFWSTPRTFGGGTKLEIKR

P3   DIELTQSPASLAVSLGQRATISCRASGNI----HNYLAWYQQKPGQPPKLLIYYTTTLADGIPARFSGSGSRTDYTLTINPVEADDVATYYCQHFWSTPRTFGGGTKLEIKR

P4   DIELTQSPASLAVSLGQRATISCRASGNI----HNYLAWYQQKPGQPPKLLIYYTTTLADGIPARFSGSGSGTDYTLTINPVEADDVATYYCQHFWSTPRTFGGGTKLEIKR

P5   DIELTQSPASLAVSLGQRATISCRASGNI----HNYLAWYQQKPGQPPKLLIYYTTTLADGIPARFSGSGSGTDYTLTINPVEADDVATYYCQHFWSTPRTFGGGTKLEIKR

D1.3 DIELTQSPASLSASVGETVTITCRASGNI----HNYLAWYQQKQGKSPQLLVYYTTTLADGVPSRFSGSGSGTQYSLKINSLQPEDFGSYYCQHFWSTPRTFGGGTKLEIKR

FIG. 4

|  | 24 | 25 | 26 | 27 | 27A | 27B | 27C | 27D | 28 | 29 | 30 | 31 | 32 | 33 | 34 |
|---|---|---|---|---|---|---|---|---|---|---|---|---|---|---|---|
| **L-CDR1-F8:** | R | A | S | E | S | V | D | S | Y | G | N | S | F | M | H |
|  | \| | \| | \| | \| | \| | \| | . |  |  |  |  | . | : | \| | \| |
| **L-CDR1-MUT:** | R | A | S | E | S | V | H | . | . | . | . | N | Y | M | H |

## FIG. 5

EP 1 120 464 B1

**VHa**: (5')AAT CCA TGC CAT GGC CCA GGT GCA GCT GCA GGA GTC TGG G(3')

**VHf**: (5')GGT CCC TTG GCC CCA GTA GTC AAA MNN MNN MNN MNN TCT TGC ACA GTA ATA CAT GGC TG(3')

**VLa**: (5')TTT GAC TAC TGG GGC CAA GGG ACC(3')

**VLf**: (5')GAG CTT GGT GCC TCC ACC GAA CGT CCA CGG MNN MNN MNN MNN TTG CTG ACA GTA ATA GGT TGC(3')

**VLg**: (5')TTC TCG ACT TGC GGC CGC CCG TTT GAT CTC GAG CTT GGT GCC TCC ACC GAA CG(3')

## FIG. 6

| H - FR1 | H - CDR1 - F8 | H - FR2 | H - CDR2 - F8 | H - FR3 | H - CDR3 | H - FR4 |

| L - FR1 | L - CDR1 - MUT | L - FR2 | L - CDR2 - F8 | L - FR3 | L - CDR3 | L - FR4 |

**FIG. 7**

SEQUENCE LISTING

<110> Ente per le Nuove Tecnologie, l'Energia e l'Ambiente (ENEA)

<120> STABILIZING PEPTIDES, POLYPEPTIDES AND ANTIBODIES WHICH INCLUDE THEM

<130> RM/X89713/EP-EBR

<140> EP 00204725.6
<141> 2000-12-21

<150> IT RM99A000803
<151> 1999-12-30

<160> 102

<170> PatentIn version 3.0

<210> 1
<211> 25
<212> PRT
<213> Artificial sequence

<220>
<221> misc_feature
<222> (24)..(24)
<223> Xaa is Ala or a non polar amino acid

<400> 1


```
Gln Val Gln Leu Gln Glu Ser Gly Gly Asp Leu Val Gln Pro Gly Gly
1               5                   10                  15

Ser Leu Lys Leu Ser Cys Ala Xaa Ser
            20                  25
```


<210> 2
<211> 14
<212> PRT
<213> Artificial sequence

<220>
<221> misc_feature
<222> (12) .. (12)
<223> Xaa is Leu or a non polar amino acid

<400> 2


```
Trp Val Arg Gln Thr Pro Asp Lys Arg Leu Glu Xaa Val Ala
1               5                   10
```


<210> 3
<211> 39
<212> PRT
<213> Artificial sequence

<220>
<221> misc_feature
<222> (2)..(2)
<223> Xaa is Pro, a non polar amino acid or an amino acid with no charg e

<220>
<221> misc_feature
<222> (3)..(3)

<223> Xaa is Asp an acid amino acid or a polar amino acid with no charg e

<220>
<221> misc_feature
<222> (13) .. (13)
<223> Xaa is Arg or a basic amino acid

<220>
<221> misc_feature
<222> (18)..(18)
<223> Xaa is Asn, or a polar amino acid with no charge

<220>
<221> misc_feature
<222> (20)..(20)
<223> Xaa is Leu or a non polar amino acid

<400> 3

```
Tyr Xaa Xaa Ser Val Lys Gly Arg Phe Thr Ile Ser Xaa Asp Asn Ala
1               5                   10              15

Lys Xaa Thr Xaa Tyr Leu Gln Met Ser Ser Leu Lys Ser Glu Asp Thr
        20                  25              30

Ala Met Tyr Tyr Cys Ala Arg
        35
```

<210> 4
<211> 11
<212> PRT
<213> Artificial sequence

<400> 4

```
Trp Gly Gln Gly Thr Thr Val Thr Val Ser Ser
1               5                   10
```

<210> 5
<211> 23
<212> PRT
<213> Artificial sequence

<400> 5

```
Asp Ile Glu Leu Thr Gln Ser Pro Ala Ser Leu Ala Val Ser Leu Gly
1               5                   10              15

Gln Arg Ala Thr Ile Ser Cys
        20
```

<210> 6
<211> 15
<212> PRT
<213> Artificial sequence

<400> 6

```
Trp Tyr Gln Gln Lys Pro Gly Gln Pro Pro Lys Leu Leu Ile Tyr
1               5               10              15
```

<210> 7
<211> 32
<212> PRT
<213> Artificial sequence

<220>
<221> misc_feature
<222> (12)..(12)
<223> Xaa is Arg, a basic amino acid or a polar amino acid with no charg

<220>
<221> misc_feature
<222> (15) .. (15)
<223> Xaa is Phe, a non polar amino acid or a polar amino acid with no charg

<400> 7

```
Gly Ile Pro Ala Arg Phe Ser Gly Ser Gly Ser Xaa Thr Asp Xaa Thr
1               5               10              15

Leu Thr Ile Asn Pro Val Glu Ala Asp Asp Val Ala Thr Tyr Tyr Cys
            20              25              30
```

<210> 8
<211> 11
<212> PRT
<213> Artificial sequence

<400> 8

```
Phe Gly Gly Gly Thr Lys Leu Glu Ile Lys Arg
1               5               10
```

<210> 9
<211> 10
<212> PRT
<213> Artificial sequence

<400> 9

```
Gly Phe Thr Phe Ser Ser Tyr Gly Met Ser
1               5               10
```

<210> 10
<211> 10
<212> PRT
<213> Artificial sequence

<400> 10

```
Thr Ile Asn Ser Asn Gly Gly Ser Thr Phe
1               5               10
```

<210> 11
<211> 16
<212> PRT
<213> Artificial sequence

<400> 11

```
        Arg Arg Asn Tyr Pro Tyr Tyr Tyr Gly Ser Arg Gly Tyr Phe Asp Tyr
        1               5               10              15
```

<210> 12
<211> 15
<212> PRT
<213> Artificial sequence

<400> 12

```
        Arg Ala Ser Glu Ser Val Asp Ser Tyr Gly Asn Ser Phe Met His
        1               5               10              15
```

<210> 13
<211> 7
<212> PRT
<213> Artificial sequence

<400> 13

```
        Arg Ala Leu Asn Leu Glu Ser
        1               5
```

<210> 14
<211> 9
<212> PRT
<213> Artificial sequence

<400> 14

```
        Gln Gln Ser Asn Glu Asp Pro Trp Thr
        1               5
```

<210> 15
<211> 8
<212> PRT
<213> Artificial sequence

<400> 15

```
        Glu Arg Asp Tyr Arg Leu Asp Tyr
        1               5
```

<210> 16
<211> 9
<212> PRT
<213> Artificial sequence

<400> 16

```
Gln His Phe Trp Ser Thr Pro Arg Thr
1               5
```

<210> 17
<211> 7
<212> PRT
<213> Artificial sequence

<400> 17

```
Thr Arg Pro Tyr Phe Asp Tyr
```

```
                    1               5
```

<210> 18
<211> 9
<212> PRT
<213> Artificial sequence

<400> 18

```
Gln Gln Val Thr Tyr Lys Pro Trp Thr
1               5
```

<210> 19
<211> 7
<212> PRT
<213> Artificial sequence

<400> 19

```
Glu Arg Trp Asp Phe Asp Tyr
1               5
```

<210> 20
<211> 9
<212> PRT
<213> Artificial sequence

<400> 20

```
Gln Gln Ala Leu Ser Pro Pro Trp Thr
1               5
```

<210> 21

<211> 7
<212> PRT
<213> Artificial sequence

<400> 21

```
                    Asn Trp Arg Arg Phe Asp Tyr
                    1               5
```

<210> 22
<211> 9
<212> PRT
<213> Artificial sequence

<400> 22

```
                    Gln Gln Gly Ala Ser Ile Pro Trp Thr
                    1               5
```

<210> 23
<211> 7
<212> PRT
<213> Artificial sequence

<400> 23

```
                    Gly Arg His Lys Phe Asp Tyr
                    1               5
```

<210> 24
<211> 9
<212> PRT
<213> Artificial sequence

<400> 24

```
                    Gln Gln Tyr Gly Arg Arg Pro Trp Thr
                    1               5
```

<210> 25
<211> 7
<212> PRT
<213> Artificial sequence

<400> 25

```
                    Asn Asn Trp Ser Phe Asp Tyr
                    1               5
```

<210> 26
<211> 9
<212> PRT
<213> Artificial sequence

<400> 26

```
Gln Gln Gly Gln Arg Lys Pro Trp Thr
1               5
```

<210> 27
<211> 7
<212> PRT
<213> Artificial sequence

<400> 27

```
Asn Asn Tyr Ser Phe Asp Tyr
1               5
```

<210> 28
<211> 9
<212> PRT
<213> Artificial sequence

<400> 28

```
Gln Gln Gly Arg Arg Ala Pro Trp Thr
1               5
```

<210> 29
<211> 7
<212> PRT
<213> Artificial sequence

<400> 29

```
Val Thr Tyr Asn Phe Asp Tyr
1               5
```

<210> 30
<211> 9
<212> PRT
<213> Artificial sequence

<400> 30

```
Gln Gln Ser Arg Arg Pro Pro Trp Thr
1               5
```

<210> 31
<211> 125
<212> PRT
<213> Artificial sequence

<400> 31

```
Gln Val Gln Leu Gln Glu Ser Gly Gly Asp Leu Val Gln Pro Gly Gly
1               5               10              15

Ser Leu Lys Leu Ser Cys Ala Ala Ser Gly Phe Thr Phe Ser Ser Tyr
            20              25              30

Gly Met Ser Trp Val Arg Gln Thr Pro Asp Lys Arg Leu Glu Leu Val
        35              40              45

Ala Thr Ile Asn Ser Asn Gly Gly Ser Thr Phe Tyr Pro Asp Ser Val
    50              55              60

Lys Gly Arg Phe Thr Ile Ser Arg Asp Asn Ala Lys Asn Thr Leu Tyr
65              70              75              80

Leu Gln Met Ser Ser Leu Lys Ser Glu Asp Thr Ala Met Tyr Tyr Cys
            85              90              95

Ala Arg Arg Arg Asn Tyr Pro Tyr Tyr Tyr Gly Ser Arg Gly Tyr Phe
        100             105             110

Asp Tyr Trp Gly Gln Gly Thr Thr Val Thr Val Ser Ser
        115             120             125
```

<210> 32
<211> 112
<212> PRT
<213> Artificial sequence

<400> 32

```
Asp Ile Glu Leu Thr Gln Ser Pro Ala Ser Leu Ala Val Ser Leu Gly
1               5               10              15

Gln Arg Ala Thr Ile Ser Cys Arg Ala Ser Glu Ser Val Asp Ser Tyr
            20              25              30

Gly Asn Ser Phe Met His Trp Tyr Gln Gln Lys Pro Gly Gln Pro Pro
        35              40              45

Lys Leu Leu Ile Tyr Arg Ala Leu Asn Leu Glu Ser Gly Ile Pro Ala
    50              55              60

Arg Phe Ser Gly Ser Gly Ser Arg Thr Asp Phe Thr Leu Thr Ile Asn
65              70              75              80

Pro Val Glu Ala Asp Asp Val Ala Thr Tyr Tyr Cys Gln Gln Ser Asn
            85              90              95

Glu Asp Pro Trp Thr Phe Gly Gly Gly Thr Lys Leu Glu Ile Lys Arg
        100             105             110
```

<210> 33
<211> 116
<212> PRT
<213> Artificial sequence

<400> 33

24

```
Gln Val Gln Leu Gln Glu Ser Gly Gly Asp Leu Val Gln Pro Gly Gly
1               5               10              15

Ser Leu Lys Leu Ser Cys Ala Val Ser Gly Phe Ser Leu Thr Gly Tyr
            20              25              30

Gly Val Asn Trp Val Arg Gln Thr Pro Asp Lys Arg Leu Glu Trp Val
        35              40              45

Ala Met Ile Trp Gly Asp Gly Asn Thr Asp Tyr Asn Ser Ser Val Lys
    50              55              60

Gly Arg Phe Thr Ile Ser Lys Asp Asn Ala Lys Ser Thr Val Tyr Leu
65              70              75              80

Gln Met Ser Ser Leu Lys Ser Glu Asp Thr Ala Met Tyr Tyr Cys Ala
            85              90              95

Arg Glu Arg Asp Tyr Arg Leu Asp Tyr Trp Gly Gln Gly Thr Thr Val
            100             105             110

Thr Val Ser Ser
            115
```

<210> 34
<211> 108
<212> PRT
<213> Artificial sequence

<400> 34

```
Asp Ile Glu Leu Thr Gln Ser Pro Ala Ser Leu Ala Val Ser Leu Gly
1               5               10              15

Gln Arg Ala Thr Ile Ser Cys Arg Ala Ser Gly Asn Ile His Asn Tyr
            20              25              30

Leu Ala Trp Tyr Gln Gln Lys Pro Gly Gln Pro Pro Lys Leu Leu Ile
        35              40              45

Tyr Tyr Thr Thr Thr Leu Ala Asp Gly Ile Pro Ala Arg Phe Ser Gly
    50              55              60

Ser Gly Ser Gly Thr Asp Tyr Thr Leu Thr Ile Asn Pro Val Glu Ala
65              70              75              80

Asp Asp Val Ala Thr Tyr Tyr Cys Gln His Phe Trp Ser Thr Pro Arg
            85              90              95

Thr Phe Gly Gly Gly Thr Lys Leu Glu Ile Lys Arg
            100             105
```

<210> 35
<211> 116
<212> PRT
<213> Artificial sequence

<400> 35

```
Gln Val Gln Leu Gln Glu Ser Gly Gly Asp Leu Val Gln Pro Gly Gly
1               5                   10                  15
Ser Leu Lys Leu Ser Cys Ala Ala Ser Gly Phe Thr Phe Ser Ser Tyr
            20                  25                  30
Gly Met Ser Trp Val Arg Gln Thr Pro Asp Lys Arg Leu Glu Leu Val
            35                  40                  45
Ala Thr Ile Asn Ser Asn Gly Gly Ser Thr Phe Tyr Pro Asp Ser Val
        50                  55                  60
Lys Gly Arg Phe Thr Ile Ser Arg Asp Asn Ala Lys Asn Thr Leu Tyr
65                  70                  75                  80
Leu Gln Met Ser Ser Leu Lys Ser Glu Asp Thr Ala Met Tyr Tyr Cys
                85                  90                  95
Ala Arg Thr Arg Pro Tyr Phe Asp Tyr Trp Gly Gln Gly Thr Thr Val
            100                 105                 110
Thr Val Ser Ser
            115
```

<210> 36
<211> 108
<212> PRT
<213> Artificial sequence

<400> 36

```
Asp Ile Glu Leu Thr Gln Ser Pro Ala Ser Leu Ala Val Ser Leu Gly
1               5                   10                  15
Gln Arg Ala Thr Ile Ser Cys Arg Ala Ser Glu Ser Val His Asn Tyr
            20                  25                  30
Met His Trp Tyr Gln Gln Lys Pro Gly Gln Pro Pro Lys Leu Leu Ile
            35                  40                  45
Tyr Arg Ala Leu Asn Leu Glu Ser Gly Ile Pro Ala Arg Phe Ser Gly
        50                  55                  60
Ser Gly Ser Arg Thr Asp Phe Thr Leu Thr Ile Asn Pro Val Glu Ala
65                  70                  75                  80
Asp Asp Val Ala Thr Tyr Tyr Cys Gln Gln Val Thr Tyr Lys Pro Trp
                85                  90                  95
Thr Phe Gly Gly Gly Thr Lys Leu Glu Ile Lys Arg
            100                 105
```

<210> 37
<211> 116
<212> PRT
<213> Artificial sequence

<400> 37

```
Gln Val Gln Leu Gln Glu Ser Gly Gly Asp Leu Val Gln Pro Gly Gly
1               5                   10                  15

Ser Leu Lys Leu Ser Cys Ala Ala Ser Gly Phe Thr Phe Ser Ser Tyr
            20                  25                  30

Gly Met Ser Trp Val Arg Gln Thr Pro Asp Lys Arg Leu Glu Leu Val
            35                  40                  45

Ala Thr Ile Asn Ser Asn Gly Gly Ser Thr Phe Tyr Pro Asp Ser Val
        50                  55                  60

Lys Gly Arg Phe Thr Ile Ser Arg Asp Asn Ala Lys Asn Thr Leu Tyr
65                  70                  75                  80

Leu Gln Met Ser Ser Leu Lys Ser Glu Asp Thr Ala Met Tyr Tyr Cys
                85                  90                  95

Ala Arg Glu Arg Trp Asp Phe Asp Tyr Trp Gly Gln Gly Thr Thr Val
            100                 105                 110

Thr Val Ser Ser
        115
```

<210> 38
<211> 108
<212> PRT
<213> Artificial sequence

<400> 38

```
Asp Ile Glu Leu Thr Gln Ser Pro Ala Ser Leu Ala Val Ser Leu Gly
1               5                   10                  15

Gln Arg Ala Thr Ile Ser Cys Arg Ala Ser Glu Ser Val His Asn Tyr
            20                  25                  30

Met His Trp Tyr Gln Gln Lys Pro Gly Gln Pro Pro Lys Leu Leu Ile
            35                  40                  45

Tyr Arg Ala Leu Asn Leu Glu Ser Gly Ile Pro Ala Arg Phe Ser Gly
        50                  55                  60

Ser Gly Ser Arg Thr Asp Phe Thr Leu Thr Ile Asn Pro Val Glu Ala
65                  70                  75                  80

Asp Asp Val Ala Thr Tyr Tyr Cys Gln Gln Ala Leu Ser Pro Pro Trp
                85                  90                  95

Thr Phe Gly Gly Gly Thr Lys Leu Glu Ile Lys Arg
            100                 105
```

<210> 39
<211> 116
<212> PRT

<213> Artificial sequence

<400> 39

```
Gln Val Gln Leu Gln Glu Ser Gly Gly Asp Leu Val Gln Pro Gly Gly
1               5                   10                  15
Ser Leu Lys Leu Ser Cys Ala Ala Ser Gly Phe Thr Phe Ser Ser Tyr
            20                  25                  30
Gly Met Ser Trp Val Arg Gln Thr Pro Asp Lys Arg Leu Glu Leu Val
            35                  40                  45
Ala Thr Ile Asn Ser Asn Gly Gly Ser Thr Phe Tyr Pro Asp Ser Val
            50                  55                  60
Lys Gly Arg Phe Thr Ile Ser Arg Asp Asn Ala Lys Asn Thr Leu Tyr
65                  70                  75                  80
Leu Gln Met Ser Ser Leu Lys Ser Glu Asp Thr Ala Met Tyr Tyr Cys
                85                  90                  95
Ala Arg Asn Trp Arg Arg Phe Asp Tyr Trp Gly Gln Gly Thr Thr Val
            100                 105                 110

                        Thr Val Ser Ser
                        115
```

<210> 40
<211> 108
<212> PRT
<213> Artificial sequence

<400> 40

```
Asp Ile Glu Leu Thr Gln Ser Pro Ala Ser Leu Ala Val Ser Leu Gly
1               5                   10                  15
Gln Arg Ala Thr Ile Ser Cys Arg Ala Ser Glu Ser Val His Asn Tyr
            20                  25                  30
Met His Trp Tyr Gln Gln Lys Pro Gly Gln Pro Pro Lys Leu Leu Ile
            35                  40                  45
Tyr Arg Ala Leu Asn Leu Glu Ser Gly Ile Pro Ala Arg Phe Ser Gly
        50                  55                  60
Ser Gly Ser Arg Thr Asp Phe Thr Leu Thr Ile Asn Pro Val Glu Ala
65                  70                  75                  80
Asp Asp Val Ala Thr Tyr Tyr Cys Gln Gln Gly Ala Ser Ile Pro Trp
                85                  90                  95
Thr Phe Gly Gly Gly Thr Lys Leu Glu Ile Lys Arg
            100                 105
```

<210> 41
<211> 116

<212> PRT
<213> Artificial sequence

<400> 41

```
Gln Val Gln Leu Gln Glu Ser Gly Gly Asp Leu Val Gln Pro Gly Gly
1               5                   10                  15

Ser Leu Lys Leu Ser Cys Ala Ala Ser Gly Phe Thr Phe Ser Ser Tyr
            20                  25                  30

Gly Met Ser Trp Val Arg Gln Thr Pro Asp Lys Arg Leu Glu Leu Val
            35                  40                  45

Ala Thr Ile Asn Ser Asn Gly Gly Ser Thr Phe Tyr Pro Asp Ser Val
        50                  55                  60

Lys Gly Arg Phe Thr Ile Ser Arg Asp Asn Ala Lys Asn Thr Leu Tyr
65                  70                  75                  80

Leu Gln Met Ser Ser Leu Lys Ser Glu Asp Thr Ala Met Tyr Tyr Cys
                85                  90                  95

Ala Arg Gly Arg His Lys Phe Asp Tyr Trp Gly Gln Gly Thr Thr Val
            100                 105                 110

Thr Val Ser Ser
            115
```

<210> 42
<211> 108
<212> PRT
<213> Artificial sequence

<400> 42

```
Asp Ile Glu Leu Thr Gln Ser Pro Ala Ser Leu Ala Val Ser Leu Gly
1               5                   10                  15

Gln Arg Ala Thr Ile Ser Cys Arg Ala Ser Glu Ser Val His Asn Tyr
            20                  25                  30

Met His Trp Tyr Gln Gln Lys Pro Gly Gln Pro Pro Lys Leu Leu Ile
            35                  40                  45

Tyr Arg Ala Leu Asn Leu Glu Ser Gly Ile Pro Ala Arg Phe Ser Gly
        50                  55                  60

Ser Gly Ser Arg Thr Asp Phe Thr Leu Thr Ile Asn Pro Val Glu Ala
65                  70                  75                  80

Asp Asp Val Ala Thr Tyr Tyr Cys Gln Gln Tyr Gly Arg Arg Pro Trp
                85                  90                  95

Thr Phe Gly Gly Gly Thr Lys Leu Glu Ile Lys Arg
            100                 105
```

<210> 43
<211> 116
<212> PRT

29

<213> Artificial sequence

<400> 43

```
Gln Val Gln Leu Gln Glu Ser Gly Gly Asp Leu Val Gln Pro Gly Gly
1               5                   10                  15

Ser Leu Lys Leu Ser Cys Ala Ala Ser Gly Phe Thr Phe Ser Ser Tyr
            20                  25                  30

Gly Met Ser Trp Val Arg Gln Thr Pro Asp Lys Arg Leu Glu Leu Val
            35                  40                  45

Ala Thr Ile Asn Ser Asn Gly Gly Ser Thr Phe Tyr Pro Asp Ser Val
        50                  55                  60

Lys Gly Arg Phe Thr Ile Ser Arg Asp Asn Ala Lys Asn Thr Leu Tyr
65                  70                  75                  80

Leu Gln Met Ser Ser Leu Lys Ser Glu Asp Thr Ala Met Tyr Tyr Cys
                85                  90                  95

Ala Arg Asn Asn Trp Ser Phe Asp Tyr Trp Gly Gln Gly Thr Thr Val
            100                 105                 110

Thr Val Ser Ser
            115
```

<210> 44
<211> 108
<212> PRT
<213> Artificial sequence

<400> 44

```
Asp Ile Glu Leu Thr Gln Ser Pro Ala Ser Leu Ala Val Ser Leu Gly
1               5                   10                  15

Gln Arg Ala Thr Ile Ser Cys Arg Ala Ser Glu Ser Val His Asn Tyr
                20                  25                  30

Met His Trp Tyr Gln Gln Lys Pro Gly Gln Pro Pro Lys Leu Leu Ile
            35                  40                  45

Tyr Arg Ala Leu Asn Leu Glu Ser Gly Ile Pro Ala Arg Phe Ser Gly
        50                  55                  60

Ser Gly Ser Arg Thr Asp Phe Thr Leu Thr Ile Asn Pro Val Glu Ala
65                  70                  75                  80

Asp Asp Val Ala Thr Tyr Tyr Cys Gln Gln Gly Gln Arg Lys Pro Trp
                85                  90                  95

Thr Phe Gly Gly Gly Thr Lys Leu Glu Ile Lys Arg
            100                 105
```

<210> 45

<211> 116
<212> PRT
<213> Artificial sequence

<400> 45

```
Gln Val Gln Leu Gln Glu Ser Gly Gly Asp Leu Val Gln Pro Gly Gly
1               5                   10                  15

Ser Leu Lys Leu Ser Cys Ala Ala Ser Gly Phe Thr Phe Ser Ser Tyr
            20                  25                  30

Gly Met Ser Trp Val Arg Gln Thr Pro Asp Lys Arg Leu Glu Leu Val
            35                  40                  45

Ala Thr Ile Asn Ser Asn Gly Gly Ser Thr Phe Tyr Pro Asp Ser Val
        50                  55                  60

Lys Gly Arg Phe Thr Ile Ser Arg Asp Asn Ala Lys Asn Thr Leu Tyr
65                  70                  75                  80

Leu Gln Met Ser Ser Leu Lys Ser Glu Asp Thr Ala Met Tyr Tyr Cys
                85                  90                  95

Ala Arg Asn Asn Tyr Ser Phe Asp Tyr Trp Gly Gln Gly Thr Thr Val
            100                 105                 110

Thr Val Ser Ser
            115
```

<210> 46
<211> 108
<212> PRT
<213> Artificial sequence

<400> 46

```
Asp Ile Glu Leu Thr Gln Ser Pro Ala Ser Leu Ala Val Ser Leu Gly
1               5                   10                  15

Gln Arg Ala Thr Ile Ser Cys Arg Ala Ser Glu Ser Val His Asn Tyr
            20                  25                  30

Met His Trp Tyr Gln Gln Lys Pro Gly Gln Pro Pro Lys Leu Leu Ile
            35                  40                  45

Tyr Arg Ala Leu Asn Leu Glu Ser Gly Ile Pro Ala Arg Phe Ser Gly
        50                  55                  60
```

```
Ser Gly Ser Arg Thr Asp Phe Thr Leu Thr Ile Asn Pro Val Glu Ala
65              70              75              80

Asp Asp Val Ala Thr Tyr Tyr Cys Gln Gln Gly Arg Arg Ala Pro Trp
                85              90              95

Thr Phe Gly Gly Gly Thr Lys Leu Glu Ile Lys Arg
            100             105
```

<210> 47
<211> 116
<212> PRT
<213> Artificial sequence

<400> 47

```
Gln Val Gln Leu Gln Glu Ser Gly Gly Asp Leu Val Gln Pro Gly Gly
1               5               10              15

Ser Leu Lys Leu Ser Cys Ala Ala Ser Gly Phe Thr Phe Ser Ser Tyr
            20              25              30

Gly Met Ser Trp Val Arg Gln Thr Pro Asp Lys Arg Leu Glu Leu Val
        35              40              45

Ala Thr Ile Asn Ser Asn Gly Gly Ser Thr Phe Tyr Pro Asp Ser Val
    50              55              60

Lys Gly Arg Phe Thr Ile Ser Arg Asp Asn Ala Lys Asn Thr Leu Tyr
65              70              75              80

Leu Gln Met Ser Ser Leu Lys Ser Glu Asp Thr Ala Met Tyr Tyr Cys
            85              90              95

Ala Arg Val Thr Tyr Asn Phe Asp Tyr Trp Gly Gln Gly Thr Thr Val
            100             105             110

Thr Val Ser Ser
        115
```

<210> 48
<211> 108
<212> PRT
<213> Artificial sequence

<400> 48

```
Asp Ile Glu Leu Thr Gln Ser Pro Ala Ser Leu Ala Val Ser Leu Gly
1               5               10              ·   15

Gln Arg Ala Thr Ile Ser Cys Arg Ala Ser Glu Ser Val His Asn Tyr
            20              25              30

Met His Trp Tyr Gln Gln Lys Pro Gly Gln Pro Pro Lys Leu Leu Ile
        35              40              45

Tyr Arg Ala Leu Asn Leu Glu Ser Gly Ile Pro Ala Arg Phe Ser Gly
    50              55              60

Ser Gly Ser Arg Thr Asp Phe Thr Leu Thr Ile Asn Pro Val Glu Ala
65              70              75              80

Asp Asp Val Ala Thr Tyr Tyr Cys Gln Gln Ser Arg Arg Pro Pro Trp
            85              90              95

    Thr Phe Gly Gly Gly Thr Lys Leu Glu Ile Lys Arg
                100             105
```

<210> 49
<211> 252
<212> PRT
<213> Artificial sequence

<400> 49

```
Gln Val Gln Leu Gln Glu Ser Gly Gly Asp Leu Val Gln Pro Gly Gly
1               5               10              15

Ser Leu Lys Leu Ser Cys Ala Ala Ser Gly Phe Thr Phe Ser Ser Tyr
            20              25              30

Gly Met Ser Trp Val Arg Gln Thr Pro Asp Lys Arg Leu Glu Leu Val
        35              40              45

Ala Thr Ile Asn Ser Asn Gly Gly Ser Thr Phe Tyr Pro Asp Ser Val
    50              55              60

Lys Gly Arg Phe Thr Ile Ser Arg Asp Asn Ala Lys Asn Thr Leu Tyr
65              70              75              80

Leu Gln Met Ser Ser Leu Lys Ser Glu Asp Thr Ala Met Tyr Tyr Cys
            85              90              95

Ala Arg Arg Arg Asn Tyr Pro Tyr Tyr Tyr Gly Ser Arg Gly Tyr Phe
        100             105             110

Asp Tyr Trp Gly Gln Gly Thr Thr Val Thr Val Ser Ser Gly Gly Gly
        115             120             125

Gly Ser Gly Gly Gly Gly Ser Gly Gly Gly Gly Ser Asp Ile Glu Leu
    130             135             140

Thr Gln Ser Pro Ala Ser Leu Ala Val Ser Leu Gly Gln Arg Ala Thr
145             150             155             160

Ile Ser Cys Arg Ala Ser Glu Ser Val Asp Ser Tyr Gly Asn Ser Phe
            165             170             175

Met His Trp Tyr Gln Gln Lys Pro Gly Gln Pro Pro Lys Leu Leu Ile
        180             185             190

Tyr Arg Ala Leu Asn Leu Glu Ser Gly Ile Pro Ala Arg Phe Ser Gly
        195             200             205

Ser Gly Ser Arg Thr Asp Phe Thr Leu Thr Ile Asn Pro Val Glu Ala
    210             215             220

Asp Asp Val Ala Thr Tyr Tyr Cys Gln Gln Ser Asn Glu Asp Pro Trp
225             230             235             240

Thr Phe Gly Gly Gly Thr Lys Leu Glu Ile Lys Arg
            245             250
```

<210> 50
<211> 239
<212> PRT
<213> Artificial sequence

<400> 50

```
Gln Val Gln Leu Gln Glu Ser Gly Gly Asp Leu Val Gln Pro Gly Gly
```

34

```
         1                 5                      10                    15

       Ser Leu Lys Leu Ser Cys Ala Val Ser Gly Phe Ser Leu Thr Gly Tyr
                   20                      25                  30

       Gly Val Asn Trp Val Arg Gln Thr Pro Asp Lys Arg Leu Glu Trp Val
                   35                  40                  45

       Ala Met Ile Trp Gly Asp Gly Asn Thr Asp Tyr Asn Ser Ser Val Lys
               50                  55                  60

       Gly Arg Phe Thr Ile Ser Lys Asp Asn Ala Lys Ser Thr Val Tyr Leu
       65                  70                  75                  80

       Gln Met Ser Ser Leu Lys Ser Glu Asp Thr Ala Met Tyr Tyr Cys Ala
                       85                  90                  95

       Arg Glu Arg Asp Tyr Arg Leu Asp Tyr Trp Gly Gln Gly Thr Thr Val
                   100                 105                 110

       Thr Val Ser Ser Gly Gly Gly Gly Ser Gly Gly Gly Gly Ser Gly Gly
                   115                 120                 125

       Gly Gly Ser Asp Ile Glu Leu Thr Gln Ser Pro Ala Ser Leu Ala Val
               130                 135                 140

       Ser Leu Gly Gln Arg Ala Thr Ile Ser Cys Arg Ala Ser Gly Asn Ile
       145                 150                 155                 160

       His Asn Tyr Leu Ala Trp Tyr Gln Gln Lys Pro Gly Gln Pro Pro Lys
                       165                 170                 175

       Leu Leu Ile Tyr Tyr Thr Thr Thr Leu Ala Asp Gly Ile Pro Ala Arg
                       180                 185                 190

       Phe Ser Gly Ser Gly Ser Gly Thr Asp Tyr Thr Leu Thr Ile Asn Pro
                   195                 200                 205

       Val Glu Ala Asp Asp Val Ala Thr Tyr Tyr Cys Gln His Phe Trp Ser
               210                 215                 220

       Thr Pro Arg Thr Phe Gly Gly Gly Thr Lys Leu Glu Ile Lys Arg
       225                 230                 235
```

<210> 51
<211> 15
<212> PRT
<213> Artificial sequence

<400> 51

```
       Gly Gly Gly Gly Ser Gly Gly Gly Gly Ser Gly Gly Gly Gly Ser
       1                 5                      10                    15
```

<210> 52
<211> 75
<212> DNA
<213> Artificial sequence

<220>
<221> CDS
<222> (1)..(75)
<223> coding DNA for H-FR1-F8

<400> 52

```
cag gtg cag ctg cag gag tct ggg gga gac tta gtg cag cct gga ggg   48
Gln Val Gln Leu Gln Glu Ser Gly Gly Asp Leu Val Gln Pro Gly Gly
1               5                   10                  15

tcc ctg aaa ctc tcc tgt gca gcc tct   75
Ser Leu Lys Leu Ser Cys Ala Ala Ser
            20              25
```

<210> 53
<211> 25
<212> PRT
<213> Artificial sequence

<400> 53

```
Gln Val Gln Leu Gln Glu Ser Gly Gly Asp Leu Val Gln Pro Gly Gly
1               5                   10                  15

Ser Leu Lys Leu Ser Cys Ala Ala Ser
            20              25
```

<210> 54
<211> 42
<212> DNA
<213> Artificial sequence

<220>
<221> CDS
<222> (1) .. (42)
<223> coding DNA for H-FR2-F8

<400> 54

```
tgg gtt cgc cag act cca gac aag agg ctg gag ttg gtc gca   42
Trp Val Arg Gln Thr Pro Asp Lys Arg Leu Glu Leu Val Ala
1               5                   10
```

<210> 55
<211> 14
<212> PRT
<213> Artificial sequence

<400> 55

```
        Trp Val Arg Gln Thr Pro Asp Lys Arg Leu Glu Leu Val Ala
        1               5               10
```

<210> 56
<211> 117
<212> DNA
<213> Artificial sequence

<220>
<221> CDS
<222> (1)..(117)
<223> coding DNA for H-FR3-F8

<400> 56

```
        tat cca gac agt gtg aag ggc cga ttc acc atc tcc aga gac aat gcc
        48
        Tyr Pro Asp Ser Val Lys Gly Arg Phe Thr Ile Ser Arg Asp Asn Ala
        1               5               10              15

        aag aac acc ctg tac ctg caa atg agc agt ctg aag tct gag gac aca
        96
        Lys Asn Thr Leu Tyr Leu Gln Met Ser Ser Leu Lys Ser Glu Asp Thr
                        20              25              30

        gcc atg tat tac tgt gca aga
        117
        Ala Met Tyr Tyr Cys Ala Arg
                        35
```

<210> 57
<211> 39
<212> PRT
<213> Artificial sequence

<400> 57

```
        Tyr Pro Asp Ser Val Lys Gly Arg Phe Thr Ile Ser Arg Asp Asn Ala
        1               5               10              15

        Lys Asn Thr Leu Tyr Leu Gln Met Ser Ser Leu Lys Ser Glu Asp Thr
                        20              25              30

        Ala Met Tyr Tyr Cys Ala Arg
                        35
```

<210> 58
<211> 33
<212> DNA
<213> Artificial sequence

<220>
<221> CDS

37

<222> (1)..(33)
<223> coding DNA for H-FR4-F8

<400> 58

```
tgg ggc caa ggg acc acg gtc acc gtc tcc tca
33
Trp Gly Gln Gly Thr Thr Val Thr Val Ser Ser
1               5               10
```

<210> 59
<211> 11
<212> PRT
<213> Artificial sequence

<400> 59

```
Trp Gly Gln Gly Thr Thr Val Thr Val Ser Ser
1               5               10
```

<210> 60

<211> 69
<212> DNA
<213> Artificial sequence

<220>
<221> CDS
<222> (1) .. (69)
<223> coding DNA for L-FR1-F8

<400> 60

```
gac atc gag ctc act cag tct cca gct tct ttg gct gtg tct cta ggg
48
Asp Ile Glu Leu Thr Gln Ser Pro Ala Ser Leu Ala Val Ser Leu Gly
1               5               10              15

cag agg gcc acc ata tcc tgc
69
Gln Arg Ala Thr Ile Ser Cys
                20
```

<210> 61
<211> 23
<212> PRT
<213> Artificial sequence

<400> 61

```
Asp Ile Glu Leu Thr Gln Ser Pro Ala Ser Leu Ala Val Ser Leu Gly
1               5               10              15

Gln Arg Ala Thr Ile Ser Cys
                20
```

<210> 62
<211> 45
<212> DNA
<213> Artificial sequence

<220>
<221> CDS
<222> (1)..(45)
<223> coding DNA for L-FR2-F8

<400> 62

```
tgg tac cag cag aaa cca gga cag cca ccc aaa ctc ctc atc tat
45
Trp Tyr Gln Gln Lys Pro Gly Gln Pro Pro Lys Leu Leu Ile Tyr
1               5               10              15
```

<210> 63
<211> 15
<212> PRT
<213> Artificial sequence

<400> 63

```
Trp Tyr Gln Gln Lys Pro Gly Gln Pro Pro Lys Leu Leu Ile Tyr
1               5               10              15
```

<210> 64
<211> 96
<212> DNA
<213> Artificial sequence

<220>
<221> CDS
<222> (1)..(96)
<223> coding DNA for L-FR3-F8

<400> 64

```
ggg atc cct gcc agg ttc agt ggc agt ggg tct agg aca gac ttc acc
48
Gly Ile Pro Ala Arg Phe Ser Gly Ser Gly Ser Arg Thr Asp Phe Thr
1               5               10              15

ctc acc att aat cct gtg gag gct gat gat gtt gca acc tat tac tgt
96
Leu Thr Ile Asn Pro Val Glu Ala Asp Asp Val Ala Thr Tyr Tyr Cys
            20              25              30
```

<210> 65
<211> 32
<212> PRT
<213> Artificial sequence

<400> 65

```
Gly Ile Pro Ala Arg Phe Ser Gly Ser Gly Ser Arg Thr Asp Phe Thr
1               5               10                  15

Leu Thr Ile Asn Pro Val Glu Ala Asp Asp Val Ala Thr Tyr Tyr Cys
            20              25              30
```

<210> 66
<211> 33
<212> DNA
<213> Artificial sequence

<220>
<221> CDS
<222> (1) .. (33)
<223> coding DNA for L-FR4-F8

<400> 66

```
ttc ggt gga ggc acc aag ctc gag atc aaa cgg
33
Phe Gly Gly Gly Thr Lys Leu Glu Ile Lys Arg
1               5               10
```

<210> 67
<211> 11
<212> PRT
<213> Artificial sequence

<400> 67

```
Phe Gly Gly Gly Thr Lys Leu Glu Ile Lys Arg
1               5               10
```

<210> 68
<211> 30
<212> DNA
<213> Artificial sequence

<220>
<221> CDS
<222> (1)..(30)
<223> coding DNA for H-CDR1-F8

<400> 68

```
gga ttc act ttc agt agc tat ggc atg tct
30
Gly Phe Thr Phe Ser Ser Tyr Gly Met Ser
1               5               10
```

<210> 69
<211> 10
<212> PRT
<213> Artificial sequence

<400> 69

```
Gly Phe Thr Phe Ser Ser Tyr Gly Met Ser
1               5               10
```

<210> 70
<211> 30
<212> DNA
<213> Artificial sequence

<220>
<221> CDS
<222> (1)..(30)

<400> 70

```
acc att aat agt aat ggt ggt agc acc ttt
30
Thr Ile Asn Ser Asn Gly Gly Ser Thr Phe
1               5               10
```

<210> 71
<211> 10
<212> PRT
<213> Artificial sequence

<400> 71

```
Thr Ile Asn Ser Asn Gly Gly Ser Thr Phe
1               5               10
```

<210> 72
<211> 48
<212> DNA
<213> Artificial sequence

<220>
<221> CDS
<222> (1)..(48)

<400> 72

41

```
aga agg aat tac ccc tat tac tac ggt agt aga ggc tac ttt gac tac
48
Arg Arg Asn Tyr Pro Tyr Tyr Tyr Gly Ser Arg Gly Tyr Phe Asp Tyr
1               5                   10                  15
```

<210> 73
<211> 16
<212> PRT
<213> Artificial sequence

<400> 73

```
Arg Arg Asn Tyr Pro Tyr Tyr Tyr Gly Ser Arg Gly Tyr Phe Asp Tyr
1               5                   10                  15
```

<210> 74
<211> 45
<212> DNA
<213> Artificial sequence

<220>
<221> CDS
<222> (1) .. (45)

<400> 74

```
aga gcc agt gaa agt gtt gat agt tat ggc aat agt ttt atg cac
45
Arg Ala Ser Glu Ser Val Asp Ser Tyr Gly Asn Ser Phe Met His
1               5                   10                  15
```

<210> 75
<211> 15
<212> PRT
<213> Artificial sequence

<400> 75

```
Arg Ala Ser Glu Ser Val Asp Ser Tyr Gly Asn Ser Phe Met His
1               5                   10                  15
```

<210> 76
<211> 21
<212> DNA
<213> Artificial sequence

<220>
<221> CDS
<222> (1)..(21)

<400> 76

```
                    cgt gca tta aat cta gaa tct
                    21
                    Arg Ala Leu Asn Leu Glu Ser
                    1               5
```

<210> 77
<211> 7
<212> PRT
<213> Artificial sequence


<400> 77


```
        Arg Ala Leu Asn Leu Glu Ser
        1               5
```


<210> 78
<211> 27
<212> DNA
<213> Artificial sequence


<220>
<221> CDS
<222> (1)..(27)


<400> 78


```
        cag caa agt aat gag gat ccg tgg acg
        27
        Gln Gln Ser Asn Glu Asp Pro Trp Thr
        1               5
```


<210> 79
<211> 9
<212> PRT
<213> Artificial sequence


<400> 79


```
        Gln Gln Ser Asn Glu Asp Pro Trp Thr
        1               5
```


<210> 80
<211> 375
<212> DNA
<213> Artificial sequence


<220>
<221> CDS
<222> (1)..(375)
<223> coding DNA for VH-F8


<400> 80

```
cag gtg cag ctg cag gag tct ggg gga gac tta gtg cag cct gga ggg
48
Gln Val Gln Leu Gln Glu Ser Gly Gly Asp Leu Val Gln Pro Gly Gly
1               5                   10                  15

tcc ctg aaa ctc tcc tgt gca gcc tct gga ttc act ttc agt agc tat
96
Ser Leu Lys Leu Ser Cys Ala Ala Ser Gly Phe Thr Phe Ser Ser Tyr
            20                  25                  30

ggc atg tct tgg gtt cgc cag act cca gac aag agg ctg gag ttg gtc
144
Gly Met Ser Trp Val Arg Gln Thr Pro Asp Lys Arg Leu Glu Leu Val
        35                  40                  45

gca acc att aat agt aat ggt ggt agc acc ttt tat cca gac agt gtg
192
Ala Thr Ile Asn Ser Asn Gly Gly Ser Thr Phe Tyr Pro Asp Ser Val
    50                  55                  60

aag ggc cga ttc acc atc tcc aga gac aat gcc aag aac acc ctg tac



240
Lys Gly Arg Phe Thr Ile Ser Arg Asp Asn Ala Lys Asn Thr Leu Tyr
65                  70                  75                  80

ctg caa atg agc agt ctg aag tct gag gac aca gcc atg tat tac tgt
288
Leu Gln Met Ser Ser Leu Lys Ser Glu Asp Thr Ala Met Tyr Tyr Cys
                85                  90                  95

gca aga aga agg aat tac ccc tat tac tac ggt agt aga ggc tac ttt
336
Ala Arg Arg Arg Asn Tyr Pro Tyr Tyr Tyr Gly Ser Arg Gly Tyr Phe
            100                 105                 110

gac tac tgg ggc caa ggg acc acg gtc acc gtc tcc tca
375
Asp Tyr Trp Gly Gln Gly Thr Thr Val Thr Val Ser Ser          .
            115                 120                 125
```

<210> 81
<211> 125
<212> PRT
<213> Artificial sequence

<400> 81

```
Gln Val Gln Leu Gln Glu Ser Gly Gly Asp Leu Val Gln Pro Gly Gly
1               5                   10                  15

Ser Leu Lys Leu Ser Cys Ala Ala Ser Gly Phe Thr Phe Ser Ser Tyr
            20                  25                  30

Gly Met Ser Trp Val Arg Gln Thr Pro Asp Lys Arg Leu Glu Leu Val
        35                  40                  45

Ala Thr Ile Asn Ser Asn Gly Gly Ser Thr Phe Tyr Pro Asp Ser Val
    50                  55                  60

Lys Gly Arg Phe Thr Ile Ser Arg Asp Asn Ala Lys Asn Thr Leu Tyr
65                  70                  75                  80

Leu Gln Met Ser Ser Leu Lys Ser Glu Asp Thr Ala Met Tyr Tyr Cys
            85                  90                  95

Ala Arg Arg Arg Asn Tyr Pro Tyr Tyr Tyr Gly Ser Arg Gly Tyr Phe
            100                 105                 110

Asp Tyr Trp Gly Gln Gly Thr Thr Val Thr Val Ser Ser
        115                 120                 125
```

<210> 82
<211> 336
<212> DNA
<213> Artificial sequence

<220>
<221> CDS
<222> (1)..(336)

<400> 82

```
gac atc gag ctc act cag tct cca gct tct ttg gct gtg tct cta ggg
48
Asp Ile Glu Leu Thr Gln Ser Pro Ala Ser Leu Ala Val Ser Leu Gly
1               5                   10                  15

cag agg gcc acc ata tcc tgc aga gcc agt gaa agt gtt gat agt tat
96
Gln Arg Ala Thr Ile Ser Cys Arg Ala Ser Glu Ser Val Asp Ser Tyr
                20                  25                  30

ggc aat agt ttt atg cac tgg tac cag cag aaa cca gga cag cca ccc
144
Gly Asn Ser Phe Met His Trp Tyr Gln Gln Lys Pro Gly Gln Pro Pro
            35                  40                  45

aaa ctc ctc atc tat cgt gca tta aat cta gaa tct ggg atc cct gcc
192
Lys Leu Leu Ile Tyr Arg Ala Leu Asn Leu Glu Ser Gly Ile Pro Ala
    50                  55                  60

agg ttc agt ggc agt ggg tct agg aca gac ttc acc ctc acc att aat
240
Arg Phe Ser Gly Ser Gly Ser Arg Thr Asp Phe Thr Leu Thr Ile Asn
65                  70                  75                  80

cct gtg gag gct gat gat gtt gca acc tat tac tgt cag caa agt aat
288
Pro Val Glu Ala Asp Asp Val Ala Thr Tyr Tyr Cys Gln Gln Ser Asn
                85                  90                  95

gag gat ccg tgg acg ttc ggt gga ggc acc aag ctc gag atc aaa cgg
336
Glu Asp Pro Trp Thr Phe Gly Gly Gly Thr Lys Leu Glu Ile Lys Arg
                100                 105                 110
```

<210> 83
<211> 112
<212> PRT
<213> Artificial sequence

<400> 83

```
Asp Ile Glu Leu Thr Gln Ser Pro Ala Ser Leu Ala Val Ser Leu Gly
1               5                   10                  15

Gln Arg Ala Thr Ile Ser Cys Arg Ala Ser Glu Ser Val Asp Ser Tyr
                20                  25                  30

Gly Asn Ser Phe Met His Trp Tyr Gln Gln Lys Pro Gly Gln Pro Pro
            35                  40                  45

Lys Leu Leu Ile Tyr Arg Ala Leu Asn Leu Glu Ser Gly Ile Pro Ala
    50                  55                  60

Arg Phe Ser Gly Ser Gly Ser Arg Thr Asp Phe Thr Leu Thr Ile Asn
65                  70                  75                  80
```

```
Pro Val Glu Ala Asp Asp Val Ala Thr Tyr Tyr Cys Gln Gln Ser Asn
                85                  90                  95


Glu Asp Pro Trp Thr Phe Gly Gly Gly Thr Lys Leu Glu Ile Lys Arg
            100                 105                 110
```

<210> 84
<211> 756
<212> DNA
<213> Artificial sequence

<220>
<221> CDS
<222> (1) .. (756)

<400> 84

```
cag gtg cag ctg cag gag tct ggg gga gac tta gtg cag cct gga ggg
48
Gln Val Gln Leu Gln Glu Ser Gly Gly Asp Leu Val Gln Pro Gly Gly
1               5                   10                  15

tcc ctg aaa ctc tcc tgt gca gcc tct gga ttc act ttc agt agc tat
96
Ser Leu Lys Leu Ser Cys Ala Ala Ser Gly Phe Thr Phe Ser Ser Tyr
            20                  25                  30

ggc atg tct tgg gtt cgc cag act cca gac aag agg ctg gag ttg gtc
144
Gly Met Ser Trp Val Arg Gln Thr Pro Asp Lys Arg Leu Glu Leu Val
            35                  40                  45

gca acc att aat agt aat ggt ggt agc acc ttt tat cca gac agt gtg
192
Ala Thr Ile Asn Ser Asn Gly Gly Ser Thr Phe Tyr Pro Asp Ser Val
    50                  55                  60

aag ggc cga ttc acc atc tcc aga gac aat gcc aag aac acc ctg tac
240
Lys Gly Arg Phe Thr Ile Ser Arg Asp Asn Ala Lys Asn Thr Leu Tyr
65                  70                  75                  80

ctg caa atg agc agt ctg aag tct gag gac aca gcc atg tat tac tgt
288
Leu Gln Met Ser Ser Leu Lys Ser Glu Asp Thr Ala Met Tyr Tyr Cys
                85                  90                  95

gca aga aga agg aat tac ccc tat tac tac ggt agt aga ggc tac ttt
336
Ala Arg Arg Arg Asn Tyr Pro Tyr Tyr Tyr Gly Ser Arg Gly Tyr Phe
            100                 105                 110

gac tac tgg ggc caa ggg acc acg gtc acc gtc tcc tca ggt gga ggc
384
Asp Tyr Trp Gly Gln Gly Thr Thr Val Thr Val Ser Ser Gly Gly Gly
        115                 120                 125

ggt tca ggc gga ggt ggc tct ggc ggt ggc gga tcg gac atc gag ctc
432
Gly Ser Gly Gly Gly Gly Ser Gly Gly Gly Gly Ser Asp Ile Glu Leu
        130                 135                 140

act cag tct cca gct tct ttg gct gtg tct cta ggg cag agg gcc acc
480
Thr Gln Ser Pro Ala Ser Leu Ala Val Ser Leu Gly Gln Arg Ala Thr
```

```
            145                      150                      155                      160
ata tcc tgc aga gcc agt gaa agt gtt gat agt tat ggc aat agt ttt
528
Ile Ser Cys Arg Ala Ser Glu Ser Val Asp Ser Tyr Gly Asn Ser Phe
                165                      170                      175

atg cac tgg tac cag cag aaa cca gga cag cca ccc aaa ctc ctc atc
576
Met His Trp Tyr Gln Gln Lys Pro Gly Gln Pro Pro Lys Leu Leu Ile
                180                      185                      190

tat cgt gca tta aat cta gaa tct ggg atc cct gcc agg ttc agt ggc
624
Tyr Arg Ala Leu Asn Leu Glu Ser Gly Ile Pro Ala Arg Phe Ser Gly
        195                      200                      205

agt ggg tct agg aca gac ttc acc ctc acc att aat cct gtg gag gct
672
Ser Gly Ser Arg Thr Asp Phe Thr Leu Thr Ile Asn Pro Val Glu Ala
        210                      215                      220

gat gat gtt gca acc tat tac tgt cag caa agt aat gag gat ccg tgg
720
Asp Asp Val Ala Thr Tyr Tyr Cys Gln Gln Ser Asn Glu Asp Pro Trp
225                      230                      235                      240

acg ttc ggt gga ggc acc aag ctc gag atc aaa cgg
756
Thr Phe Gly Gly Gly Thr Lys Leu Glu Ile Lys Arg
                245                      250
```

<210> 85
<211> 252
<212> PRT
<213> Artificial sequence

<400> 85

```
Gln Val Gln Leu Gln Glu Ser Gly Gly Asp Leu Val Gln Pro Gly Gly
1               5               10                  15

Ser Leu Lys Leu Ser Cys Ala Ala Ser Gly Phe Thr Phe Ser Ser Tyr
            20              25                  30

Gly Met Ser Trp Val Arg Gln Thr Pro Asp Lys Arg Leu Glu Leu Val
            35              40                  45

Ala Thr Ile Asn Ser Asn Gly Gly Ser Thr Phe Tyr Pro Asp Ser Val
        50              55                  60

Lys Gly Arg Phe Thr Ile Ser Arg Asp Asn Ala Lys Asn Thr Leu Tyr
65              70              75                      80

Leu Gln Met Ser Ser Leu Lys Ser Glu Asp Thr Ala Met Tyr Tyr Cys
                85              90                  95

Ala Arg Arg Arg Asn Tyr Pro Tyr Tyr Tyr Gly Ser Arg Gly Tyr Phe
            100             105             110
```

```
Asp Tyr Trp Gly Gln Gly Thr Thr Val Thr Val Ser Ser Gly Gly Gly
        115                 120             125

Gly Ser Gly Gly Gly Gly Ser Gly Gly Gly Gly Ser Asp Ile Glu Leu
        130                 135             140

Thr Gln Ser Pro Ala Ser Leu Ala Val Ser Leu Gly Gln Arg Ala Thr
145                 150             155                 160

Ile Ser Cys Arg Ala Ser Glu Ser Val Asp Ser Tyr Gly Asn Ser Phe
        165                 170             175

Met His Trp Tyr Gln Gln Lys Pro Gly Gln Pro Pro Lys Leu Leu Ile
        180                 185             190

Tyr Arg Ala Leu Asn Leu Glu Ser Gly Ile Pro Ala Arg Phe Ser Gly
        195                 200             205

Ser Gly Ser Arg Thr Asp Phe Thr Leu Thr Ile Asn Pro Val Glu Ala
        210                 215             220

Asp Asp Val Ala Thr Tyr Tyr Cys Gln Gln Ser Asn Glu Asp Pro Trp
225                 230             235                 240

Thr Phe Gly Gly Gly Thr Lys Leu Glu Ile Lys Arg
                245                 250
```

<210> 86
<211> 717
<212> DNA
<213> Artificial sequence

<220>
<221> CDS
<222> (1)..(717)
<223> codingDNA for scFv(P5)

<400> 86

```
cag gtg cag ctg cag gag tct ggg gga gac tta gtg cag cct gga ggg
48
Gln Val Gln Leu Gln Glu Ser Gly Gly Asp Leu Val Gln Pro Gly Gly
1               5               10              15

tcc ctg aaa ctc tcc tgt gca gtc tct gga ttc agt ttg act ggc tat
96
Ser Leu Lys Leu Ser Cys Ala Val Ser Gly Phe Ser Leu Thr Gly Tyr
            20              25              30

ggc gtg aat tgg gtt cgc cag act cca gac aag agg ctg gag tgg gtc
144
Gly Val Asn Trp Val Arg Gln Thr Pro Asp Lys Arg Leu Glu Trp Val
        35              40              45

gca atg att tgg ggt gat ggt aac acc gat tat aat tcc agt gtg aag
```

```
                192
                Ala Met Ile Trp Gly Asp Gly Asn Thr Asp Tyr Asn Ser Ser Val Lys
                    50              55              60

                ggc cga ttc acc atc tcc aaa gac aat gcc aag agc acc gtg tac ctg
                240
                Gly Arg Phe Thr Ile Ser Lys Asp Asn Ala Lys Ser Thr Val Tyr Leu
                65              70              75              80

                caa atg agc agt ctg aag tct gag gac aca gcc atg tat tac tgt gca
                288
                Gln Met Ser Ser Leu Lys Ser Glu Asp Thr Ala Met Tyr Tyr Cys Ala
                            85              90              95

                aga gaa agg gat tac cgc ctt gac tac tgg ggc caa ggg acc acg gtc
                336
                Arg Glu Arg Asp Tyr Arg Leu Asp Tyr Trp Gly Gln Gly Thr Thr Val
                        100             105             110

                acc gtc tcc tca ggt gga ggc ggt tca ggc gga ggt ggc tct ggc ggt
                384
                Thr Val Ser Ser Gly Gly Gly Gly Ser Gly Gly Gly Gly Ser Gly Gly
                        115             120             125

                ggc gga tcg gac atc gag ctc act cag tct cca gct tct ttg gct gtg
                432
                Gly Gly Ser Asp Ile Glu Leu Thr Gln Ser Pro Ala Ser Leu Ala Val
                    130             135             140

                tct cta ggg cag agg gcc acc ata tcc tgc aga gcc agt gga aat att
                480
                Ser Leu Gly Gln Arg Ala Thr Ile Ser Cys Arg Ala Ser Gly Asn Ile
                145             150             155             160

                cat aat tat ttg gcc tgg tac cag cag aaa cca gga cag cca ccc aar
                528
                His Asn Tyr Leu Ala Trp Tyr Gln Gln Lys Pro Gly Gln Pro Pro Lys
                            165             170             175

                ctc ctc atc tat tat aca aca act cta gca gat ggg atc cct gcc agg
                576
                Leu Leu Ile Tyr Tyr Thr Thr Thr Leu Ala Asp Gly Ile Pro Ala Arg
                        180             185             190

                ttc agt ggc agt ggg tct ggg aca gac tac acc ctc acc att aat cct
                624
                Phe Ser Gly Ser Gly Ser Gly Thr Asp Tyr Thr Leu Thr Ile Asn Pro
                        195             200             205

                gtg gag gct gat gat gtt gca acc tat tac tgt cag cac ttt tgg tcg
                672
                Val Glu Ala Asp Asp Val Ala Thr Tyr Tyr Cys Gln His Phe Trp Ser
                    210             215             220

                act ccg agg acg ttc ggt gga ggc acc aag ctc gag atc aaa cgg
                717
                Thr Pro Arg Thr Phe Gly Gly Gly Thr Lys Leu Glu Ile Lys Arg
                225             230             235
```

<210> 87
<211> 239

53

<212> PRT
<213> Artificial sequence

<400> 87

Gln Val Gln Leu Gln Glu Ser Gly Gly Asp Leu Val Gln Pro Gly Gly
1               5               10              15

Ser Leu Lys Leu Ser Cys Ala Val Ser Gly Phe Ser Leu Thr Gly Tyr
            20              25              30

Gly Val Asn Trp Val Arg Gln Thr Pro Asp Lys Arg Leu Glu Trp Val
        35              40              45

Ala Met Ile Trp Gly Asp Gly Asn Thr Asp Tyr Asn Ser Ser Val Lys
    50              55              60

Gly Arg Phe Thr Ile Ser Lys Asp Asn Ala Lys Ser Thr Val Tyr Leu
65              70              75              80

Gln Met Ser Ser Leu Lys Ser Glu Asp Thr Ala Met Tyr Tyr Cys Ala
            85              90              95

Arg Glu Arg Asp Tyr Arg Leu Asp Tyr Trp Gly Gln Gly Thr Thr Val
            100             105             110

Thr Val Ser Ser Gly Gly Gly Gly Ser Gly Gly Gly Gly Ser Gly Gly
        115             120             125

Gly Gly Ser Asp Ile Glu Leu Thr Gln Ser Pro Ala Ser Leu Ala Val
    130             135             140

Ser Leu Gly Gln Arg Ala Thr Ile Ser Cys Arg Ala Ser Gly Asn Ile
145             150             155             160

His Asn Tyr Leu Ala Trp Tyr Gln Gln Lys Pro Gly Gln Pro Pro Lys
            165             170             175

Leu Leu Ile Tyr Tyr Thr Thr Thr Leu Ala Asp Gly Ile Pro Ala Arg
            180             185             190

Phe Ser Gly Ser Gly Ser Gly Thr Asp Tyr Thr Leu Thr Ile Asn Pro
    195             200             205

Val Glu Ala Asp Asp Val Ala Thr Tyr Tyr Cys Gln His Phe Trp Ser
    210             215             220

Thr Pro Arg Thr Phe Gly Gly Gly Thr Lys Leu Glu Ile Lys Arg
225             230             235

<210> 88
<211> 10
<212> PRT

<213> Artificial sequence

<220>
<221> misc_feature
<222> (1)..(1)
<223> Xaa is any amino acid

<220>
<221> misc_feature
<222> (2)..(2)
<223> Xaa is any amino acid

<220>
<221> misc_feature
<222> (3)..(3)
<223> Xaa is any amino acid

<220>
<221> misc_feature
<222> (4)..(4)
<223> Xaa is any amino acid

<220>
<221> misc_feature
<222> (5)..(5)
<223> Xaa is any amino acid

<220>
<221> misc_feature
<222> (6)..(6)
<223> Xaa is any amino acid

<220>
<221> misc_feature
<222> (7)..(7)
<223> Xaa is any amino acid

<220>
<221> misc_feature
<222> (8)..(8)
<223> Xaa is any amino acid

<220>
<221> misc_feature
<222> (9) .. (9)
<223> Xaa is any amino acid

<220>
<221> misc_feature
<222> (10)..(10)
<223> Xaa is any amino acid

<400> 88

```
          Xaa Xaa Xaa Xaa Xaa Xaa Xaa Xaa Xaa Xaa
          1               5                   10
```

<210> 89
<211> 10
<212> PRT
<213> Artificial sequence

<220>
<221> misc_feature
<222> (1)..(1)
<223> Xaa is any amino acid

<220>
<221> misc_feature
<222> (2) .. (2)
<223> Xaa is any amino acid

<220>
<221> misc_feature
<222> (3)..(3)
<223> Xaa is any amino acid

<220>
<221> misc_feature
<222> (4) .. (4)
<223> Xaa is any amino acid

<220>
<221> misc_feature
<222> (5)..(5)
<223> Xaa is any amino acid

<220>
<221> misc_feature
<222> (6)..(6)
<223> Xaa is any amino acid

<220>
<221> misc_feature
<222> (7)..(7)
<223> Xaa is any amino acid

<220>
<221> misc_feature
<222> (8)..(8)
<223> Xaa is any amino acid

<220>
<221> misc_feature
<222> (9)..(9)
<223> Xaa is any amino acid

<220>
<221> misc_feature
<222> (10)..(10)
<223> Xaa is any amino acid

<400> 89

```
Xaa Xaa Xaa Xaa Xaa Xaa Xaa Xaa Xaa Xaa
1               5                   10
```

<210> 90
<211> 16
<212> PRT
<213> Artificial sequence

<220>
<221> misc_feature
<222> (1)..(1)
<223> Xaa is any amino acid

<220>
<221> misc_feature
<222> (2)..(2)
<223> Xaa is any amino acid

<220>
<221> misc_feature
<222> (3)..(3)
<223> Xaa is any amino acid

<220>
<221> misc_feature
<222> (4)..(4)
<223> Xaa is any amino acid

<220>
<221> misc_feature
<222> (5)..(5)
<223> Xaa is any amino acid

<220>
<221> misc_feature
<222> (6)..(6)
<223> Xaa is any amino acid, and can be eliminated by deletion

<220>
<221> misc_feature
<222> (7)..(7)
<223> Xaa is any amino acid, and can be eliminated by deletion

<220>
<221> misc_feature
<222> (8)..(8)
<223> Xaa is any amino acid, and can be eliminated by deletion

<220>
<221> misc_feature
<222> (9)..(9)
<223> Xaa is any amino acid, and can be eliminated by deletion

<220>
<221> misc_feature
<222> (10)..(10)

<223> Xaa is any amino acid, and can be eliminated by deletion

<220>
<221> misc_feature
<222> (11)..(11)
<223> Xaa is any amino acid, and can be eliminated by deletion

<220>
<221> misc_feature
<222> (12)..(12)
<223> Xaa is any amino acid, and can be eliminated by deletion

<220>
<221> misc_feature
<222> (13)..(13)
<223> Xaa is any amino acid, and can be eliminated by deletion

<220>
<221> misc_feature
<222> (14) .. (14)
<223> Xaa is any amino acid

<220>
<221> misc_feature
<222> (15)..(15)
<223> Xaa is any amino acid

<220>
<221> misc_feature
<222> (16)..(16)
<223> Xaa is any amino acid

<400> 90

```
    Xaa Xaa Xaa Xaa Xaa Xaa Xaa Xaa Xaa Xaa Xaa Xaa Xaa Xaa Xaa Xaa
    1                   5                   10                  15
```

<210> 91
<211> 15
<212> PRT
<213> Artificial sequence

<220>
<221> misc_feature
<222> (1)..(1)
<223> Xaa is any amino acid

<220>
<221> misc_feature
<222> (2)..(2)
<223> Xaa is any amino acid

<220>
<221> misc_feature
<222> (3)..(3)
<223> Xaa is any amino acid

<220>
<221> misc_feature
<222> (4)..(4)
<223> Xaa is any amino acid

<220>
<221> misc_feature
<222> (5)..(5)
<223> Xaa is any amino acid

<220>
<221> misc_feature
<222> (6)..(6)
<223> Xaa is any amino acid

<220>
<221> misc_feature
<222> (7)..(7)
<223> Xaa is any amino acid, and can be eliminated by deletion

<220>
<221> misc_feature
<222> (8)..(8)
<223> Xaa is any amino acid, and can be eliminated by deletion

<220>
<221> misc_feature
<222> (9)..(9)
<223> Xaa is any amino acid, and can be eliminated by deletion

<220>
<221> misc_feature
<222> (10)..(10)
<223> Xaa is any amino acid, and can be eliminated by deletion

<220>
<221> misc_feature
<222> (11)..(11)
<223> Xaa is any amino acid

<220>
<221> misc_feature
<222> (12)..(12)
<223> Xaa is any amino acid

<220>
<221> misc_feature
<222> (13)..(13)
<223> Xaa is any amino acid

<220>
<221> misc_feature
<222> (14)..(14)
<223> Xaa is any amino acid

<220>
<221> misc_feature
<222> (15)..(15)

<223> Xaa is any amino acid

<400> 91

```
        Xaa Xaa Xaa Xaa Xaa Xaa Xaa Xaa Xaa Xaa Xaa Xaa Xaa Xaa Xaa
        1                   5                   10                  15
```

<210> 92
<211> 7
<212> PRT
<213> Artificial sequence

<220>
<221> misc_feature
<222> (1)..(1)
<223> Xaa is any amino acid

<220>
<221> misc_feature
<222> (2) .. (2)
<223> Xaa is any amino acid

<220>
<221> misc_feature
<222> (3) .. (3)
<223> Xaa is any amino acid

<220>
<221> misc_feature
<222> (4) .. (4)
<223> Xaa is any amino acid

<220>
<221> misc_feature
<222> (5) .. (5)
<223> Xaa is any amino acid

<220>
<221> misc_feature
<222> (6)..(6)
<223> Xaa is any amino acid

<220>
<221> misc_feature
<222> (7) .. (7)
<223> Xaa is any amino acid

<400> 92

```
                        Xaa Xaa Xaa Xaa Xaa Xaa Xaa
                        1                   5
```

<210> 93
<211> 9
<212> PRT
<213> Artificial sequence

<220>
<221> misc_feature
<222> (1)..(1)
<223> Xaa is any amino acid

<220>
<221> misc_feature
<222> (2)..(2)
<223> Xaa is any amino acid

<220>
<221> misc_feature
<222> (3)..(3)
<223> Xaa is any amino acid

<220>
<221> misc_feature
<222> (4)..(4)
<223> Xaa is any amino acid

<220>
<221> misc_feature
<222> (5)..(5)
<223> Xaa is any amino acid

<220>
<221> misc_feature
<222> (6)..(6)
<223> Xaa is any amino acid

<220>
<221> misc_feature
<222> (7)..(7)
<223> Xaa is any amino acid

<220>
<221> misc_feature
<222> (8)..(8)
<223> Xaa is any amino acid

<220>
<221> misc_feature
<222> (9)..(9)
<223> Xaa is any amino acid

<400> 93

```
Xaa Xaa Xaa Xaa Xaa Xaa Xaa Xaa Xaa
1               5
```

<210> 94
<211> 11
<212> PRT
<213> Artificial sequence

<400> 94

```
Arg Ala Ser Glu Ser Val His Asn Tyr Met His
1               5                   10
```

<210> 95
<211> 33
<212> DNA
<213> Artificial sequence

<220>
<221> CDS
<222> (1)..(33)
<223> coding DNA for L-CDR1-MUT

<400> 95

```
aga gcc agt gaa agt gtt cat aat tat atg cac
33
Arg Ala Ser Glu Ser Val His Asn Tyr Met His
1               5                   10
```

<210> 96
<211> 11
<212> PRT
<213> Artificial sequence

<400> 96

```
Arg Ala Ser Glu Ser Val His Asn Tyr Met His
1               5                   10
```

<210> 97
<211> 10
<212> PRT
<213> Artificial: peptide

<400> 97

```
Gly Phe Ser Leu Thr Gly Tyr Gly Val Asn
1               5                   10
```

<210> 98
<211> 16
<212> PRT
<213> Artificial sequence

<400> 98

```
Met Ile Trp Gly Asp Gly Asn Thr Asp Tyr Asn Ser Ser Val Lys Gly
1               5                   10                  15
```

<210> 99
<211> 11
<212> PRT

<213> Artificial sequence

<400> 99

```
Arg Ala Ser Gly Asn Ile His Asn Tyr Leu Ala
         1               5                    10
```

<210> 100
<211> 7
<212> PRT
<213> Artificial sequence

<400> 100

```
                              Tyr Thr Thr Thr Leu Ala Asp
                              1               5
```

<210> 101
<211> 125
<212> PRT
<213> Artificial sequence

<220>
<221> misc_feature
<222> (24)..(24)
<223> Xaa is Ala

<220>
<221> misc_feature
<222> (27)..(27)
<223> Xaa is any amino acid

<220>
<221> misc_feature
<222> (28)..(28)
<223> Xaa is any amino acid

<220>
<221> misc_feature
<222> (29)..(29)
<223> Xaa is any amino acid

<220>
<221> misc_feature
<222> (30)..(30)
<223> Xaa is any amino acid

<220>
<221> misc_feature
<222> (31)..(31)
<223> Xaa is any amino acid

<220>
<221> misc_feature
<222> (32)..(32)

<223> Xaa is any amino acid

<220>
<221> misc_feature
<222> (33)..(33)
<223> Xaa is any amino acid

<220>
<221> misc_feature
<222> (34)..(34)
<223> Xaa is any amino acid

<220>
<221> misc_feature
<222> (35) .. (35)
<223> Xaa is any amino acid

<220>
<221> misc_feature
<222> (36) .. (36)
<223> Xaa is any amino acid

<220>
<221> misc_feature
<222> (48)..(48)
<223> Xaa is Leu or a non polar amino acid

<220>
<221> misc_feature
<222> (51)..(51)
<223> Xaa is any amino acid

<220>
<221> misc_feature
<222> (52) .. (52)
<223> Xaa is any amino acid

<220>
<221> misc_feature
<222> (53) .. (53)
<223> Xaa is any amino acid

<220>
<221> misc_feature
<222> (54)..(54)
<223> Xaa is any amino acid

<220>
<221> misc_feature
<222> (55) .. (55)
<223> Xaa is any amino acid

<220>
<221> misc_feature
<222> (56) .. (56)
<223> Xaa is any amino acid

<220>

<221> misc_feature
<222> (57) .. (57)
<223> Xaa is any amino acid

<220>
<221> misc_feature
<222> (58)..(58)
<223> Xaa is any amino acid

<220>
<221> misc_feature
<222> (59)..(59)
<223> Xaa is any amino acid

<220>
<221> misc_feature
<222> (60)..(60)
<223> Xaa is any amino acid

<220>
<221> misc_feature
<222> (62) .. (62)
<223> Xaa is Pro, a non polar amino acid or an amino acid with no charge

<220>
<221> misc_feature
<222> (63)..(63)
<223> Xaa is Asp, a non polar amino acid or an amino acid with no charg

<220>
<221> misc_feature
<222> (73)..(73)
<223> Xaa is Arg or a basic amino acid

<220>
<221> misc_feature
<222> (78)..(78)
<223> Xaa is Asn, or a polar amino acid with no charge

<220>
<221> misc_feature
<222> (80)..(80)
<223> Xaa is Leu or a non polar amino acid

<220>
<221> misc_feature
<222> (99)..(99)
<223> Xaa is any amino acid

<220>
<221> misc_feature
<222> (100)..(100)
<223> Xaa is any amino acid

<220>
<221> misc_feature
<222> (101)..(101)
<223> Xaa is any amino acid

<220>
<221> misc_feature
<222> (102)..(102)
<223> Xaa is any amino acid

<220>
<221> misc_feature
<222> (103)..(103)
<223> Xaa is any amino acid

<220>
<221> misc_feature
<222> (104)..(104)
<223> Xaa is any amino acid, and can be eliminated by deletion

<220>
<221> misc_feature
<222> (105) .. (105)
<223> Xaa is any amino acid, and can be eliminated by deletion

<220>
<221> misc_feature
<222> (106)..(106)
<223> Xaa is any amino acid, and can be eliminated by deletion

<220>
<221> misc_feature
<222> (107)..(107)
<223> Xaa is any amino acid, and can be eliminated by deletion

<220>
<221> misc_feature
<222> (108)..(108)
<223> Xaa is any amino acid, and can be eliminated by deletion

<220>
<221> misc_feature
<222> (109)..(109)
<223> Xaa is any amino acid, and can be eliminated by deletion

<220>
<221> misc_feature
<222> (110)..(110)
<223> Xaa is any amino acid, and can be eliminated by deletion

<220>
<221> misc_feature
<222> (111)..(111)
<223> Xaa is any amino acid, and can be eliminated by deletion

<220>
<221> misc_feature
<222> (112)..(112)
<223> Xaa is any amino acid

<220>
<221> misc_feature
<222> (113)..(113)

<223> Xaa is any amino acid

<220>
<221> misc_feature
<222> (114)..(114)
<223> Xaa is any amino acid

<400> 101

```
Gln Val Gln Leu Gln Glu Ser Gly Gly Asp Leu Val Gln Pro Gly Gly
1               5               10              15

Ser Leu Lys Leu Ser Cys Ala Xaa Ser Xaa Xaa Xaa Xaa Xaa Xaa Xaa
            20              25              30

Xaa Xaa Xaa Trp Val Arg Gln Thr Pro Asp Lys Arg Leu Glu Xaa Val
        35              40              45

Ala Xaa Xaa Xaa Xaa Xaa Xaa Xaa Xaa Xaa Xaa Tyr Xaa Xaa Ser Val
    50              55              60

Lys Gly Arg Phe Thr Ile Ser Xaa Asp Asn Ala Lys Xaa Thr Xaa Tyr
65              70              75              80

Leu Gln Met Ser Ser Leu Lys Ser Glu Asp Thr Ala Met Tyr Tyr Cys
            85              90              95

Ala Arg Xaa Xaa Xaa Xaa Xaa Xaa Xaa Xaa Xaa Xaa Xaa Xaa Xaa Xaa
        100             105             110

Xaa Xaa Trp Gly Gln Gly Thr Thr Val Thr Val Ser Ser
        115             120             125
```

<210> 102
<211> 112
<212> PRT
<213> Artificial sequence

<220>
<221> misc_feature
<222> (24)..(24)
<223> Xaa is any amino acid

<220>
<221> misc_feature
<222> (25) .. (25)
<223> Xaa is any amino acid

<220>
<221> misc_feature
<222> (26)..(26)
<223> Xaa is any amino acid

<220>
<221> misc_feature
<222> (27)..(27)
<223> Xaa is any amino acid

<220>

<221> misc_feature
<222> (28)..(28)
<223> Xaa is any amino acid

<220>
<221> misc_feature
<222> (29)..(29)
<223> Xaa is any amino acid

<220>
<221> misc_feature
<222> (30)..(30)
<223> Xaa is any amino acid, and can be eliminated by deletion

<220>
<221> misc_feature
<222> (31)..(31)
<223> Xaa is any amino acid, and can be eliminated by deletion

<220>
<221> misc_feature
<222> (32)..(32)
<223> Xaa is any amino acid, and can be eliminated by deletion

<220>
<221> misc_feature
<222> (33)..(33)
<223> Xaa is any amino acid, and can be eliminated by deletion

<220>
<221> misc_feature
<222> (34)..(34)
<223> Xaa is any amino acid

<220>
<221> misc_feature
<222> (35)..(35)
<223> Xaa is any amino acid

<220>
<221> misc_feature
<222> (36)..(36)
<223> Xaa is any amino acid

<220>
<221> misc_feature
<222> (37)..(37)
<223> Xaa is any amino acid

<220>
<221> misc_feature
<222> (38)..(38)
<223> Xaa is any amino acid

<220>
<221> misc_feature
<222> (54)..(54)
<223> Xaa is any amino acid

<220>
<221> misc_feature
<222> (55)..(55)
<223> Xaa is any amino acid

<220>
<221> misc_feature
<222> (56)..(56)
<223> Xaa is any amino acid

<220>
<221> misc_feature
<222> (57)..(57)
<223> Xaa is any amino acid

<220>
<221> misc_feature
<222> (58)..(58)
<223> Xaa is any amino acid

<220>
<221> misc_feature
<222> (59)..(59)
<223> Xaa is any amino acid

<220>
<221> misc_feature
<222> (60)..(60)
<223> Xaa is any amino acid

<220>
<221> misc_feature
<222> (72)..(72)
<223> Xaa is Arg, a non polar amino acid or an amino acid with no charg

<220>
<221> misc_feature
<222> (75)..(75)
<223> Xaa is Phe, a non polar amino acid or an amino acid with no charg

<220>
<221> misc_feature
<222> (93)..(93)
<223> Xaa is any amino acid

<220>
<221> misc_feature
<222> (94)..(94)
<223> Xaa is any amino acid

<220>
<221> misc_feature
<222> (95) .. (95)
<223> Xaa is any amino acid

<220>
<221> misc_feature
<222> (96)..(96)

<223> Xaa is any amino acid

<220>
<221> misc_feature
<222> (97)..(97)
<223> Xaa is any amino acid

<220>
<221> misc_feature
<222> (98)..(98)
<223> Xaa is any amino acid

<220>
<221> misc_feature
<222> (99)..(99)
<223> Xaa is any amino acid

<220>
<221> misc_feature
<222> (100)..(100)
<223> Xaa is any amino acid

<220>
<221> misc_feature
<222> (101)..(101)
<223> Xaa is any amino acid

<400> 102

```
Asp Ile Glu Leu Thr Gln Ser Pro Ala Ser Leu Ala Val Ser Leu Gly
1               5                   10                  15

Gln Arg Ala Thr Ile Ser Cys Xaa Xaa Xaa Xaa Xaa Xaa Xaa Xaa Xaa
            20                  25                  30

Xaa Xaa Xaa Xaa Xaa Xaa Trp Tyr Gln Gln Lys Pro Gly Gln Pro Pro
        35                  40                  45

Lys Leu Leu Ile Tyr Xaa Xaa Xaa Xaa Xaa Xaa Xaa Gly Ile Pro Ala
    50                  55                  60

Arg Phe Ser Gly Ser Gly Ser Xaa Thr Asp Xaa Thr Leu Thr Ile Asn

    65                  70                  75                  80

Pro Val Glu Ala Asp Asp Val Ala Thr Tyr Tyr Cys Xaa Xaa Xaa Xaa
                85                  90                  95

Xaa Xaa Xaa Xaa Xaa Phe Gly Gly Gly Thr Lys Leu Glu Ile Lys Arg
            100                 105                 110
```